# DEMANDE DE BREVET EUROPEEN

(11) **EP 1 695 964 A1**
(43) Date de publication de la demande: **30.08.2006**
(21) Numéro de dépôt: 06010725.7
(22) Date de dépôt: 14.11.2001
(51) Int. Cl.: C07D 215/20

(54) **Dérivés quinoléines comme intermédiaires pour la préparation des pipéridines hétérocyclylalcoyl**

(30) Priorité: 15.11.2000 FR 0014738
(62) Demande divisionnaire de: 01996538.3
(71) Demandeur: NOVEXEL, 93230 Romainville (FR)
(72) Inventeur: Bacqe, Eric, 91190 Gil Sur Yvette (FR); Carry, Jean Christophe, 94100 Saint Maur Des Fosses (FR); El-Ahmad, Youssef, 94000 Creteil (FR); Evers, Michel, 94510 La Queue En Brie (FR); Hubert, Philippe, 94700 Maisons-Alfort (FR); Malleron, Jean Luc, 91460 Marcoussis (FR); Mignani, Serge, 92290 Chatenay-Malabry (FR); Pantel, Guy, 94510 La Queue En Brie (FR); Tabart, Michel, 91290 La Norville (FR)
(74) Mandataire: Hirsch & Associés

(57) **Abrégé**

L'un quelconque des composés dont les noms suivent:
- la 4-chloro-3-fluoro-6-méthoxy-quinoléine,
- la 4-bromo-3-fluoro-6-méthoxy-quinoléine,
- la 4-iodo-3-fluoro-6-méthoxy-quinoléine,
- la 4-bromo-3-chloro-6-méthoxy-quinoléine,
- la 4-iodo-3-chloro-6-méthoxy-quinoléine,
- la 4-hydroxy-3-chloro-6-méthoxy-quinoléine, et
- la 3-fluoro-6-méthoxy-quinoléine.

## Description

La présente invention concerne des dérivés hétérocyclylalcoyl pipéridine de formule générale : 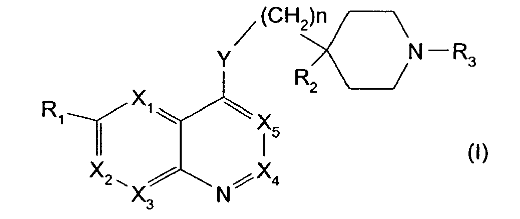 qui sont actifs comme antimicrobiens, leur préparation et les compositions les contenant et a pour objet de nouveaux composés intermédiaires.

Dans les demandes de brevet WO 99/37635 et WO 00/43383 ont été décrits des dérivés de quinolyl propyl pipéridine antimicrobiens, de formule générale : 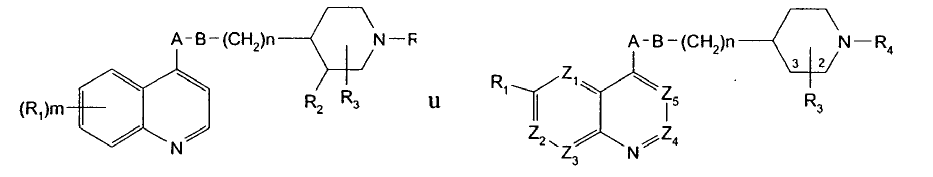 dans laquelle le radical R₁ est notamment alcoxy (C1-6), R₂ est hydrogène, R₃ est en position -2 ou -3 et représente alcoyle (C1-6) pouvant être éventuellement substitué par 1 à 3 substituants choisis parmi thiol, halogène, alcoylthio, trifluorométhyl, carboxy, alcoyloxycarbonyle, alcoylcarbonyle, alcènyloxycarbonyle, alcènylcarbonyle, hydroxy éventuellement substitué par alcoyle ..., R₄ est un groupe-CH₂-R₅ pour lequel R₅ est selectionné parmi alcoyle hydroxyalcoyle, alcènyle, alcynyle, tétrahydrofuryle, phénylalcoyle éventuellement substitué, phénylalcényle éventuellement substitué, hétéroarylalcoyle éventuellement substitué, hétéroaroyle éventuellement substitué ..., n est 0 à 2, m est 1 ou 2 et A et B sont notamment oxygène, soufre, sulfinyle, sulfonyle, NR₁₁, CR₆R₇ pour lequel R₆ et R₇ représentent H, thiol, alcoylthio, halo, trifluorométhyle, alcènyle, alcènylcarbonyle, hydroxy, amino, et Z₁ à Z₅ sont N ou CR₁ₐ ... Ces produits manifestent une activité antimicrobienne. Cependant aucun dérivé disubstitué en position -4 de la pipéridine n'avait été synthétisé jusqu'à ce jour et par conséquent aucune activité biologique n'avait été découverte non plus pour de tels produits. antimicrobienne. Cependant aucun dérivé disubstitué en position -4 de la pipéridine n'avait été synthétisé jusqu'à ce jour et par conséquent aucune activité biologique n'avait été découverte non plus pour de tels produits.

De légères modifications sur les structures déjà connues pouvant entraîner des variations importantes d'activité, il n'était pas évident que des dérivés disubstitués en position -4 de la pipéridine aient également une activité antibactérienne.

Dans la demande de brevet européen EP30044 ont été décrits des dérivés de quinoléine utiles comme cardiovasculaires, répondant à la formule générale : 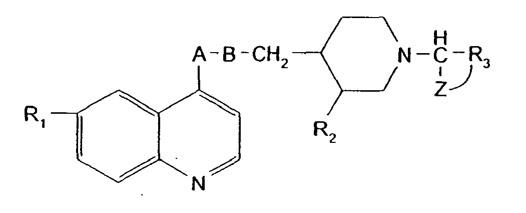 dans laquelle R₁ est notamment alcoyloxy, A-B est -CH₂-CH₂-, -CHOH-CH₂-, -CH₂-CHOH-, -CH₂-CO- ou -CO-CH₂-, R₁ est H, OH ou alcoyloxy, R₂ est éthyle ou vinyle, R₃ est notamment alcoyle, hydroxyalcoyle, cycloalcoyle, hydroxy, alcènyle, alcynyle, tétrahydrofuryle, phénylalcoyle, diphénylalcoyle éventuellement substitué, phénylalcényle éventuellement substitué, benzoyl ou benzoylalcoyle éventuellement substitué, hétéroaryle ou hétéroarylalcoyle éventuellement substitué et Z est H ou alcoyle ou forme avec R₃ un radical cycloalcoyle.

Il a maintenant été trouvé que les produits de formule générale (I) pour lesquels :
X₁, X₂, X₃, X₄ et X₅ représentent respectivement >C-R'₁ à >C-R'₅,
R₁, R'₁, R'₂, R'₃, R'₄, R'₅, sont identiques ou différents et représentent un atome d'hydrogène ou d'halogène ou un radical alcoyle, alcoyloxy, ou représentent un radical méthylène substitué par alcoyloxy,
R₂ représente carboxy, alcoyloxycarbonyle, cycloalcoyloxycarbonyle, cyano, -CONRaRb (pour lequel Ra et Rb représentent respectivement hydrogène, alcoyle, cycloalcoyle, phényle, hétérocyclyle aromatique mono ou bicyclique, ou l'un de Ra ou Rb représente hydroxy, alcoyloxy, cycloalcoyloxy, ou Ra et Rb forment ensemble avec l'atome d'azote auquel ils sont attachés un hétérocycle à 5 ou 6 chaînons pouvant éventuellement contenir un autre hétéroatome choisi parmi O, S ou N et portant le cas échéant un substituant alcoyle, phényle ou hétérocyclyle aromatique mono ou bicyclique sur l'atome d'azote ou le cas échéant dont l'atome de soufre est oxydé à l'état sulfinyle ou sulfonyle), ou R₂ représente hydroxyméthyle, alcoyle contenant 1 ou 2 atomes de carbone substitué par carboxy, alcoyloxycarbonyle, cycloalcoyloxycarbonyle, cyano ou -CONRaRb pour lequel Ra et Rb sont définis conune ci-dessus, ou R₂ représente un radical de structure -CF₂-Rc, -C(CH₃)₂-Rc, -CO-Rc, -CHOH-Rc, -C(cycloalcoyle)-Rc, ou -CH=CH-Rc pour lequel Rc est carboxy, alcoyloxycarbonyle, cycloalcoyloxycarbonyle, ou -CONRaRb pour lequel Ra et Rb sont définis comme ci-dessus,
R₃ représente un radical phényle, hétérocyclyle aromatique mono ou bicyclique, alk-R°₃ pour lequel alk est un radical alcoyle et R°₃ représente hydrogène, halogène, hydroxy, alcoyloxy, alcoylthio, alcoylsulfinyle, alcoylsulfonyle, alcoylamino, dialcoylamino, cycloalcoyle, cycloalcoyloxy, cycloalcoylthio, cycloalcoylsulfinyle, cycloalcoylsulfonyle, cycloalcoylamino, N-cycloalcoyl N-alcoyl amino, -N-(cycloalcoyle)₂, acyle, cycloalcoylcarbonyle, phényle, phénoxy, phénylthio, phénylsulfinyle, phénylsulfonyle, phénylamino, N-alcoyl N-phényl amino, N-cycloalcoyl N-phényl amino, -N-(phényle)₂, phénylalcoyloxy, phénylalcoylthio, phénylalcoylsulfinyle, phénylalcoylsulfonyle, phénylalcoylamino, N-alcoyl N-phényl aminoalcoyle, N-cycloalcoyl N-phénylalcoyl amino, benzoyle, hétérocyclyle aromatique mono ou bicyclique, hétérocyclyloxy, hétérocyclylthio, hétérocyclylsulfinyle, hétérocyclylsulfonyle, hétérocyclylamino, N-alcoyl N-hétérocyclyl amino, N-cycloalcoyl N-hétérocyclyl amino, hétéroyclylcarbonyle, hétérocyclylalcoyloxy, hétérocyclylalcoylthio, hétérocyclylalcoylsulfinyle, hétérocyclylalcoylsulfonyle, hétérocyclylalcoylamino, N-alcoyl N-hétérocyclyl aminoalcoyle, N-cycloalcoyl N-hétérocyclyl aminoalcoyle, (les parties hétérocyclyle citées ci-avant étant aromatiques mono ou bicycliques), carboxy, alcoyloxycarbonyle, -NRaRb ou -CO-NRaRb pour lesquel Ra et Rb sont définis comme ci-dessus dans la définition de R₂, ou bien R°₃ représente -CR'b=CR'c-R'a pour lequel R'a représente phényle, phénylalcoyle, hétérocyclyle ou hétérocyclylalcoyle dont la partie hétérocyclyle est aromatique mono ou bicyclique, phénoxyalcoyle, phénylthloalcoyle, phénylsulfinylalcoyle, phénylsulfonylalcoyle, phénylaminoalcoyle, N-alcoyl N-phényl aminoalcoyle, hétérocyclyloxyalcoyle, hétérocyclylthioalcoyle, hétérocyclylsulfinylalcoyle, hétérocyclylsulfonylalcoyle, hétérocyclylaminoalcoyle, N-alcoyl N-hétérocyclyl aminoalcoyle, hétérocyclylthio, hétérocyclylsulfinyle, hétérocyclylsulfonyle, (les parties hétérocyclyle citées ci-avant étant aromatiques mono ou bicycliques), phénylthio, phénylsulfinyle, phénylsulfonyle, et pour lequel R'b et R'c représentent hydrogène, alcoyle ou cycloalcoyie, ou bien R°₃ représente un radical -C=C-Rd pour lequel Rd est alcoyle, phényle, phénylalcoyle, phénoxyalcoyle, phénylthioalcoyle, N-alcoyl N-phényl aminoalcoyle, hétérocyclyle aromatique mono ou bicyclique, hétérocyclylalcoyle, hétérocyclyloxyalcoyle, hétérocyclylthioalcoyle, hétérocyclylaminoalcoyle, N-alcoyl N-hétérocyclyl aminoalcoyle, (les parties hétérocyclyle citées ci-avant étant aromatiques mono ou bicycliques), ou bien R°₃ représente un radical -CF₂-phénylc ou -CF₂-hétérocyclyle aromatique mono ou bicyclique,
Y représente un radical >CH-Re pour lequel Re est hydrogène, fluoro, hydroxy, alcoyloxy, cycloalcoyloxy, carboxy, alcoyloxycarbonyle, cycloalcoyloxycarbonyle, -NRaRb ou ―CO-NRaRb pour lesquel Ra et Rb sont définis comme ci-avant pour R₂ ou représentent l'un un atome d'hydrogène et l'autre un radical alcoyloxycarbonyle, acyle, cycloalcoylcarbonyle, benzoyle ou hétérocyclylcarbonyle dont la partie hétérocyclyle est aromatique mono ou bicyclique, ou bien Y représente un radical difluorométhylène, carbonyle, hydroxyiminométhylène, alcoyloxyiminométhylène, cycloalcoyloxyiminométhylène, ou un radical cycloalcoylène-1,1 contenant 3 à 6 atomes de carbone, et
n est un nombre entier de 0 à 4
étant entendu que les radicaux ou portions phényle, benzyle, benzoyle ou hétérocyclyle mentionnés ci-dessus peuvent être éventuellement substitués -sur le cycle par 1 à 4 substituants choisis parmi halogène, hydroxy, alcoyle, alcoyloxy, alcoyloxyalcoyle, halogénoalcoyle, trifluorométhyle, trifluorométhoxy, trifluorométhylthio, carboxy, alcoyloxycarbonyle, cyano, alcoylamino, -NRaRb pour lequel Ra et Rb sont définis comme ci-dessus, phényle, hydroxyalcoyle, alcoyithloalcoyle, alcoylsulfinylalcoyle, alcoylsulfonylalcoyle,
sous leurs formes énantiomères ou diastéréoisomères ou les mélanges de ces formes, et/ou le cas échéant sous leur forme syn ou anti ou leur mélange ainsi que leurs sels, sont de puissants agents antibactériens.

Il est entendu que les radicaux et portions alcoyle ou acyle contiennent (sauf mention spéciale) 1 à 10 atomes de carbone en chaîne droite ou ramifiée et que les radicaux cycloalcoyle contiennent 3 à 6 atomes de carbone.

Il est également entendu que les radicaux qui représentent ou portent un atome d'halogène représentent un halogène choisi parmi fluor, chlore, brome ou iode, de préférence le fluor.

Dans la formule générale ci-dessus, lorsque les radicaux représentent ou portent un substituant hétérocyclyle aromatique mono ou bicyclique, ce dernier contient 5 à 10 chaînons et peut être choisi (à titre non limitatif) parmi thiényle, furyle, pyrrolyle, imidazolyle, thiazolyle, oxazolyle, thiadiazolyle, oxadiazolyle, tétrazolyle, pyridyle, pyridazinyle, pyrazinyle, pyrimidinyle, indolyle, benzothiényle, benzofuranyle indazolyle, benzothiazolyle, naphtyridinyle, quinolyle, isoquinolyle, cinnolyle, quinazotyle, quinoxalyle, benzoxazolyle, benzimidazolyle pouvant être éventuellement substitués par les substituants énoncés ci-dessus.

Les produits de formule générale (I) peuvent être obtenus par condensation de la chaîne R₃ sur le dérivé hétérocyclylalcoyl pipéridine de formule générale: 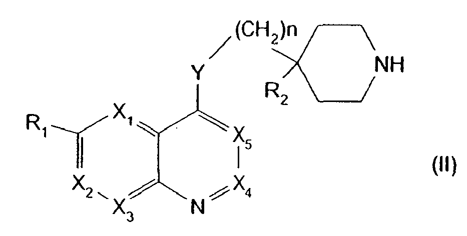 dans laquelle X₁, X₂, X₃, X₄, X₅, R₁, R₂, Y et n sont définis comme précédemment, et R₂ étant protégé lorsqu'il porte un radical carboxy ou amino, suivie le cas échéant de l'élimination du radical protecteur d'acide ou d'amine, éventuellement de la séparation des formes énantiomères ou diastéréoisomères et/ou le cas échéant des formes syn ou anti et éventuellement de la transformation du produit obtenu en un sel.

La condensation de la chaîne R₃ sur la pipéridine s'effectue avantageusement par action d'un dérivé de formule générale :

R₃-X (IIa)

dans laquelle R₃ est défini comme précédemment et X représente un atome d'halogène, un radical méthylsulfonyle, un radical trifluorométhylsulfonyle ou p.toluènesulfonyle, en opérant en milieu anhydre, de préférence inerte (azote ou argon par exemple) dans un solvant organique tel qu'un amide (diméthylformamide par exemple), une cétone (acétone par exemple) ou un nitrile (acétonitrile par exemple) en présence d'une base telle qu'une base organique azotée (par exemple triéthylamine) ou une base minérale (carbonate alcalin : carbonate de potassium par exemple) à une température comprise entre 20°C et la température de reflux du solvant. Il est entendu que l'atome d'azote de la pipéridine du dérivé de formule générale (II) est éventuellement protégé selon les méthodes habituelles qui n'altèrent pas le reste de la molécule ou la réaction ; par exemple la protection s'effectue par un radical protecteur choisi parmi t.butoxycarbonyle ou benzyloxycarbonyle.
De préférence, on fait agir un dérivé de formule générale (IIa) pour lequel X est un atome de brome ou d'iode.

Lorsque R₃ représente un radical phényle, il est également possible de faire agir le dérivé R₃-X iodé ou brome en présence d'un catalyseur au palladium selon la méthode décrite dans J. Org. Chem., 6066 (1997) ou Tet. Lett., 6359 (1997). Le catalyseur au palladium peut être choisi parmi le tris dibenzylidèneacétone dipalladium, ou le diacétate de palladium avec un ligand comme le 2,2'-bis (diphénylphosphino)-1,1'-binaphtyle ou le 2-(di-t-butylphosphino)biphényle par exemple, une base comme le tertiobutylate de sodium ou le carbonate de césium dans un solvant comme le tétrahydrofuranne, le tétraglyme ou le toluène, en présence éventuellement d'un éther couronne comme le 18-C-6 (1,4,7,10,13,16-hexaoxacyclooctadécane). La réaction s'effectue à une température comprise entre 20 et 110°C.

Lorsque R₃ représente un radical -alk-R°₃ pour lequel alk est un radical alcoyle et R°₃ représente -C=C-Rd dans lequel Rd est phényle, phénylalcoyie, hétérocyclyle ou hétérocyclylalcoyle aromatique mono ou bicyclique, il est souvent préférable de condenser un halogénure d'alcynyle : HC≡C-alk-X pour lequel alk est défini comme ci-dessus et X est un atome d'halogène, puis de substituer la chaîne par un radical phényle, phénylalcoyle, hétérocyclyle ou hétérocyclylalcoyle.

Dans cette alternative, l'addition de la chaîne alcynyle s'effectue au moyen d'un halogénure d'alcynyle HC≡C-alk-X pour lequel X est de préférence l'atome de brome, dans les conditions énoncées ci-dessus pour la condensation de la chaîne R₃, en présence ou non d'un iodure de métal alcalin comme par exemple l'iodure de potassium ou de sodium.

La substitution par un radical phényle ou hétérocyclyle s'effectue par action d'un halogénure dérivé du radical cyclique à substituer, en présence de triéthylamine, en milieu anhydre dans un solvant tel qu'un amide (diméthylformamide par exemple) ou un nitrile (acétonitrile par exemple) et en présence d'un sel de palladium comme par exemple le tétrakis triphénylphosphine palladium et d'iodure cuivreux, à une température comprise entre 20°C et la température de reflux du solvant. La substitution par un radical phénylalcoyle ou hétérocyclylalcoyle s'effectue par action de l'halogénure correspondant, en milieu basique par exemple en présence de l'hydrure de potassium ou de sodium ou du n-butyllithium, dans un solvant comme un éther (tétrahydrofuranne) ou un amide (diméthylfonnamide) à une température comprise entre -60°C et la température d'ébullition du mélange réactionnel.

Il est entendu que, si les radicaux alcoyle représentés par R₃ portent des substituants carboxy ou amino, ces derniers sont préalablement protégés, puis libérés après la réaction. On opère selon les méthodes habituelles qui n'altèrent pas le reste de la molécule, notamment selon les méthodes décrites par T.W. Greene et P.G.M. Wuts, Protective Groups in Organic Synthesis (2ème éd.), A. Wiley - Interscience Publication (1991), ou par Mc Omie, Protective Groups in Organic Chemistry, Plenum Press (1973).

Le radical carboxy protégé porté par R₂ peut être choisi parmi les esters facilement hydrolysables. A titre d'exemple peuvent être cités les esters méthylique, benzylique, tertiobutylique, ou bien les esters de phénylpropyle ou d'allyle. Eventuellement la protection du radical carboxy s'effectue simultanément à la réaction.

Le cas échéant, la protection du radical amino s'effectue au moyen des radicaux protecteurs habituels cités dans les références ci-dessus.

La mise en place et l'élimination de ces radicaux protecteurs s'effectue selon les méthodes habituelles, mentionnées ci-dessus pour R₃.

Lorsque R₃ représente un radical -alk-R°₃ pour lequel alk est un radical alcoyle et R°₃ représente un radical phénoxy, phénylthio, phénylamino, hétérocyclyloxy, hétérocyclylthio ou hétérocyclylamino dont la partie hétérocyclyle est aromatique, il peut être préférable de construire la chaîne étapes par étapes en condensant d'abord une chaîne HO-alk-X pour laquelle X est un atome d'halogène, de préférence l'iode, dans les conditions décrites ci-dessus pour la réaction du produit de formule générale (IIa), puis en transformant la chaîne hydroxyalcoyle en une chaîne halogénoalcoyle, méthanesulfonylalcoyle ou p.toluènesulfonylalcoyle et enfin en faisant agir en milieu basique un dérivé aromatique de structure Ar-ZH pour lequel Ar est un radical phényle ou hétérocyclyle aromatique et Z est un atome de soufre, d'oxygène ou d'azote.

La transformation de la chaîne hydroxylée en chaîne halogénoalcoyle ou p.toluènesulfonyle s'effectue selon les méthodes habituelles d'halogénation ou de sulfonylation, notamment on fait agir un agent d'halogénation comme le chlorure de thionyle, les dérivés halogénés du phosphore : trichlorure ou tribromure de phosphore par exemple) ou un agent de sulfonylation comme par exemple le chlorure de méthanesulfonyle, le chlorure de p.toluènesulfonyle ou l'anhydride trifluorométhane-sulfonique. La réaction s'effectue dans un solvant organique comme un solvant chloré (dichlorométhane ou chloroforme par exemple), à une température comprise entre 0 et 60°C. Dans certains cas il peut être avantageux d'opérer en présence d'une base comme la pyridine ou la triéthylamine.

La réaction du dérivé aromatique Ar-ZH s'effectue avantageusement comme décrit précédemment pour l'action du dérivé de formule générale (IIa), en présence d'une base comme une base azotée par exemple, dans un solvant organique tel qu'un amide (diméthylformamide par exemple), une cétone (acétone par exemple) ou un nitrile (acétonitrile par exemple) en présence d'une base telle qu'une base organique azotée (par exemple triéthylamine) ou une base minérale (carbonate alcalin : carbonate de potassium par exemple) à une température comprise entre 20°C et la température de reflux du mélange réactionnel. Il peut être avantageux d'opérer en présence d'iodure de potassium.

Les dérivés quinolylalcoyl pipéridine de formule générale (II) peuvent être préparés selon la méthode de condensation décrite ci-après, puis le cas échéant transformés selon l'une des méthodes ① à ⑥ suivantes, par opération subséquente à partir de l'un des dérivés de formule générale (II) déjà obtenu, pour préparer les dérivés répondant aux différentes alternatives de Y et/ou de R₁, R'₁, R'₂, R'₃, R'₄ ou R'₅.

Il est entendu que lorsque des radicaux acide carboxylique sont présents sur la molécule, ces derniers sont préalablement protégés puis libérés après la réaction selon les méthodes habituelles qui n'altèrent pas le reste de la molécule, notamment selon les méthodes mentionnées dans les références citées précédemment. Il est également entendu que préalablement aux réactions pouvant interférer avec l'amine de la pipéridine du dérivé de formule générale (II), cette dernière est protégée, puis libérée après la réaction. La protection s'effectue selon les méthodes habituelles, comme précisé ci-dessus, notamment par un radical t.butoxycarbonyte ou benzyloxycarbonyle.

La préparation des produits de formule générale (II) pour lesquels Re dans Y est un atome d'hydrogène s'effectue par condensation d'un dérivé hétérocyclique de formule générale : 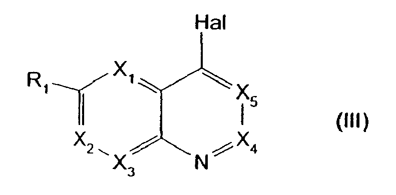 dans laquelle R₁, X₁, X₂, X₃, X₄ et X₅ sont définis comme précédemment et Hal représente un atome d'halogène, sur un dérivé de la pipéridine de formule générale : 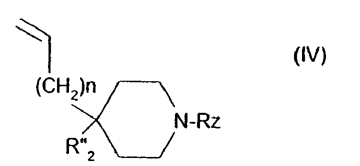 dans laquelle Rz est un radical protecteur et R"₂ est défini comme précédemment ou représente un radical protégé si R₂ représente ou porte une fonction acide carboxylique, suivie de l'élimination des radicaux protecteurs et/ou suivie de la transformation, par opération subséquente, des substituants du bicycle du dérivé hétérocyclylalcoyl pipéridine de formule générale (II) ainsi obtenu, pour conduire au dérivé portant le radical R₁, R'₁, R'₂, R'₃, R'₄, R'₅ attendu, et le cas échéant élimination du/des radicaux protecteurs encore présents sur la molécule.

Le radical Rz peut être tout groupement protecteur de l'atome d'azote compatible avec la réaction (t-butyloxycarbonyle, benzyloxycarbonyle par exemple). Les groupements protecteurs des fonctions acides sont choisis parmi les groupements habituelles dont la mise en place et l'élimination n'affectent pas le reste de la molécule, notamment ceux mentionnés dans les références citées précédemment.

La réaction s'effectue par action successive d'un organoborane (9-borabicyclo[3,3,1]nonane par exemple) dans un solvant tel qu'un éther (tétrahydrofuranne, dioxanne par exemple) à une température comprise entre -20 et 20°C puis du dérivé bicyclique de formule générale (III) pour lequel Hal représente de préférence un atome de brome ou d'iode ou un atome de chlore, par analogie avec les méthodes décrites par Suzuki et al. Pure and Appl. Chem., 57, 1749 (1985). La réaction s'effectue généralement en présence d'un sel de palladium (chlorure de palladium diphénylphosphinoférrocène par exemple) et d'une base comme le phosphate de potassium à une température comprise entre 20°C et la température de reflux du solvant.

① Les quinolylalcoyl pipéridines de formule générale (11) pour lesquelles Re dans Y représente un radical hydroxy, peuvent être préparées par oxydation en milieu basique du dérivé correspondant hétérocyclylalcoyl pipéridine de formule générale (II) pour lequel Re dans Y est un atome d'hydrogène. L'oxydation s'effectue par action de l'oxygène, de préférence au sein d'un solvant inerte tel que le diméthylsulfoxyde en présence de tert-butanoi et d'une base telle le tert-butylate de potassium ou de sodium à une température comprise entre à 0 et 100°C.

Le dérivé quinolylalcoyl pipéridine pour lequel Re dans Y est un atome de fluor, est préparé par fluoration à partir du dérivé pour lequel Re est hydroxy. La réaction est mise en oeuvre en présence d'un fluorure de soufre [par exemple en présence d'un trifluorure d'aminosoufre (trifluorure de diéthylamino soufre (Tetrahedron, 44, 2875 (1988), trifluorure de bis(2-méthoxyéthyl)amino soufre (Deoxofluor® ), trifluorure de morpholino soufre par exemple) ou alternativement en présence de tétrafluorure de soufre (J. Org. Chem., 40, 3808 (1975)] ; alternativement la réaction de fluoration peut aussi s'effectuer au moyen d'un agent de fluoration comme fhexafluoropropyl diéthylamine (JP 2 039 546) ou la N-(chloro-2 trifluoro-1,1,2 éthyl) diéthylamine.
On opère dans un solvant organique tel qu'un solvant chloré (par exemple dichlorométhane, dichloréthane, chloroforme) ou dans un éther (tétrahydrofuranne, dioxanne par exemple) à une température comprise entre -78 et 40°C (de préférence entre 0 et 30°C). Il est avantageux d'opérer en milieu inerte (argon ou azote notamment).

Le dérivé quinolylalcoyl pipéridine de formule générale (II) pour lequel Re dans Y est un radical alcoyloxy ou cycloalcoyloxy, est préparé par action d'un halogénure d'alcoyle ou de cycloalcoyle sur le dérivé correspondant de formule générale (II) pour lequel Re est hydroxy. La réaction s'effectue généralement au moyen du bromure ou du chlorure, dans un solvant inerte tel que le N,N-diméthylformamide ou le diméthylsulfoxyde, en présence d'un accepteur d'acide tel qu'une trialkylamine (triéthylamine par exemple) ou un hydrure de métal alcalin (hydrure de sodium par exemple), à une température comprise entre 20 et 100°C.

Le dérivé quinolylalcoyl pipéridine de formule générale (II) pour lequel Y est un radical carbonyle peut être préparé par oxydation du dérivé correspondant de formule générale (II) pour lequel Re dans Y est un radical hydroxy. Cette oxydation s'effectue par exemple au moyen de permanganate de potassium éventuellement dans d'une solution de soude (par exemple soude 3N), à une température comprise entre -20 et 20°C, ou bien par action de chlorure d'oxalyle en présence de diméthylsulfoxyde, suivie de l'addition d'une amine telle la triéthylamine, dans un solvant inerte tel le dichlorométhane, le diméthylsulfoxyde à une température comprise entre -60 et 20°C par analogie avec la méthode décrite par D. SWERN et coll., J. Org. Chem., 44, 4148 (1979).

Le dérivé quinolylalcoyl pipéridine de formule générale (II) pour lequel Y est un radical difluorométhylène peut être préparé par dihalogénation du produit de formule générale (II) pour lequel Y est carbonyle dans des conditions analogues à celles de la fluoration décrite précédemment.

Le dérivé quinolylalcoyl pipéridine de formule générale (II) pour lequel Y est un radical hydroxyiminométhylène peut être préparé par action de l'hydroxylamine sur un dérivé de formule générale (II) pour lequel Y est un radical carbonyle. La réaction s'effectue généralement dans un solvant inerte tel qu'un alcool (éthanol, méthanol) et éventuellement en présence de soude (soude 1N par exemple) à une température comprise entre 0°C et la température d'ébullition du mélange réactionnel.

Le dérivé quinolylalcoyl pipéridine de formule générale (II) pour lequel Y est un radical alcoyloxyiminométhylène ou cycloalcoyloxyiminométhylène peut être préparé par action d'un halogénure d'alcoyle ou de cycloalcoyle sur le dérivé correspondant de formule générale (II) pour lequel Y est hydroxyiminométhylène. La réaction s'effectue généralement au sein d'un solvant inerte tel que la N,N-diméthylformamide ou le diméthylsulfoxyde, en présence d'un accepteur d'acide tel qu'une trialkylamine (triéthylamine par exemple) ou un hydrure de métal alcalin (hydrure de sodium par exemple), à une température comprise entre 20 et 100°C. De préférence on utilise un bromure.

Le dérivé quinolylalcoyl pipéridine de formule générale (II) pour lequel Re dans Y est un radical -NRaRb peut être préparé à partir du dérivé tosyloxy correspondant par action d'une amine HNRaRb (ou le cas échéant de l'ammoniac lorsque Re est -NH₂) dans un solvant inerte tel le N,N-diméthylformamide ou le diméthylsulfoxyde à une température comprise entre 20°C et la température d'ébullition du mélange réactionnel. Lorsque l'on fait agir l'ammoniac, on opère de préférence sous pression (2 à 20 atmosphères) à une température comprise entre 20 et 100°C. Lorsque l'on opère au moyen d'une amine HNRaRb, la réaction s'effectue éventuellement en présence d'une base comme une trialkylamine (triéthylamine par exemple), la pyridine ou un hydrure de métal alcalin (hydrure de sodium par exemple). Le dérivé pour lequel Re dans Y est tosyloxy est obtenu à partir du produit de formule générale (II) pour lequel Re dans Y est hydroxy, par action du chlorure de tosyle dans la pyridine, à une température comprise entre 10 et 20°C.

Le dérivé quinolylalcoyl pipéridine de formule générale (II) pour lequel Re dans Y est un radical carboxy peut être préparé par action d'un cyanure alcalin sur le dérivé tosyloxy correspondant dans un solvant organique comme le diméthylformamide ou le diméthylsulfoxyde, ou en milieu hydroorganique, par exemple un mélange eau-alcool, à une température comprise entre 0°C et la tempétature d'ébullition du mélange réactionnel, suivie de l'hydrolyse du nitrile obtenu par action d'un acide fort tel que l'acide chlorhydrique, éventuellement d'un alcool aliphatique inférieur, à une température comprise entre 0°C et la tempétature d'ébullition du mélange réactionnel. De préférence on utilise le cyanure de sodium ou de potassium.

Le dérivé quinolylalcoyl pipéridine de formule générale (II) pour lequel Re dans Y est un radical alcoyloxycarbonyle, cycloalcoyloxycarbonyle ou -CO-NRaRb peut être préparé par action respectivement de l'alcool ou de l'amine correspondant sur le dérivé de formule générale (II) pour lequel Re dans Y est un radical carboxy. La réaction s'effectue en présence d'un agent de condensation comme un carbodiimide (par exemple le N,N'-dicyclohexylcarbodiimide) ou le N,N'-diimidazolecarbonyle, dans un solvant inerte tel qu'un éther (tétrahydrofuranne, dioxanne par exemple), un amide (N,N-diméthylformamide), un solvant chloré (chlorure de méthylène, dichloro-1,2-éthane, chloroforme par exemple) ou le diméthylsulfoxyde à une température comprise entre 0°C et la température de reflux du mélange réactionnel.

② Le dérivé quinotylalcoyl pipéridine de formule générale (II) pour lequel Y est un radical cycloalcoylène-1,1 peut être préparé par action, en milieu basique, d'un produit de structure Hal-Alk-Hal pour lequel Alk est le radical alcoylène correspondant au cycloalcoylène attendu, sur un dérivé hétérocyclylalcoyl pipéridine pour lequel Re dans Y est un atome d'hydrogène. La réaction s'effectue généralement dans un solvant inerte tel que le N,N-diméthylformamide ou le diméthylsulfoxyde, en présence d'un accepteur d'acide tel qu'un hydrure de métal alcalin (hydrure de sodium par exemple), à une température comprise entre 20 et 100°C.

③ Les dérivés quinolylalcoyl pipéridine de formule générale (II) pour lesquels l'un de R₁, R'₁, R'₂, R'₃, R'₄, R'₅ représente un radical alcoyle peuvent être préparés par action d'un dérivé du bore de structure R'ᵢB(OH)₂ (R'i signifiant l'un des substituants R₁, R'₁, R'₂, R'₃, R'₄ ou R'₅) ou de structure 9-alcoyl -9-borabicyclo [3.3.1]nonane sur un dérivé de formule générale (II) pour lequel le substituant R'ᵢ est un atome de brome, d'iode ou de chlore par analogie avec les méthodes décrites par F. DIEDERICH et P. J. STANG, Metal Catalysed Cross-coupling Reactions, Wiley-VCH, (1997) en présence d'un sel de palladium (par exemple le tétrakis triphénylphosphine ou le chlorure de palladium diphénylphosphino ferrocène) et d'une base comme le phosphate de potassium, dans un solvant inerte tel qu'un amide (N,N-diméthylformamide par exemple), un éther (tétrahydrofuranne par exemple) ou un nitrile (acétonitrile par exemple), à une température comprise entre 20°C et la température de reflux du mélange réactionnel.

④ Les dérivés quinolylalcoyl pipéridine de formule générale (II) pour lesquels l'un de R₁, R'₁, R'₂, R'₃, R'₄, R'₅ représente un radical alcoyloxy, peuvent être préparés par action de l'alcool correspondant sur le dérivé hétérocyclylalcoyl pipéridine pour lequel l'un des R'ᵢ est un atome de brome, d'iode ou de chlore. La réaction s'effectue généralement dans un solvant inerte tel qu'un éther (tétrahydrofuranne, dioxanne par exemple), un amide (N,N-diméthylformamide par exemple) ou le diméthylsulfoxyde, en présence d'un accepteur d'acide tel qu'une trialkylamine (triéthylamine par exemple), un hydrure de métal alcalin (hydrure de sodium par exemple), le méthyllithium ou le n-butylithium à une température comprise entre 0°C et la température de reflux du mélange réactionnel.

Les dérivés quinolylalcoyl pipéridine de formule générale (II) pour lesquels l'un de R₁, R'₁, R'₂, R'₃, R'₄, R'₅ représente un radical hydroxy peuvent être préparés à partir du dérivé correspondant pour lequel l'un des R'i est méthoxy par action d'un acide fort tel que l'acide bromhydrique à une température comprise entre 20°C et la température de reflux du mélange réactionnel.

Les dérivés quinolylalcoyl pipéridine de formule générale (II) pour lesquels l'un de R₁, R'₁, R'₂, R'₃, R'₄, R'₅ représente un radical alcoyloxy, peuvent être obtenus par action du dérivé halogéné correspondant sur le dérivé de formule générale (II) pour lequel le R'ᵢ à modifier est hydroxy. On opère de préférence au moyen du dérivé bromé, dans un solvant inerte tel qu'un éther (tétrahydrofuranne, dioxanne par exemple), un amide (N,N-diméthylformamide par exemple) ou diméthylsulfoxyde, en présence d'un accepteur d'acide tel qu'une trialkylamine (triéthylamine par exemple), un hydrure de métal alcalin (hydrure de sodium par exemple), le méthylithium, n-bulyllithium éventuellement en présence d'un sel de palladium [par analogie avec les méthodes décrites dans J. Am. Chem. Soc., 4369 (1999) ; Tetrahedron Lett., 8005 (1997) ; Angew. Chem. Int. Ed. Engl., 2047 (1998)], à une température comprise entre 0°C et la température de reflux du mélange réactionnel.

⑤ Les dérivés quinolylalcoyl pipéridine de formule générale (II) substitués par un radical cyano peuvent être obtenus à partir du dérivé correspondant pour lequel l'un des R'ᵢ est un atome d'halogène de préférence un atome de brome ou d'iode par application des méthodes décrites par HALLEY F. et coll., Synth. Comm., 27, 7, 1199 (1997) et TSCHAEN D. M. et coll., J. Org. Chem., 60, 14, 4324 (1995), en présence de CuCN, de KCN éventuellement en présence d'un catalyseur.

Les dérivés quinolylalcoyl pipéridine de formule générale (II) substitués par un radical carboxy, peuvent être préparés à partir du dérivé cyano, selon les méthodes habituelles de transformation en un acide, en un ester et en un amide, qui n'affectent pas le reste de la molécule et dont certaines des conditions de mise en oeuvre ont été rappelées ci-avant. Notamment, en présence d'un carbodiimide (N,N'-dicyclohexylcarbodiimide) ou le N,N'-diimidazole carbonyle, dans un éther (tétrahydrofuranne, dioxanne), un amide (N,N-diméthylformamide) ou un solvant chloré (chlorure de méthylène, dichloro-1,2-éthane, chloroforme) à une température comprise entre 0°C et la température de reflux du mélange réactionnel.

⑥ Les dérivés quinolylalcoyl pipéridine de formule générale (II) substitués par un radical hydroxyméthyle peuvent être obtenus par réduction d'un dérivé hétérocyclylalcoyl pipéridine de formule générale (II) pour lequel l'un des R'ᵢ représente un radical carboxy, au moyen d'un agent de réduction comme par exemple l'hydrure de lithium et d'aluminium, un borohydrure dans d'un solvant inerte tel qu'un éther (tétrahydrofuranne, dioxanne par exemple) ou un solvant chloré (chlorure de méthylène, dichloro-1,2-éthane, chloroforme par exemple) à une température comprise entre 0°C et la température de reflux du mélange réactionnel.

Les dérivés quinolylalcoyl pipéridine de formule générale (II) pour lesquels l'un de R₁, R'₁, R'₂, R'₃, R'₄, R'₅ représente un radical alcoyloxyméthyle peuvent être obtenus par action du dérivé halogéné correspondant (de préférence le dérivé brome) sur le dérivé quinolylalcoyl pipéridine correspondant pour lequel le R'ᵢ représente un radical hydroxyméthyle. La réaction s'effectue généralement dans un solvant inerte tel qu'un éther (tétrahydrofuranne, dioxanne par exemple), un amide (N,N-diméthylformamide par exemple) ou le diméthylsulfoxyde, en présence d'un accepteur d'acide tel qu'une trialkylamine (triéthylamine par exemple) ou un hydrure de métal alcalin (hydrure de sodium par exemple), à une température comprise entre 20 et 100°C.

Les dérivés hétérocycliques de formule générale (III) pour lesquels Hal est un atome de brome, R₁ est défini comme précédemment, X₁ à X₄ représentent respectivement >C-R'₁ à >C-R'₄ et X₅ représente >CH, peuvent être préparés par bromation des 4-hydroxyquinoléines correspondantes au moyen d'un agent de bromation comme l'oxytribromure de phosphore ou le bromure de thionyle sans solvant, à une température comprise entre 20 et 115°C.
Les 4-hydroxyquinoléines peuvent être préparées par décarboxylation des 3-carboxy-4-hydroxyquinoléines correspondantes en opérant dans un solvant comme le diphényléther à une température comprise entre 100 et 260°C.
Les 3-carboxy-4-hydroxyquinoléines peuvent être préparées par analogie avec la méthode décrite dans la demande de brevet européen EP 379 412 à partir du dérivé d'aniline souhaité.

Les dérivés hétérocycliques de formule générale (III) pour lesquels Hal est un atome de brome, R₁ est défini comme précédemment, X₁ à X₄ représentent respectivement >C-R'₁ à >C-R'₄ et X₅ représente >C-Cl, peuvent être préparés par bromation de la 3-chloro-4-hydroxy-quinoléine correspondante. La bromation s'effectue généralement avec du dibromure de triphénylphosphine dans l'acétonitrile à une température comprise entre 20 et de 85°C.
Les 3-chloro-4-hydroxy-quinoléines peuvent être préparées par chloration d'une 4-hydroxyquinoléines. La chloration s'effectue par exemple au moyen de N-chlorosuccinimide dans un solvant comme l'acide acétique à une température comprise entre 20 et 100°C.

Les dérivés hétérocycliques de formule générale (III) pour lesquels Hal est un atome d'iode, R₁ est un radical méthoxy, X₁ à X₄ représentent respectivement >C-R'₁ à >C-R'₄ et X₅ représente >C-F, peuvent être préparés par analogie avec les travaux de E. Arzel et al., Tetrahedron, 55, 12149-12156 (1999) à partir de 3-fluoro-6-méthoxyquinoléine, par action successive d'une base puis de l'iode. On utilise par exemple le diisopropylamidure de lithium dans un solvant tel qu'un éther (tétrahydrofuranne) à une température comprise entre -80 et 20°C.
La 3-fluoro-6-méthoxyquinoléine peut être obtenue par pyrolyse du 3-tétrafluoroborate ou du 3-hexafluorophosphate de diazonium de 6-méthoxyquinoléine selon la réaction de Balz-Schieman, Org. Synth., Coll 5, 133 (1973), à une température comprise entre 100 et 240°C. Le 3-tétrafluoroborate de diazonium de 6-méthoxyquinoléine ou le 3-hexafluorophophate de diazonium de 6-méthoxyquinoléine peuvent être obtenus à partir de la 3-amino-6-méthoxyquinoléine par action d'un nitrite alcalin (nitrite de sodium par exemple) en milieu acide (acide tétrafluoroborique ou acide hexafluorophophorique) dans un solvant tel que l'eau à une température comprise entre -10 et +20°C, par analogie avec les travaux de A. Roe et al., J. Am. Chem. Soc., 71, 1785-86 (1949) ou par action d'un nitrite d'alcoyle (comme par exemple le nitrite d'isoamyle) et du complexe de trifluoroborate diéthyléther dans un solvant tel qu'un éther (tétrahydrofuranne par exemple) à une température comprise entre -10 et +10°C.
La 3-amino-6-méthoxyquinoléine est préparée comme décrit par N. Heindel, J. Med. Chem. (1970) 13, 760.

Les composés de formule (III) dont les noms suivent :
- la 4-chloro-3-fluoro-6-méthoxy-quinoléine,
- la 4-bromo-3-fluoro-6-méthoxy-quinoléine,
- la 4-iodo-3-fluoro-6-méthoxy-quinoléine,
- la 4-bromo-3-chloro-6-méthoxy-quinoléine,
- la 4-lodo-3-ehloro-6-méthoxy-quinoléine,
ainsi que
- la 4-hydroxy-3-chloro-6-méthoxy-quinoléine et
- la 3-fluoro-6-méthoxy-quinoléine
sont des composés nouveaux et constituent l'objet de la présente demande.

Les dérivés de la pipéridine de formule générale (IV) pour lesquels n = 0 et R"₂ représente carboxy peuvent être préparés à partir du dérivé de pipéridine correspondant de formule générale: 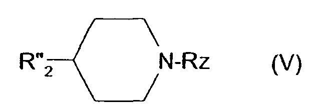 dans laquelle R"₂ défini comme ci-dessus est préalablement protégé et Rz est défini comme précédemment, par analogie avec les travaux de Koppel, J. Chem. Soc. Chem. Commun., 473 (1975) par réaction entre le dérivé de pipéridine avec le suffoxyde de vinyle et de phényle en présence d'une base (hydrure de sodium, diisopropytamidure de lithium ou hexaméthyldisyllyl amidure de lithium par exemple) dans un solvant tel qu'un éther (tétrahydrofuranne par exemple) à une température comprise entre 0 et 100°C. L'adduit intermédiaire obtenu est ensuite thermolysé entre 60 et 150°C dans un solvant inerte (chloroforme, tétrahydrofuranne, toluène ou xylène par exemple). Rz est avantageusement un groupement protecteur de l'atome d'azote comme t-butyloxycarbonyle par exemple.

Les dérivés de la pipéridine de formule générale (IV) pour lesquels n = 1 ou 2 et R"₂ représente carboxy peuvent être préparés à partir du dérivé de pipéridine correspondant de formule générale (V) pour lequel R"₂ est préalablement protégé, selon ou par analogie avec les méthodes décrites ci-après dans les exemples. Notamment on opère par action successive d'une base comme par exemple le diisopropylamidure de lithium ou le n-butyllithium, dans un solvant tel qu'un éther (tétrahydrofuranne par exemple) à une température comprise entre -80 et 0°C puis d'un halogénure d'alkényle (halogénure d'allyle ou halogéno-1-but-3-éne).

Les dérivés de la pipéridine de formule générale (IV) pour lesquels n est défini comme précédemment et R"₂ est alcoyloxycarbonyle, cycloalcoyloxycarbonyle, -CO-NRaRb, alcoyloxycarbonylméthyle, alcoyloxycarbonyléthyle, cycloalcoyloxycarbonylméthyle, cycloalcoyloxycarbonyléthyle, -CH₂-CONRaRb, -(CH₂)₂-CONRaRb ou pour lesquels Rc dans R₂ représente alcoyloxycarbonyle, cycloalcoyloxycarbonyle ou -CO-NRaRb peuvent être préparés à partir du dérivé acide carboxylique correspondant, selon les méthodes habituelles de transformation en ester ou en amide qui n'altèrent pas le reste de la molécule. La préparation des esters s'effectue en présence d'un agent de condensation tel qu'un carbodiimide (N,N'-dicyclocarbodiimide par exemple) ou le N,N'-carbonyldiimidazole dans un éther (tétrahydrofuranne ou dioxanne par exemple), un amide (diméthylformamide par exemple) ou un solvant chloré (dichlorométhane, dichloro-1,2-éthanc, chloroforme par exemple) à une température comprise entre 0°C et la température de reflux du mélange réactionnel. La préparation des amides s'effectue par action de l'amine correspondante dans les conditions identiques à celles décrites précédemment. Notamment aussi, lorsqu'il s'agit de la préparation d'un dérivé pour lequel Rc dans R"₂ est un ester, par analogie avec les méthodes décrites par Saha et al., J. Chem. Soc. Perkin 1, 505 (1985) par action d'un diazoalkane (diazométhane par exemple) dans un éther (diéthyléther par exemple) à une température comprise entre - 10 et 5°C.

Les dérivés de la pipéridine de formule générale (IV) pour lesquels n est défini comme précédemment et R"₂ représente cyano, -CH₂-CN ou -(CH₂)₂-CN peuvent être préparés à partir des amides correspondants par action d'un agent de déshydratation, par adaptation des méthodes décrites par Bieron et al., Zabicky « The chemistry of amides » Wiley, pp. 274-283 (1970). La réaction s'effectue en présence de pentoxyde de phosphore ou d'oxychlorure de phosphore avec ou sans solvant, à une température comprise entre 20°C et la température de reflux du mélange réactionnel.

Les dérivés de la pipéridine de formule générale (IV) pour lesquels n est défini comme précédemment et R"₂ représente hydroxyméthyle, cyanométhyle ou carboxyméthyle peuvent être préparés à partir du dérivé de pipéridine de formule générale : 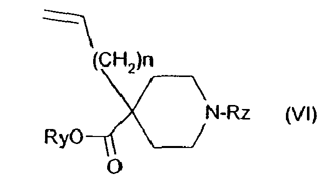 dans laquelle Rz et n sont définis comme précédemment, et Ry représente un radical protecteur facilement hydrolysable, notamment par action d'un hydrure (hydrure d'aluminium et de lithium ou hydrure de diisobutyl aluminium par exemple) dans un solvant tel qu'un éther (tétrahydrofuranne par exemple) à une température comprise entre 20 et 60°C pour préparer le dérivé de pipéridine pour lequel R"₂ est hydroxyméthyle, puis transformation du radical hydroxyméthyle en un radical cyanométhyle puis carboxyméthyle selon les méthodes habituelles qui n'altèrent pas le reste de la molécule.
Lorsque Ry représente un radical facilement hydrolysable il peut être notamment choisi parmi alcoyle (1 à 4 atomes de carbone en chaîne droite ou ramifiée), benzyle, cycloalcoyle, phénylpropyle ou allyle.
La transformation en acide peut notamment être mise en oeuvre à partir de ce dernier, par action d'un agent d'halogénation comme par exemple le chlorure de thionyle ou le trichlorure ou le tribromure de phosphore ou par action d'un chlorure d'alcanesulfonyle (chlorure de méthane sulfonyle ou chlorure de p-toluènesulfonyle par exemple) dans un solvant inerte (dichlororméthane par exemple) puis action d'un cyanure alcalin (cyanure de potassium ou cyanure de sodium par exemple) et hydrolyse. La réaction d'halogénation s'effectue dans un solvant chloré (dichlorométhane, chloroforme par exemple), à une température comprise entre 0°C et la température de reflux du mélange réactionnel. La réaction du cyanure alcalin peut être mise en oeuvre dans le diméthylsulfoxyde, un amide (diméthylformamide par exemple), une cétone (acétone par exemple), un éther (par exemple le tétrahydrofuranne) ou un alcool (par exemple méthanol ou éthanol), à une température comprise entre 20°C et la température de reflux du mélange réactionnel. L'hydrolyse du nitrile s'effectue selon les méthodes classiques qui n'altèrent pas le reste de la molécule, notamment par action de l'acide chlorhydrique en milieu méthanolique, à une température comprise entre 20 et 70°C, suivi de la saponification de l'ester obtenu (par exemple par l'hydroxyde de sodium dans un mélange de dioxanne et d'eau), ou bien directement par action de l'acide sulfurique aqueux à une température comprise entre 50 et 80°C.

Les dérivés de la pipéridine de formule générale (IV) pour lesquels n et Rz sont définis comme précédemment et R"₂ représente un radical 2-carboxyéthyle peuvent être préparés à partir du dérivé de formule générale (IV) pour lequel R"₂ représente un radical hydroxyméthyle par l'intermédiaire du dérivé halogéné (préparé comme décrit ci-dessus) puis condensation du sel de sodium du malonate de diéthyle suivie de l'hydrolyse acide en milieu aqueux du produit obtenu.

Les dérivés de la pipéridine de formule générale (IV) pour lesquels n et Rz sont définis comme précédemment et R"₂ représente carboxy hydroxy méthyle ou carboxycarbonyle peuvent être préparés par homologation du dérivé de pipéridine de formule générale : 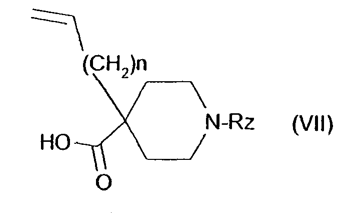 dans laquelle Rz et n sont définis comme précédemment, par application ou adaptation des méthodes décrites par M. Mizuno et al., Tetrahedron Lett. 39, 9209 (1998). La réaction s'effectue par action d'un phosphorocyanidate de dialkyle (phosphorocyanidate de diéthyle par exemple) en présence d'une base organique (triéthylamine par exemple) dans un éther (tétrahydrofuranne par exemple) à une température comprise entre -50 et 10°C. Le dicyanophosphonate intermédiairement obtenu est ensuite hydrolysé en milieu acide (acide chlorhydrique concentre par exemple) dans un solvant polaire (eau par exemple) à la température de reflux du mélange réactionnel. Les dérivés pour lesquels R"₂ est carboxycarbonyle sont obtenus par oxydation de l'ester correspondant, par adaptation des méthodes décrites par Burhardt et al., Tetrahedron Lett., 29, 3433 (1988) suivie de l'hydrolyse du produit obtenu. Notamment par action d'un oxydant comme le triacetoxy-1,1-dihydro-1,2-benziodoxol-3(1H)-one dans un solvant tel qu'un nitrile ou un dérivé chloré (acétonitrile ou dichlorométhane par exemple) à une température comprise entre 0 et 40°C, suivie de l'hydrolyse par action d'une base (hydroxyde de sodium par exemple) dans un solvant hydroalcoolique (eau - méthanol par exemple) à une température comprise entre 20 et la température de reflux du mélange réactionnel.

Les dérivés de la pipéridine de formule générale (IV) pour lesquels n et Rz sont définis comme précédemment et R"₂ représente -CF₂-Rc peuvent être préparés par action d'un agent de fluoration sur un dérivé de la pipéridine de formule générale (IV) pour lequel R"₂ est un radical -CO-Rc, Rc étant un ester, par analogie avec les méthodes décrites par M. Parisi et al., J. Org. Chem, 60, 5174 (1995), puis éventuellement hydrolyse de l'ester si l'on veut obtenir un dérivé de la pipéridine pour lequel Rc est carboxy. Les conditions de fluoration sont similaires à celles décrites précédemment pour la préparation de dérivés pour lesquels Re dans Y est un atome de fluor. L'hydrolyse s'effectue par action d'une base dans un solvant hydroalcoolique dans les conditions décrites ci-avant.

Les dérivés de la pipéridine de formule générale (IV) pour lesquels n et Rz sont définis comme précédemment et R"₂ représente un radical -CH=CH-Rc peuvent être préparés par oxydation en aldéhyde du dérivé de formule générale (IV) pour lequel R"₂ représente un radical hydroxyméthyle par adaptation des méthodes décrites dans Org. Synth. Coll., Vol. II, p. 541, Coll. Vol. 5 p. 242 puis transformation en un dérivé pour lequel R"₂ est -CH=CH-Rc où Rc est un ester par application de la méthode de Wittig puis éventuellement hydrolyse de l'ester obtenu en un acide. L'oxydation s'effectue par action d'un oxydant (bichromate de potassium par exemple) en milieu acide (acide sulfurique par exemple) dans un solvant polaire (eau par exemple) ou oxyde de chrome en présence d'une base (pyridine par exemple) dans un solvant chloré (dichlorométhane par exemple) à une température comprise entre 0 et 20°C. La transformation en un dérivé insaturé s'effectue par adaptation des méthodes décrites par Johnson dans « Ylid Chemistry » Academic Press (1966) par action d'un ylure de phosphore (carbéthoxymethylène triphénylphosphorane par exemple) dans un hydrocarbure (toluène par exemple) à une température comprise entre 60°C et la température de reflux du mélange réactionnel. L'hydrolyse s'effectue selon les méthodes décrites précédemment.

Les dérivés de la pipéridine de formule générale (IV) pour lesquels n et Rz sont définis comme précédemment et R"₂ représente un radical C(CH₃)₂Rc ou -C(cycloalk)Rc peuvent être préparés à partir d'un dérivé de formule générale (IV) pour lequel R"₂ est un ester de l'acide pour lequel R"₂ est -CH₂COOH par adaptation des méthodes décrites par Ashutosh et al., Tetrahedron Lett., 40, 4733 (1999) et Sauers, J. Org. Chem., 57, 671 (1992) puis hydrolyse éventuelle de l'ester obtenu. La réaction s'effectue notamment par action successive d'un amidure (diisopropylamidure de lithium par exemple puis d'un halogénure de méthyle (iodure de méthyle par exemple) ou d'un dérivé de formule Hal-Alk-Hal (Hal étant de préférence un atome de brome) dans un solvant polaire (hexaméthylphosphorotriamide par exemple) à une température comprise entre 0 et 60°C.

Il est entendu que les procédés énoncés ci-dessus pour la préparation des dérivés de pipéridine de formule générale (IV) peuvent également être appliqués aux dérivés de formule générale (II) si l'on préfère condenser en premier lieu la pipéridine sur le dérivé hétérocyclique de formule générale (III), puis transformer le radical R₂ dans les conditions décrites ci-avant.
Il est également entendu que les méthodes décrites ci-après dans les exemples font également partie de la présente invention.

Il est entendu que les dérivés de formule générale (I) et (II), peuvent exister sous des formes énantiomères ou diastéréoisomères ou sous forme syn ou anti. Les formes énantiomères ou diastéréoisomères et syn ou anti et leurs mélanges entrent aussi dans le cadre de la présente invention. Ces formes peuvent être séparés selon les méthodes habituelles. Notamment par chromatographie sur silice ou par Chromatographie Liquide Haute Performance (CLHP).

Les dérivés hétérocyclylalcoyl pipéridine de formule générale (1) peuvent être purifiés le cas échéant par des méthodes physiques telles que la cristallisation ou la chromatographie.

Les dérivés hétérocyclylalcoyl pipéridine de formule générale (I) peuvent être le cas échéant transformés en sels d'addition avec les acides, par les méthodes connues.

Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Certains des dérivés hétérocyclylalcoyl pipéridine de formule générale (1) portant un radical carboxy peuvent être transformés à l'état de sels métalliques ou en sels d'addition avec les bases azotées selon les méthodes connues en soi.

Les sels peuvent être obtenus par action d'une base métallique (par exemple alcaline ou alcalino-terreuse), de l'ammoniac ou d'une amine, sur un produit selon l'invention, dans un solvant approprié tel qu'un alcool, un éther ou l'eau, ou par réaction d'échange avec un sel d'un acide organique. Le sel formé précipite après concentration éventuelle de la solution, il est séparé par filtration, décantation ou lyophilisation. Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl- -phénéthylamine, NN'-dibenzyléthylènediamine, diphénylènediamine, benzhydrylamine, quinine, choline, arginine, lysine, leucine, dibenzylamine).

Les dérivés hétérocyclylalcoyl pipéridine de formule (I) sont des agents antibactériens particulièrement intéressants.

In vitro, les dérivés hétérocyclylalcoyl pipéridine de formule (I)se sont montrés actifs sur germes gram positifs à des concentrations comprises entre 0,03 et 4 µg/ml sur *Staphylococcus aureus* AS5155 résistante à la méticilline, ainsi que la majorité d'entre eux à des concentrations comprises entre 0,03 et 8 µg/ml sur *Streptococcus pneumoniae* 6254-Ol ; ils se sont également montrés actifs sur les germes à gram négatifs comme par exemple, et à titre non limitatif, sur *Moraxella catarrhalis* IPA 152, à des concentrations comprises entre 0,12 et 64 µg/ml. In vivo, ils se sont montrés actifs sur les infections expérimentales de la souris à *Staphylococcus aureus* IP8203 soit à des doses comprises entre 18 et 150 mg/kg par voie sous cutanée (DC₅₀), soit à des doses comprises entre 20 et 150 mg/kg par voie orale.

Enfin, les produits de formule (I)sont particulièrement intéressants du fait de leur faible toxicité. Aucun des produits n'a manifesté de toxicité à la dose de 100 mg/kg par voie sous cutanée chez la souris (2 administrations).

Dans la formule générale (I), les produits pour lesquels
X₁, X₂, X₃, X₄ et X₅ représentent respectivement >C-R'₁ à >C-R'₅,
R₁, R'₁, R'₂, R'₃, R'₄, R'₅, sont identiques ou différents et représentent un atome d'hydrogène ou d'halogène ou un radical alcoyle, alcoyloxy, ou représentent un radical méthylène substitué par alcoyloxy
R₂ représente carboxy, alcoyloxycarbonyle, -CONRaRb (pour lequel Ra représente un atome d'hydrogène et Rb représente un atome d'hydrogène ou un radical hydroxy) ou R₂ représente hydroxyméthyle, alcoyle contenant 1 ou 2 atomes de carbone substitué par carboxy, alcoyloxycarbonyle,
R₃ représente un radical alk-R°₃ pour lequel alk est un radical alcoyle et R°₃ représente hydrogène, cycloalcoyle, cycloalcoylthio, phényle, phénoxy, phénylthio, phénylamino, hétérocyclyloxy, hétérocyclylthio ou bien R°₃ représente -CR'b=CR'c-R'a pour lequel R'a représente phényle, et pour lequel R'b et R'c représentent hydrogène,
Y représente un radical >CH-Re pour lequel Re est hydrogène, fluoro ou hydroxy, n est un nombre entier de 2 à 3
étant entendu que les radicaux ou portions phényle ou hétérocyclyle mentionnés ci-dessus peuvent être éventuellement substitués sur le cycle par 1 à 4 halogènes, sont particulièrement intéressants,
notamment les dérivés de quinolylalcoyl pipéridine de formule générale (I) cités ci-après :
   ■ l'acide 4-[3-(3-chloro-6-méthoxyquinolin-4-yl)propyl]-1-[2-(thién-2-yl)thioéthyl]-pipéridine-4-carboxylique,
   ■ l'acide 4-[3-(3-chloro-6-méthoxyquinolin-4-yl)propyl]-1-[2-(3,5-difluorophénoxy)éthyl]-pipéridine-4-carboxylique,
   ■ l'acide 4-[3-(3-chloro-6-méthoxyquinolin-4-yl)-propyl]-1-(2-thiazol-2-thioéthyl)-pipéridin-4-carboxylique,
   ■ l'acide 1-(2-cyclopentylthio-éthyl)-4-[3-(3-fluoro-6-méthoxy-quinolin-4-yl)-propyl]-pipéridine-4-carboxylique,
   ■ l'acide 4-[3-(3-fluoro-6-méthoxy-quinolin-4-yl)-propyl]-1-(3-phényl-allyl)-pipéridine-4-carboxylique,
ainsi que leurs sels.

Les exemples suivants donnés à titre non limitatif illustrent la présente invention.

### Exemple 1

### Dichlorhydrate de l'acide-4-[3-(3-chloro-6-méthoxyquinolin-4-yl)propyl]-1-[2-(thién-2-yl)thioéthyl]-pipéridine-4-carboxylique.

Un mélange de 0,6 g de 4-[3-(3-chloro-6-méthoxyquinolin-4-yl)propyl]-1-[2-(thién-2-yl)thloéthyl]-pipéridine-4-carboxylate de benzyle dans 7,72 cm³ d'acide chlorhydrique aqueux 6N est porté à une température voisine de 100°C, sous agitation et sous atmosphère inerte pendant 2 heures. Après refroidissement à environ 20°C, le mélange réactionnel est concentré à sec sous pression réduite (5 kPa) à une température voisine de 40°C. Le résidu obtenu est repris dans 10 cm³ d'un mélange de dichlorométhane - méthanol (90/10 en volumes). Le mélange est concentré à sec dans les conditions ci-dessus. On obtient 0,58 g de dichlorhydrate de l'acide 4-[3-(3-chloro-6-méthoxyquinolin-4-yl)propyl]-1-[2-(thién-2-yl)thioéthyl]-pipéridine-4-carboxylique, sous forme d'une meringue de couleur beige fondant à 130°C en se décomposant.
Spectre de R.M.N. ¹H (250 MHz, (CD₃)₂SO d6 à une température de 373K, δ en ppm) : de 1,50 à 2,30 (mts : 8H en totalité) ; de 2,70 à 3,80 (mts : 10H en totalité) ; 3,99 (s : 3 H) ; 7,09 (dd, J = 5 et 3,5 Hz : 1H) ; 7,29 (d large, J = 3,5 Hz : 1H) ; 7,40 (d, J = 2,5 Hz : 1H) ; 7,46 (dd, J = 9 et 2,5 Hz : 1H) ; 7,64 (d large, J = 5 Hz : 1H) ; 7,99 (d, J = 9 Hz : 1H) ; 8,67 (s : 1H).

### 4-[3-(3-Chloro-6-méthoxyquinolin-4-yl)propyl]-1-[2-(thién-2-yl)thioéthyl]-pipéridine-4-carboxylate de benzyle.

Un mélange de 0,6 g de 4-[3-(3-chloro-6-méthoxyquinolin-4-yl)propyl]-pipéridine-4-carboxylate de benzyle, 0,36 g de 2-(2-bromoéthylthio)thlophène et 0,22 g de carbonate de potassium dans 20 cm³ d'acétonitrile est chauffé pendant 16 heures à une température voisine de 80°C, sous agitation et sous atmosphère inerte. Après refroidissement à une température voisine de 20°C, le mélange réactionnel est concentré sous pression réduite (5 kPa) à une température voisine de 40°C. On obtient un résidu que l'on purifie par chromatographie, sous une pression d'azote de 100 kPa, sur une colonne de gel de silice (granulométrie 40-63 µ ; diamètre 3,5 cm ; hauteur 28 cm), en éluant par un mélange de dichlorométhane-méthanol (97,5/2,5 en volumes) et en recueillant des fractions de 35 cm³. Les fractions 15 à 20 sont réunies puis concentrées à sec dans les conditions ci-dessus. On obtient 0,67 g de 4-[3-(3-chloro-6-méthoxyquinolin-4-yl)propyl]-1-[2-(thién-2-yl)thioéthyl]-pipéridine-4-carboxylate de benzyle, sous forme d'une huile visqueuse de couleur orange.

Spectre infra rouge (CCl₄) 2955 ; 1727 ; 1622 ; 1503 ; 1229 ; 1117 ; 833 et 698 cm⁻¹.

### 4-[3-(3-Chloro-6-méthoxyquinolin-4-yl)propyl]-pipéridine-4-carboxylate de benzyle.

A une solution de 2,05 g de 4-[3-(3-chloro-6-méthoxyquinolin-4-yl)propyl]-1-(*tert-*butyloxycarbonyl)-pipéridine-4-carboxylate de benzyle dans 50 cm³ de dichlorométhane, on ajoute sous agitation et sous atmosphère inerte, à une température voisine de 20°C, 1,27 cm³ d'acide trifluoroacétique. Au bout de 30 minutes, on ajoute à nouveau 1,27 cm³ d'acide trifluoroacétique, puis une nouvelle fois 1,27 cm³ au bout de 30 minutes supplémentaires. La réaction est complétée par une dernière addition de 1,27 cm³ d'acide trifluoroacétique. Après une heure, le mélange réactionnel est concentré sous pression réduite (5 kPa) à une température voisine de 40°C. Le résidu d'évaporation est repris dans 50 cm³ d'acétate d'éthyle et 20 cm³ d'eau. Après addition de 5 g de carbonate de potassium et agitation pendant 5 minutes, le mélange est décanté, la phase organique séparée, lavée par 2 fois 10 cm³ d'eau distillée, puis par 20 cm³ d'une solution aqueuse de chlorure de sodium à 10 % (en poids). Après séchage sur sulfate de magnésium, puis filtration, la solution organique est concentrée sous pression réduite (5 kPa) à une température voisine de 40°C. Le résidu obtenu est purifié par chromatographie, sous une pression d'azote de 100 kPa, sur une colonne de gel de silice (granulométrie 40-63 µ; diamètre 3,5 cm ; hauteur 30 cm), en éluant par un mélange de dichlorométhane-méthanol-ammoniaque à 32 % (89/10/1 en volumes), et en recueillant des fractions de 40 cm³. Les fractions 14 à 23 sont réunies puis concentrées à sec dans les conditions ci-dessus. On obtient 1,36 g de 4-[3-(3-chloro-6-méthoxyquinolin-4-yl)propyl]-pipéridine-4-carboxylate de benzyle, sous forme d'un solide de couleur beige fondant à 95°C.

Spectre infra rouge (KBr) 2960 ; 1721 ; 1621 ; 1503 ; 1232 ; 1115 ; 829 et 744 cm⁻¹.

### 4-[3-(3-Chloro-6-méthoxyquinolin-4-yl)propyl]-1-(tert-butyloxycarbonyl)-pipéridine-4-carboxylate de benzyle.

On refroidit à une température voisine de -30°C 1,98 g de 4-allyl-1-(*tert-*butyloxycarbonyl)-pipéridine-4-carboxylate de benzyle et l'on ajoute sous agitation et sous atmosphère inerte 11,32 cm³ d'une solution 0,5 M de 9-borabicyclo[3,3,1]nonane dans le tétrahydrofuranne. Après l'addition, la température du mélange est ramenée à environ 20°C. La solution obtenue est agitée pendant encore 4 heures, puis l'on ajoute 40 cm³ de dioxanne, 0,183 g de chlorure de palladium diphénylphosphino ferrocène, 2 g de 4-bromo-3-chloro-6-méthoxy quinoléine et 3,0 g de phosphate de potassium tribasique. Après 16 heures d'agitation à une température voisine de 60°C, le mélange réactionnel est refroidi au voisinage de 20°C puis filtré. L'insoluble est lavé par 3 fois 20 cm³ d'acétate d'éthyle, puis le filtrat et les eaux de lavages sont réunis, agités avec 40 cm³ d'eau et 100 cm³ d'acétate d'éthyle. La phase organique est décantée, lavée par 2 fois 20 cm³ d'eau, puis par 40 cm³ d'une solution aqueuse de chlorure de sodium à 10 % (en poids), séchée sur sulfate de magnésium, filtrée, concentrée sous pression réduite (5 kPa) à une température voisine de 40°C. Le résidu obtenu est purifié par chromatographie, sous une pression d'azote de 100 kPa, sur une colonne de gel de silice (granulométrie 40-63 µ ; diamètre 3,5 cm ; hauteur 30 cm), en éluant par un mélange de dichlorométhane - méthanol (98,5/1,5 en volumes), et en recueillant des fractions de 35 cm³. Les fractions 22 à 29 sont réunies puis concentrées dans les conditions ci-dessus. On obtient 2,09 g de 4-[3-(3-chloro-6-méthoxyquinolin-4-yl)propyl]-1-(*tert-*butyloxycarbonyl)-pipéridine-4-carboxylate de benzyle, sous forme d'une huile épaisse de couleur jaune.
Spectre infra rouge (CCl₄) 2930 ; 1728 ; 1695 ; 1622 ; 1503 ; 1230 ; 1172 ; 833 et 697 cm⁻¹.

### 4-Allyl-1-(tert-butyloxycarbonyl)-pipéridine-4-carboxylate de benzyle.

A une solution de 20 g d'acide 4-allyl-1-(*tert*-butyloxycarbonyl)-pipéridine-4-carboxylique dans 200 cm³ de diméthylformamide, on ajoute à une température voisine de 20°C, sous agitation et sous atmosphère inerte, 15,4 g de carbonate de potassium, puis 10,6 cm³ de bromure de benzyle. Le mélange est agité pendant 16 heures à environ 20°C, puis filtré. L'insoluble est lavé par 2 fois 100 cm³ d'acétate d'éthyle. Le filtrat et les eaux de lavages sont réunis, additionnés de 250 cm³ d'eau, puis l'ensemble est extrait par 1 fois 500 cm³ et 1 fois 150 cm³ d'acétate d'éthyle. Les extraits organiques sont réunis, lavés par 2 fois 125 cm³ d'une solution aqueuse à 10 % (en poids) de chlorure de sodium, séchés sur sulfate de magnésium, filtrés, concentrés sous pression réduite (5 kPa) à une température voisine de 40°C. On obtient une huile que l'on purifie par chromatographie, sous une pression de 100 kPa d'azote, sur une colonne de gel de silice (granulométrie 40-63 µ ; diamètre 7 cm ; hauteur 30 cm), en éluant par un mélange de dichlorométhane-méthanol (99/1 en volumes) et en recueillant des fractions de 200 cm³. Les fractions 6 à 16 sont réunies, concentrées à sec sous pression réduite (5 kPa), à une température voisine de 40°C. On obtient 25 g de 4-allyl-1-(*tert*-butyloxycarbonyl)-pipéridine-4-carboxylate de benzyle, sous forme d'un liquide de couleur jaune clair.
Spectre infra rouge (CH₂Cl₂) 2980 ; 1725 ; 1683 ; 1426 ; 1171 ; 1142 ; 974 et 924 cm⁻¹.

### Acide 4-allyl-1-(tert-butyloxycarbonyl)-pipéridine-4-carboxylique.

A un mélange de 48,52 g de *tert*-butylate de potassium dans 350 cm³ de tétrahydrofuranne, refroidi à une température voisine de 0°C, on ajoute sous agitation et sous atmosphère inerte 4,44 cm³ d'eau, puis 30,62 g de 4-allyl*-*1*-*(*tert-*butyloxycarbonyl)-pipéridine-4-carboxylate d'éthyle préalablement solubilisés dans 150 cm³ de tétrahydrofuranne. Après avoir laissé revenir la température au voisinage de 20°C, le mélange est agité pendant 24 heures à cette température. On ajoute 300 cm³ d'eau glacée au mélange réactionnel, puis le mélange est concentré à sec sous pression réduite (5 kPa) à une température voisine de 40°C. Le résidu aqueux est extrait par 300 cm³ d'éther diéthylique. Après un repos de 16 heures, la phase aqueuse est acidifiée à un pH voisin de 3-4 par addition d'environ 215 cm³ d'acide chlorhydrique aqueux, puis extraite par 3 fois 300 cm³ d'éther diéthylique. Les extraits éthérés sont réunis, séchés sur sulfate de magnésium, filtrés, concentrés à sec sous pression réduite (5 kPa), à une température voisine de 40°C. On obtient 26,1 g d'acide 4-allyl-1-(*tert*-butyloxycarbonyl)-pipéridine-4-carboxylique, sous forme d'un solide de couleur blanc cassé.
- Spectre de masse :: IE m/z=269 M⁺ m/z=168 (M-C₅H₉O₂)⁺ m/z=124(m/z=168 - CO₂)⁺ /z=57 C₄H₉⁺ pic de base

### Allyl-1-(tert-butylooycarbonyl)-pipéridine-4-carboxylate d'éthyle.

A 150 cm³ de tétrahydrofuranne refroidis à une température voisine de -70°C, on ajoute sous agitation et sous atmosphère inerte 70 cm³ d'une solution de butyllithium dans l'hexane (de concentration 2,5 M), puis 50 cm³ de tétrahydrofuranne et 23 cm³ de diisopropylamine préalablement solubilisés dans 300 cm³ de tétrahydrofuranne. Après une nouvelle addition de 50 cm³ de tétrahydrofuranne, le mélange est agité pendant 15 minutes à environ -70°C, puis on ajoute 45,15 g de 1-(*tert-*butyloxycarbonyl)isonipécotate d'éthyle préalablement solubilisés dans 400 cm³ de tétrahydrofuranne, et enfin 50 cm³ de ce même solvant. Après 1 heure d'agitation du mélange à une température voisine de -70°C, on ajoute 16,7 cm³ de bromure d'allyle préalablement solubilisés dans 150 cm³ de tétrahydrofuranne, puis le mélange est ramené aux environs de 20°C, agité pendant 17 heures. Le mélange est versé sur 200 cm³ d'une solution aqueuse saturée de chlorure d'ammonium, puis extrait par environ 2 litres d'acétate d'éthyle. Les extraits réunis sont séchés sur sulfate de sodium, filtrés, concentrés sous pression réduite (5 kPa) à une température voisine de 40°C. On obtient une huile que l'on purifie par chromatographie, sous une pression d'azote de 100 kPa, sur une colonne de gel de silice (diamètre 12 cm ; hauteur 50 cm), en éluant par un mélange de dichlorométhane - méthanol (99,5/0,5 en volumes), et en recueillant des fractions de 200 cm³ Les fractions 20 à 84 sont réunies, concentrées sous pression réduite (5 kPa) à une température voisine de 40°C. On obtient 27,85 g de 4-allyl-1-(*tert*-butyloxycarbonyl)-pipéridine-4-carboxylate d'éthyle, sous forme d'une huile de couleur jaune.
- Spectre de masse :: IE m/z=297 M⁺ m/z=240 (M - C₄H₉)⁺ m/z=196(m/z=240 - CO₂)⁺ m/z=168 (m/z=240 - CO₂Et)⁺ m/z=124 (m/z=168 - CO₂)⁺m/z=57 C₄H₉⁺pic de base

### 1-(tert-Butyloxycarbonyl)isonipécotate d'éthyle

A une solution de 100 g d'isonipécotate d'éthyle dans 1500 cm³ de dichlorométhane refroidie à une température voisine de 5°C, on ajoute en 1 heure, sous agitation et sous atmosphère inerte, 88,3 cm³ de triéthylamine, puis dans le même temps 166,6 g de di-*tert*-butyl dicarbonate préalablement solubilisés dans 300 cm³ de dichlorométhane. Le mélange réactionnel est agité pendant 16 heures en laissant revenir la température au voisinage de 20°C. Après une addition complémentaire de 41,6 g de di-*tert*-butyl dicarbonate dissous dans 70 cm³ de dichlorométhane, le mélange réactionnel est agité pendant 3 jours à environ 20°C, puis lavé par 2 fois 600 m³ d'une solution aqueuse saturée de chlorure de sodium, séché sur sulfate de sodium, filtré, concentré sous pression réduite (5 kPa) à une température voisine de 40°C. On obtient 171 g de 1-(*tert*-butyloxycarbonyl)isonipécotate d'éthyle, sous forme d'une huile de couleur brune.
Spectre de masse : DCI /*z*=275 MNH₄⁺ pic de base m/z=258 MH⁺

Le 2-(2-bromoéthylsulfanyl)thlophène peut être préparé selon SADYKHOV, K.I ; ALIEV, S.M et SEIDOV, M.M. Khim. Geterotsikl. Soedin, 3, 344-5 (1975).

### 4-Bromo-3-chloro-6-méthoxy quinoléine

Un mélange de 20 g de 3-chloro-4-hydroxy-6-méthoxy quinoléine dans 1000 cm³ d'acétonitrile additionnés de 80,8 g de dibromure de triphénylphosphine est agité pendant 2 heures 30 minutes à une température voisine de 85°C. La solution obtenue est refroidie au voisinage de 20°C, puis agitée pendant 16 heures à cette même température. Le mélange réactionnel est concentré sous pression réduite (5 kPa) à une température voisine de 40°C, puis le résidu d'évaporation est repris par 200 cm³ d'une solution aqueuse saturée d'hydrogénocarbonate de sodium et 200 cm³ d'acétate d'éthyle. La phase organique est décantée, lavée par 2 fois 200 cm³ d'eau distillée. La phase aqueuse est extraite encore 1 fois à l'acétate d'éthyle, puis les extraits organiques sont réunis, séchés sur sulfate de magnésium, filtrés, concentrés sous pression réduite (5 kPa) à une température voisine de 40°C. On obtient un produit que l'on purifie par chromatographie sous une pression d'azote de 100 kPa, sur une colonne de gel de silice (diamètre 7,5 cm ; masse de silice 700 g), en éluant par un mélange de cyclohexane - acétate d'éthyle (70/30 en volumes). On recueille les fractions correspondant au produit attendu. Celles-ci sont réunies puis concentrées dans les mêmes conditions que ci-dessus. On obtient 20,7 g de 4-bromo-3-chloro-6-méthoxy quinoléine, sous forme d'un solide blanc fondant à 108°C.
Spectre de masse : IE m/z=271 M⁺ pic de base m/z=256 (M-CH₃)⁺ m/z=228 (m/z=256 - CO)⁺ m/z=149 (m/z=228-Br)⁺ m/z=114(m/z=149 - Cl)⁺

### 3-Chloro-4-hydroxy-6-méthoxy quinoléine.

A un mélange de 17 g de 4-hydroxy-6-méthoxy quinoléine dans 700 cm³ d'acide acétique on ajoute à une température voisine de 20°C, et sous agitation, 14,26 g de N-chlorosuccinimide, puis on chauffe le mélange à une température comprise entre 50 et 70°C pendant 4 heures. La solution obtenue est ensuite refroidie aux environs de 20°C, puis concentrée à sec sous pression réduite (5 kPa) à une température voisine de 40°C. Le résidu solide est repris par 250 cm³ d'une solution aqueuse saturée d'hydrogénocarbonate de sodium. Le mélange est agité pendant 1 heure. L'insoluble est filtré, lavé par 3 fois 250 cm³ d'eau. Les cristaux obtenus sont séchés sous pression réduite (10 Pa) pendant 3 heures à une température voisine de 20°C. On obtient 20 g de 3-chloro-4-hydroxy-6-méthoxy quinoléine, sous forme d'un solide de couleur jaune.
Spectre de masse : IE m/z=209 M⁺ pic de base m/z=194 (M - CH₃)⁺ m/z=166 (m/z=194 - CO)⁺.

### 4-Hydroxy-6-méthoxy quinoléine

Une suspension de 53,5 g d'acide 4-hydroxy-6-méthoxy quinoléine-3-carboxylique dans 1000 cm³ de diphényle éther est chauffée sous agitation à une température comprise entre 250 et 260°C pendant 2 heures 45 minutes. Le mélange réactionnel est refroidi aux environs de 20°C. Après 16 heures d'agitation à cette température, le mélange est versé sous agitation sur 1 litre de pentane, puis filtré. Le gâteau obtenu est lavé par 3 fois 100 cm³ de pentane, puis 3 fois 100 cm³ d'éther diisopropylique. On obtient, après séchage à l'air, 37 g de 4-hydroxy-6-méthoxy quinoléine, sous forme d'un solide de couleur beige.
Spectre de masse : DCI m/z=176 MH⁺ pic de base

L'acide 4-hydroxy-6-méthoxy quinoléine-3-carboxylique peut être préparé selon B.R. BAKER et RAY R. BRAMHALL, J. Med. Chem. 15, 230 (1972).

### Exemple 2

### Acide 4-[3-(3-chloro-6-méthoxyquinolin-4-yl)propyl]-1-(2-(3,5-difluorophénoxy) éthyl)-pipéridine-4-carboxylique

Un mélange de 0,7 g de 4-[3-(3-chloro-6-méthoxyquinolin-4-yl)propyl]-1-[2-(3,5-difluorophénoxy) éthyl]-pipéridine-4-carboxylate de benzyle dans 10 cm³ d'acide chlorhydrique aqueux 5M est agité pendant 5 heures à une température voisine de 100°C. Après refroidissement aux environs de 20°C, le mélange réactionnel est concentré à sec sous pression réduite (5 kPa) à une température voisine de 40°C. Le résidu obtenu est purifié par chromatographie sous une pression d'azote de 100 kPa, sur une colonne de gel de silice (granulométrie 40-63 µ ; diamètre 2,5 cm ; hauteur 35 cm), en éluant par un mélange de dichlorométhane - méthanol - ammoniaque à 28 % (89/10/1 en volumes) et en recueillant des fractions de 25 cm³. On recueille les fractions 16 à 25. Celles-ci sont réunies puis concentrées dans les conditions ci-dessus. Le résidu d'évaporation obtenu est trituré dans 10 cm³ d'éther düsopropylique. Le produit cristallisé résultant est filtré, lavé par 2 fois 5 cm³ du même solvant, séché à l'air. On obtient 0,37 g d'acide 4-[3-(3-chloro-6-méthoxy quinolin-4-yl)propyl]-1-[2-(3,5-difluorophénoxy)éthyl]-pipéridine-4-carboxylique, sous forme d'un solide blanc fondant à 204°C.
Spectre de R.M.N. ¹H (300 MHz, (CD₃)₂SO d6, δ en ppm) : de 1,20 à 2,15 (mt : 10H) ; 2,62 (t, J = 5,5 Hz : 2H) ; de 2,65 à 2,80 (mt : 2H) ; 3,18 (mt : 2H) ; 3,96 (s : 3H) ; 4,08 (t, J = 5,5 Hz : 2H) ; de 6,60 à 6,85 (mt : 3H) ; 7,38 (d, J = 2,5 Hz : 1H) ; 7,46 (dd, J = 9 et 2,5 Hz : 1H) ; 7,97 (d, J = 9 Hz : 1H) ; 8,68 (s : 1H).

### 4-[3-(3-Chloro-6-méthoxyquinolin-4-yl)propyl]-1-[2-(3,5-difluorophénoxy)éthyl]-pipéridine-4-carboxylate de benzyle

Une suspension composée de -1,36 g de 4-[3-(3-chloro-6-méthoxyquinolin-4-yl)propyl]-pipéridine-4-carboxylate de benzyle, 0,95 g de 1-(2-bromoéthoxy)-3,5-difluorobenzène (à 90 % de pureté), 0,5 g de carbonate de potassium dans 45 cm³ d'acétonitrile est chauffée à une température voisine de 80°C pendant 16 heures, sous agitation et sous atmosphère inerte. Après refroidissement à une température voisine de 20°C, le mélange réactionnel est filtré, l'insoluble lavé par de l'acétonitrile. Le filtrat et les eaux de lavage sont réunis, concentrés sous pression réduite (5 kPa) à une température voisine de 40°C. Le résidu d'évaporation est purifié par chromatographie sous une pression d'azote de 100 kPa, sur une colonne de gel de silice (granulométrie 40-63 µ ; diamètre 3,5 cm ; hauteur 45 cm), en éluant par un mélange de dichlorométhane - méthanol (97/3 en volumes), et en recueillant des fractions de 40 cm³. On recueille les fractions 18 à 23. Celles-ci sont réunies puis concentrées sous pression réduite (5 kPa) à une température voisine de 40°C. On obtient 1,56 g de 4-[3-(3-chloro-6-méthoxy quinolin-4-yl)propyl]-1-[2-(3,5-difluorophénoxy)éthyl]-pipéridine-4-carboxylate de benzyle, sous forme d'une huile orangée.
Spectre infra rouge (CH₂Cl₂) : 2955 ; 1723 ; 1622 ; 1599 ; 1229 ; 1153 ; 1116 et 843 cm⁻¹.

Le 4-[3-(3-chloro-6-méthoxyquinolin-4-yl)propyl]-pipéridine-4-carboxylate de benzyle a été préparé dans l'exemple 1.

### Exemple 3

### Acide 4-[3-(3-chloro-6-méthoxyquinolin-4-yl)propyl]-1-(2-cyclohexyléthyl)-pipéridine-4-carboxylique

Un mélange de 0,6 g de 4-[3-(3-chloro-6-méthoxyquinolin-4-yl)propyl]-1-(2-cyclohexyléthyl)-pipéridine-4-carboxylate de benzyle dans 9,6 cm³ d'acide chlorhydrique aqueux 5M est chauffé à une température voisine de 100°C, sous agitation pendant 5 heures. La solution obtenue est concentrée sous pression réduite (5 kPa) à une température voisine de 40°C. Le résidu d'évaporation obtenu est trituré dans l'éther diisopropylique. Le produit cristallisé résultant est filtré, lavé par le même solvant, séché à l'étuve à une température voisine de 60°C, sous pression réduite (10 Pa). On obtient un solide que l'on purifie par chromatographie sous une pression d'azote de 100 kPa, sur une colonne de gel de silice (granulométrie 40-63 µ ; diamètre 2,5 cm ; hauteur 40 cm), en éluant par un mélange de dichlorométhane - méthanol - ammoniaque à 32 % (89/10/1 en volumes), et en recueillant des fractions de 25 cm³. Les fractions 16 à 25 sont réunies, puis concentrées sous pression réduite (5 kPa) à une température voisine de 40°C. On obtient un produit cristallisé que l'on agite dans 5 cm³ d'éther diisopropylique. Le produit obtenu est filtré, lavé par le même solvant, séché à l'air. On obtient 0,33 g d'acide 4-[3-(3-chloro-6-méthoxyquinolin-4-yl)propyl]-1-(2-cyclohexyléthyl)-pipéridine-4-carboxylique, sous forme d'un solide blanc fondant à 234°C.
Spectre de R.M.N.¹H (300 MHz, CD₃OD d4, δ en ppm) : de 0,95 à 2,20 - 2,42 - de 2,90 à 3,15 et de 3,30 à 3,50 (respectivement mt , d large J = 13,5 Hz , mt et mt : 29H en totalité) ; 4,11 (s : 3H) ; 7,52 (mt : 2H) ; 8,02 (d large, J = 9 Hz : 1H) ; 8,68 (s : 1H).

### 4-[3-(3-Chloro-6-méthoxyquinolin-4-yl)propyl]-1-(2-cyclohexyléthyl)-pipéridine-4-carboxylate de benzyle

A une solution de 1,36 g de 4-[3-(3-chloro-6-méthoxyquinolin-4-yl)propyl]-pipéridine-4-carboxylate de benzyle dans 50 cm³ d'acétonitrile, on ajoute sous agitation et sous atmosphère inerte, à une température voisine de 20°C, 0,56 cm³ de bromure de 2-cyclohexyléthyle et 0,5 g de carbonate de potassium. La suspension obtenue est chauffée aux environs de 80°C pendant 16 heures, puis après refroidissement à une température voisine de 20°C, le mélange réactionnel est filtré, concentré à sec sous pression réduite (5 kPa) à une température voisine de 40°C. Le résidu obtenu est purifié par chromatographie sous une pression d'azote de 100 kPa, sur une colonne de gel de silice (granulométrie 40-63 µ ; diamètre 3,5 cm ; hauteur 45 cm), en éluant par un mélange de dichlorométhane - méthanol (97/3 en volumes), et en recueillant des fractions de 40 cm³. Les fractions 22 à 30 sont réunies, concentrées à sec sous pression réduite (5 kPa) à une température voisine de 40°C. On obtient 1,33 g de 4-[3-(3-chloro-6-méthoxyquinolin-4-yl)propyl]-1-(2-cyclohexyléthyl)-pipéridine-4-carboxylate de benzyle, sous forme d'une huile de couleur orangée.
Spectre infra rouge (CCl₄) : 2925 ; 1727 ; 1622 ; 1503 ; 1230; 1116; 833 et 697 cm⁻¹.

Le 4-[3-(3-chloro-6-méthoxyquinolin-4-yl)propyl]-pipéridine-4-carboxylate de benzyle a été préparé dans l'exemple 1.

### Exemple 4

### Dichlorhydrate de l'acide 4-[3-(3-chloro-6-méthoxyquinolin-4-yl)propyl]-1-(3-phénylpropyl)-pipéridine-4-carboxylique

On chauffe à une température voisine de 100°C, sous agitation, pendant 5 heures, 0,5 g de 4-[3-(3-chloro-6-méthoxyquinolin-4-yl)propyl]-1-(3-phénylpropyl)-pipéridine-4-carboxylate de benzyle dans 8 cm³ d'acide chlorhydrique 5 M. Après refroidissement aux environs de 20°C, la masse réactionnelle est concentrée sous pression réduite (5 kPa) à une température voisine de 40°C. Le résidu d'évaporation obtenu est repris dans 6 cm³ d'un mélange de dichlorométhane - méthanol (90/10 en volumes), puis le mélange est de nouveau concentré à sec dans les conditions ci-dessus. On obtient une meringue que l'on triture dans 5 cm³ d'éther düsopropylique. Le produit cristallisé formé est filtré, lavé par 3 fois 5 cm³ du même solvant, séché à l'étuve sous pression réduite (10 Pa), à une température voisine de 50°C. On obtient 0,46 g de dichlorhydrate de l'acide 4-[3-(3-chloro-6-méthoxyquinolin-4-yl)propyl]-1-(3-phénylpropyl)-pipéridine-4-carboxylique, sous forme d'un solide de couleur beige.
Spectre de R.M.N. ¹H (300 MHz, (CD₃)₂SO d6, δ en ppm) : de 1,40 à 2,25 et de 2,50 à 3,60 (mts : 20H en totalité) ; 3,96 (s : 3H) ; de 7,10 à 7,45 (mt : 5H) ; 7,39 (d, J = 2,5 Hz : 1H) ; 7,47 (dd, J = 9 et 2,5 Hz : 1H) ; 7,98 (d, J = 9 Hz : 1H) ; 8,70 (s : 1H); 10,25 (mf étalé : 1 H).

### 4-[3-(3-Chloro-6-méthoxyquinolin-4-yl)propyl]-1-(3-phénylpropyl)-pipéridine-4-carboxylate de benzyle

Un mélange composé de 1,36 g de 4-[3-(3-chloro-6-méthoxyquinolin-4-yl)propyl]-pipéridine-4-carboxylate de benzyle, 0,55 cm³ de 1-bromo-3-phénylpropane, 0,5 g de carbonate de potassium dans 45 cm³ d'acétonitrile est chauffé sous agitation et sous atmosphère inerte pendant 16 heures à une température voisine de 80°C. Après refroidissement, le mélange réactionnel est filtré puis l'insoluble lavé par de l'acétonitrile. Le filtrat est concentré à sec sous pression réduite (5 kPa) à une température voisine de 40°C. On obtient une huile que l'on purifie par chromatographie sous une pression d'azote de 100 kPa, sur une colonne de gel de silice (granulométrie 40-63 µ ; diamètre 3,5 cm ; hauteur 30 cm), en éluant par un mélange de dichlorométhane - méthanol (97/3 en volumes) et en recueillant des fractions de 40 cm³. Les fractions 21 à 25 sont réunies, puis concentrées comme ci-dessus. On obtient 1 21 g de 4-[3-(3-chloro-6-méthoxyquinolin-4-yl)propyl]-1-(3-phénylpropyl)-pipéridine-4-carboxylate de benzyle, sous forme d'une huile visqueuse de couleur orangée.
Spectre infra rouge (CH₂Cl₂) : 2948 ; 2812 ; 1722 ; 1622 ; 1504 ; 1229 ; 1118 ; 1029 et 834 cm⁻¹.

Le 4-[3-(3-chloro-6-méthoxyquinolin-4-yl)propyl]-pipéridine-4-carboxylate de benzyle a été préparé dans l'exemple 1.

### Exemple 5

### Trichlorhydrate de l'acide 4-[3-(3-chloro-6-niéthoxyquinolin-4-yl)propyl]-1-[2-(pyridin-2-yl)thioéthyl]-pipéridine-4-carboxylique

Un mélange de 0,4 g de 4-[3-(3-chloro-6-méthoxyquinolin-4-yl)propyl]-1-[2-(pyridin-2-yl)thioéthyl]-pipéridine-4-carboxylate d'éthyle dans 7 cm³ d'acide chlorhydrique aqueux 5 M est porté à une température voisine de 100°C, sous agitation et sous atmosphère inerte pendant 4 heures. Après refroidissement à environ 20°C, le mélange réactionnel est concentré à sec sous pression réduite (5 kPa) à une température voisine de 40°C. Le résidu obtenu est repris dans 10 cm³ d'un mélange de dichlorométhane-méthanol (90/10 en volumes). Le mélange est concentré à sec dans les conditions ci-dessus. On obtient 0,45 g de trichlorhydrate de l'acide-4-[3-(3-chloro-6-méthoxyquinolin-4-yl)propyl]-1-[2-(pyridin-2-yl)thioéthyl]-pipéridine-4-carboxylique, sous forme d'une meringue fondant à 132°C en se décomposant.
Spectre de R.M.N.¹H (300 MHz, (CD₃)₂SO d6, δ en ppm) : 1,59 (mt : 2H) ; de 1,65 à 1,95 (mt : 4H) ; 2,20 (d large, J = 13,5 Hz : 2H) ; 2,86 (mt: 2H) ; de 3,10 à 3,65 (mt : 8H) ; 3,99 (s : 3H) ; 7,19 (dd large, J = 7,5 et 4,5 Hz : 1H) ; de 7,35 à 7,50 (mt : 2H) ; 7,50 (dd, J = 9 et 3 Hz : 1H) ; 7,71 (t dédoublé, J = 7,5 et 1,5 Hz : 1H) ; 8,01 (d, J = 9 Hz : 1H) ; 8,48 (d large, J = 4,5 Hz : 1H) ; 8,74 (s : 1H) ; 10,70 (mf : 1H).

### 4-[3-(3-Chloro-6-méthoxyquinolin-4-yl)propyl]-1-[2-(2-pyridin-2-yl)thioéthyl]-pipéridine-4-carboxylate d'éthyle

Le 4-[3-(3-chloro-6-méthoxyquinolin-4-yl)propyl]-1-[2-(2-pyridin-2-yl)thioéthyl]-pipéridine-4-carboxylate d'éthyle est préparé par analogie avec la méthode décrite à l'exemple 1 à partir de chlorhydrate de 4-[3-(3-chloro-6-méthoxyquinolin-4-yl)propyl]-pipéridine-4-carboxylate d'éthyle.
Spectre infra rouge (CCl₄) 2955 ; 1726 ; 1622 ; 1580 ; 1503 ; 1414 ; 1229 ; 1125 ; 1030 et 833 cm⁻¹.

### Chlorhydrate de 4-[3-(3-chloro-6-méthoxyquinolin-4-yl)propyl]-pipéridine-4-carboxylate d'éthyle

Un mélange de 2,6 g de 4-[3-(3-chloro-6-méthoxyquinolin-4-yl)propyl]-1-(*tert-*butyloxy carbonyl)-pipéridine-4-carboxylate d'éthyle dans 40 cm³ de dioxanne additionnés de 14 cm³ de dioxanne chlorhydrique 4 N est agité pendant 16 heures à une température voisine de 20°C. La suspension obtenue est diluée par addition de 100 cm³ d'éther diéthylique, agitée à environ 20°C pendant 1 heure, puis filtrée. Le gâteau est lavé par 2 fois 40 cm³ d'éther diéthylique, puis séché au dessicateur sous pression réduite (5 kPa). On obtient 1,9 g de chlorhydrate de 4-[3-(3-chloro-6-méthoxyquinolin-4-yl)propyl]-pipéridine-4-carboxylate d'éthyle, sous forme d'un solide blanc.
Spectre infra rouge (KBr) : 2965 ; 2474 ; 1720 ; 1620 ; 1584 ; 1416 ; 1241 ; 1119 ; 1019 ; 872 et 743 cm⁻¹.

### 4-[3-(3-Chloro-6-méthoxyquinolin-4-yl)propyl]-1-(tert-butyloxycarbonyl)-pipéridine-4-carboxylate d'éthyle

A une solution de 2,96 g de 4-allyl-1-(*tert*-butyloxycarbonyl)-pipéridine-4-carboxylate d'éthyle dans 30 cm³ de tétrahydrofuranne, agitée à une température voisine de -10°C sous atmosphère inerte, on ajoute 30 cm³ d'une solution 0,5 M de 9-borabicyclo[3,3,1]nonane dans le tétrahydrofuranne, en maintenant la température au-dessous de 0°C. Après l'addition, la température du mélange est ramenée à environ 20°C, puis le mélange est agité pendant encore 4 heures. On ajoute 3,1 g de 4-bromo-3-chloro-6-méthoxy quinoléine, puis 50 cm³ de dioxanne, 6,4 g de phosphate de potassium tribasique et 0,22 g de chlorure de palladium diphénylphosphino ferrocène. Le mélange réactionnel est chauffé à une température voisine de 50°C pendant 16 heures. Après refroidissement à une température voisine de 20°C, le mélange est filtré, puis le gâteau est lavé par 3 fois 50 cm³ d'acétate d'éthyle. Le filtrat est lavé par 100 cm³ d'eau, puis 2 fois 50 cm³ d'une solution aqueuse saturée de chlorure de sodium. La phase organique est séchée sur sulfate de magnésium, filtrée, concentrée sous pression réduite (5 kPa), à une température voisine de 40°C. On obtient une huile brune que l'on purifie par chromatographie, sous une pression de 50 kPa d'azote, sur une colonne de gel de silice (granulométrie 20-4S µ ; diamètre 4,5 cm ; hauteur 42 cm), en éluant par un mélange de cyclohexane - acétate d'éthyle (80/20 en volumes). On recueille les fractions contenant le produit attendu. Celles-ci sont réunies, puis concentrées sous pression réduite (5 kPa) à une température voisine de 40°C. On obtient 2,62 g de 4-[3-(3-chloro-6-méthoxyquinolin-4-yl)propyl]-1-(*tert-*butyloxycarbonyl)-pipéridine-4-carboxylate d'éthyle, sous forme d'une huile de couleur jaune.
Spectre infra rouge : (CH₂Cl₂) 1720 ; 1682 ; 1622 ; 1504 ; 1423 ; 1367 ; 1229 ; 1174 ; 1027 et 834 cm⁻¹.

Le 4-allyl-1-(*tert*-butyloxycarbonyl)-pipéridine-4-carboxylate d'éthyle a été préparé dans l'exemple 1

### Exemple 6

### Sel de sodium de l'acide 4-[3-(3-chloro-6-méthoxyquinolin-4-yl)propyl]-1-heptyl-pipéridine-4-carboxylique

Un mélange de 0,48 g de 4-[3-(3-chloro-6-méthoxyquinolin-4-yl)propyl]-1-heptyl-pipéridine-4-carboxylate de benzyle dans 7 cm³ d'acide chlorhydrique aqueux 5 M est porté à une température voisine de 100°C, sous agitation et sous atmosphère inerte pendant 6 heures. Après refroidissement à environ 20°C, le mélange réactionnel est agité pendant 24 heures puis évaporé sous pression réduite (5 kPa) à une température voisine de 40°C. Le résidu obtenu est purifié par chromatographie sous une pression d'azote de 100 kPa, sur une colonne de gel de silice (granulométrie 40-60µ ; diamètre 2,5 cm, hauteur 35 cm), en éluant par un mélange de dichlorométhane - méthanol - ammoniaque (84/15/1 en volumes) et en recueillant des fractions de 40 cm³. Les fractions 19 à 24 sont réunies puis concentrées comme précédemment. Le solide obtenue est agité dans 10 cm³ d'éther diisopropylique, filtré, lavé par 3 fois 5 cm³ d'éther diisopropylique. On obtient 0,35 g de sel de sodium de l'acide 4-[3-(3-chloro-6-méthoxyquinolin-4-yl)propyl]-1-heptyl-pipéridine-4-carboxylique, sous forme d'un solide fondant à 223°C.
Spectre de R.M.N.¹H (300 MHz, (CD₃)₂SO d6, en ppm) : 0,87 (t, J = 7 Hz : 3H) ; de 1,10 à 1,45 (mt : 12H) ; de 1,45 à 1,70 (mt : 4H) ; de 1,85 à 2,05 (mt : 2H) ; 1,97 (d large, J = 10,5 Hz : 2H) ; 2,17 (t large, J = 7,5 Hz : 2H) ; de 2,45 à 2,60 (mt : 2H) ; 3,15 (mt : 2H) ; 3,97 (s : 3H) ; 7,40 (mt : 1H) ; 7,44 (dd, J = 9 et 3 Hz : 1H) ; 7,95 (d, J=9Hz: 1H) ; 8,66 (s: 1H).

### 4-[3-(3-Chloro-6-méthoxyquinolin-4-yl)propyl]-1-heptyl-pipéridine-4-carboxylate de benzyle

A une solution de 0,61 cm³ de 1-iodoheptane dans 45 cm³ d'acétonitrile on ajoute à une température voisine de 20°C, sous agitation et sous atmosphère inerte, 1,36 g de 4-[3-(3-chloro-6-méthoxyquinolin-4-yl)propyl]-pipéridine-4-carboxylate de benzyle et 0,5 g de carbonate de potassium. Après chauffage pendant 18 heures à une température voisine de 80°C, on ajoute 1,17 cm³ de 1-iodoheptane supplémentaire. Après chauffage pendant 40 heures à une température voisine de 80°C le mélange réactionnel est refroidi à environ 20°C, filtré, concentré sous pression réduite (1 kPa) à une température voisine de 50°C. Le résidu obtenu est purifié par chromatographie sous une pression d'azote de 100 kPa, sur une colonne de gel de silice (granulométrie 40-60 µ ; diamètre 3,5 cm ; hauteur 35 cm), en éluant par un mélange de dichlorométhane - méthanol (95/05 en volumes) et en recueillant des fractions de 35 cm³. Les fractions 18 à 26 sont réunies puis concentrées comme précédemment. On obtient 0,36 g de 4-[3-(3-chloro-6-méthoxyquinolin-4-yl)propyl]-1-heptyl-pipéridine-4-carboxylate de benzyle.
Spectre infra rouge (CH₂Cl₂) 2957 ; 2931 ; 1722 ; 1622 ; 1504 ; 1229 ; 1159 ; 1118 ; 1028 et 834 cm⁻¹.

4-[3-(3-chloro-6-méthoxyquinolin-4-yl)propyl]-pipéridine-4-carboxylate de benzyle a été préparé comme décrit dans l'exemple 1.

### Exemple 7

### Dichlorhydrate de l'acide 4-[3-(3-chloro-6-méthoxyquinolin-4-yl)propyl]-1-(2-(cyclopentylthio)éthyl]-pipéridine-4-carboxylique

Un mélange de 0,55 g de 4-[3-(3-chloro-6-méthoxyquinolin-4-yl)propyl]-1-[2-(cyclopentylthio)éthyl]-pipéridine-4-carboxylate d'éthyle dans 8 cm³ d'acide chlorhydrique aqueux 6 N est chauffé à une température voisine de 100°C, sous agitation et sous atmosphère inerte, pendant 5 heures. Après 18 heures d'agitation à une température voisine de 20°C la solution obtenue est concentrée sous pression réduite (5 kPa) à une température voisine de 40°C. Le résidu d'évaporation obtenu est repris dans 10 cm³ d'un mélange de dichlorométhane - méthanol (90/10 en volumes), puis concentré dans les mêmes conditions que précédemment. On obtient 0,59 g du dichlorhydrate de l'acide 4-[3-(3-chloro-6-méthoxyquinolin-4-yl)propyl]-1-[2-(cyclopentylthio)éthyl]-pipéridine-4-carboxylique, sous forme d'une meringue fondant à 129°C en se ramollissant.
Spectre RMN : ¹H (300 MHz, (CD₃)₂SO d6, en ppm) : de 1,30 à 2,10 (mt : 14H) ; 2,15 (d large, J = 13,5 Hz : 2H) ; de 2,65 à 3,00 (mt : 4H) ; de 3,05 à 3,40 (mt : 5H) ; 3,46 (d large, J= 12 Hz : 2H) ; 3,97 (s : 3H) ; 7,42 (d, J = 2,5 Hz : 1H) ; 7,48 (dd, J = 9 et 2,5 Hz : 1H) ; 7,99 (d, J = 9 Hz : 1H) ; 8,72 (s : 1H) ; de 10,55 à 10,90 (mf : 1H).

### 4-[3-(3-Chloro-6-méthoxyquinolin-4-yl)propyl]-1-[2-(cyclopentylthio)éthyl]-pipéridine-4-carboxylate d'éthyle

A une solution de 0,607 g de (2 chloroéthylthio)cyclopentane dans 50 cm³ d'acétonitrile on ajoute à une température voisine de 20°C, sous agitation et sous atmosphère inerte, 1,2 g de 4-[3-(3-chloro-6-méthoxyquinolin-4-yl)propyl]-pipéridine-4-carboxylate d'éthyle et 0,51 g de carbonate de potassium et 0,61 g d'iodure de potassium. Après chauffage pendant 20 heures à une température voisine de 80°C, le mélange réactionnel est refroidi à environ 20°C, filtré, concentré sous pression réduite (1 kPa) à une température voisine de 50°C. Le résidu obtenu est purifié par chromatographie sous une pression d'azote de 100 kPa, sur une colonne de gel de silice (granulométrie 40-60 µ ; diamètre 3,5 cm), en éluant par un mélange de dichlorométhane - méthanol (98/2 en volumes) et en recueillant des fractions de 35 cm³. Les fractions 25 à 31 sont réunies puis concentrées comme précédemment. On obtient 0,9 g de 4-[3-(3-chloro-6-méthoxyquinolin-4-yl)propyl]-1-[2-(cyclopentylthio)éthyl]-pipéridine-4-carboxylate d'éthyle.
Spectre infra rouge (CCl₄) : 958 ; 1726 ; 1622 ; 1503 ; 1229 ; 1117 ; 1030 et 833 cm⁻¹.

Le 4-[3-(3-chloro-6-méthoxyquinolin-4-yl)propyl]-pipéridine-4-carboxylate d'éthyle a été préparé comme décrit dans l'exemple 5.

### Exemple 8

### Acide 4-[3-(3-Chloro-6-méthoxyquinolin-4-yl)propyl]-1-[2-(2-thién-2-yl)thioéthyl]-pipéridine-4-acétique

En opérant de manière analogue aux exemples précédents, on prépare l'acide 4-[3-(3-chloro-6-méthoxyquinolin-4-yl)propyl]-1-[2-(2-thién-2-yl)thioéthyl]-pipéridine-4-acétique.
Spectre de R.M.N. ¹H (300 MHz, (CD₃)₂SO d6, en ppm) : de 1,30 à 1,70 (mt : 8H) ; 2,14 (s : 2H) ; 2,33 (mt : 4H) ; de 2,45 à 2,60 (mt : 2H) ; 2,92 (t large, J = 7 Hz : 2H) ; 3,13 (mt : 2H) ; 3,96 (s : 3H) ; 7,05 (dd, J = 5,5 et 3,5 Hz : 1H); 7,18 (dd, J = 3,5 et 1 Hz : 1H) ; 7,39 (d, J = 3 Hz : 1H) ; 7,44 (dd, J = 9 et 3 Hz : 1H) ; 7,60 (dd, J = 5,5 et 1 Hz : 1H) ; 7,95 (d, J = 9 Hz : 1H) ; 8,66 (s : 1H).

### Exemple 9

### {4-[3-(3-chloro-6-méthoxyquinolin-4-yl)propyl]-1-[2-(cyclopentylthio)éthyl]-pipéridine-4-yl} méthanol

En opérant de manière analogue aux exemples précédents, on prépare le {4-[3-(3-chloro-6-méthoxyquinolin-4-yl)propyl]-1-[2-(cyclopentylthio)éthyl]-pipéridine-4-yl}méthanol.
Spectre de R.M.N.¹H (300 MHz, (CD₃)₂SO d6, en ppm) : de 1,15 à 1,75 (mt : 14H) ; 1,95 (mt : 2H) ; de 2,20 à 2,40 (mt : 4H) ; 2,44 (mt : 2H) ; 2,57 (mt : 2H) ; de 3,05 à 3,25 (mt : 5H) ; 3,97 (s : 3H) ; 4,38 (t, J = 5,5 Hz : 1H) ; 7,38 (d, J = 3 Hz : 1H) ; 7,45 (dd, J = 9 et 3 Hz : 1H) ; 7,96 (d, J = 9 Hz : 1H); 8,68 (s : 1H).

### Exemple 10

### 4-[3-(3-chloro-6-méthoxyquinolin-4-yl)propyl]-1-[(3-phénylpropyl)-pipéridine-4-yl]méthanol

En opérant de manière analogue aux exemples précédents, on prépare le 4-[3-(3-chloro-6-méthoxyquinolin-4-yl)propyl]-1-[(3-phénylpropyl)-pipéridine-4-yl] méthanol.
Spectre de R.M.N.¹H (300 MHz, (CD₃)₂SO d6, en ppm) : 1,59 (mt : 2H) ; de 1,65 à 1,95 (mt : 4H) ; 2,20 (d large, J = 13,5 Hz : 2H) ; 2,86 (mt : 2H) ; de 3,10 à 3,65 (mt : 8H) ; 3,99 (s : 3H) ; 7,19 (dd large, J = 7,5 et 4,5 Hz : 1H) ; de 7,35 à 7,50 (mt : 2H) ; 7,50 (dd, J = 9 et 3 Hz : 1H) ; 7,71 (t dédoublé, J = 7,5 et 1,5 Hz : 1H) ; 8,01 (d, J = 9 Hz : 1H) ; 8,48 (d large, J = 4,5 Hz : 1H) ; 8,74 (s : 1H) ; 10,70 (mf : 1H).

### Exemple 11

### Acide-4-[3-(3-fluoro-6-méthoxyquinolin-4-yl)-propyl]-1-[2-(thién-2-yl)-thioéthyl]-pipéridin-4-carboxylique

Un mélange de 0,4 g de 4-[3-(3-fluoro-6-méthoxyquinolin-4-yl)-propyl]-1-[2-(thién-2-yl)-thioéthyl]-pipéridin-4-carboxylate d'éthyle dans 5 cm³ d'acide chlorhydrique aqueux 5 N et 3 cm³ de dioxanne est porté à une température voisine de 100°C, sous agitation et sous atmosphère inerte pendant 20 heures. Après refroidissement à environ 20°C, le mélange réactionnel est concentré à sec sous pression réduite (2 kPa) à une température voisine de 40°C. Le mélange est filtré puis chromatographié, sous pression atmosphérique, sur une colonne de gel de silice (granulométrie 40-63 µ ; diamètre 1,5 cm ; masse 55 g), en éluant par un mélange de chloroforme-méthanol-ammoniaque (12/3/0,5 en volumes) et en recueillant des fractions de 15 cm³. Les fractions 5 à 12 sont réunies puis concentrées à sec dans les conditions de ci-dessus. On obtient 0,26 g d'acide 4-[3-(3-fluoro-6-méthoxyquinolin-4-yl)-propyl]-1-[2-(thién-2-yl)-thioéthyl]-pipéridin-4-carboxylique, sous forme d'un solide cristallin blanc fondant à 180°C.
Spectre de R.M.N. ¹H (300 MHz, (CD₃)₂SO d6, δ en ppm) : 1,31 (t très large, J = 13 Hz : 2H) ; de 1,50 à 1,70 (mt : 4H) ; de 1,85 à 2,05 (mt : 4H) ; 2,45 (t large, J = 7 Hz : 2H) ; 2,60 (d large, J = 11 Hz : 2H) ; 2,91 (t large, J = 7 Hz : 2H) ; 3,04 (t très large, J = 6 Hz : 2H) ; 3,96 (s : 3H) ; 7,04 (dd, J = 5 et 3,5 Hz : 1H) ; 7,17 (dd, J = 3,5 et 1 Hz : 1H) ; 7,35 (d, J = 2,5 Hz : 1H) ; 7,40 (dd, J = 9 et 2,5 Hz : 1H) ; 7,60 (dd, J = 5 et 1 Hz : 1H); 7,97 (d, J = 9 Hz : 1H) ; 8,70 (s large : 1H).

### 4-[3-(3-Fluoro-6-méthoxyquinolin-4-yl)-propyl]-1-[2-(thién-2-yl)thioéthyl]-pipéridin-4-carboxylate d'éthyle

Un mélange de 0,8 g de 4-[3-(3-fluoro-6-méthoxyquinolin-4-yl)-propyl]-pipéridin-4-carboxylate d'éthyle, 0,6 g de 2-(2-bromoéthylthio)-thiophène et 1,5 g de carbonate de potassium dans 10 cm³ d'acétonitrile est chauffé pendant 18 heures à une température voisine de 80°C, sous agitation et sous atmosphère inerte. Après refroidissement à une température voisine de 20°C, le mélange réactionnel est concentré à sec sous pression réduite (2 kPa) à une température voisine de 40°C. Le résidu obtenu est repris par du dichlorométhane et de l'eau. La phase organique est lavée avec de l'eau et une solution saturée de chlorure de sodium, séchée sur du sulfate de magnésium, filtrée, concentrée à sec dans les conditions ci-dessus. Le résidu est purifié par chromatographie sous pression atmosphérique, sur une colonne de gel de silice (granulométrie 70-200 µ ; diamètre 3 cm ; masse 50 g), en éluant par un mélange de d'acétate d'éthyle-éther de pétrole (40-65°C) (75/25 en volumes) et en recueillant des fractions de 30 cm³. Les fractions 3 à 5 sont réunies puis concentrées à sec dans les conditions de ci-dessus. On obtient 0,7 g de 4-[3-(3-fluoro-6-méthoxyquinolin-4-yl)propyl]-1-[2-(thién-2-yl)-thioéthyl]-pipéridin-4-carboxylate d'éthyle, sous forme d'une huile épaisse incolore.
Spectre de R.M.N. ¹H (300 MHz, (CD₃)₂SO d6, δ en ppm) : 0,99 (t, J = 7 Hz : 3H) ; 1,34 (t très large, J = 12 Hz : 2H) ; de 1,45 à 1,65 (mt : 4H) ; de 1,85 à 2,00 (mt : 4H) ; 2,44 (t large, J = 7 Hz : 2H) ; 2,59 (d large, J = 11,5 Hz : 2H) ; 2,89 (t large, J = 7 Hz : 2H) ; 3,03 (t très large, J = 6,5 Hz : 2H) ; 3,94 (s : 3H) ; 3,96 (q, J = 7 Hz : 2H) ; 7,02 (dd, J = 5 et 3,5 Hz : 1H) ; 7,16 (dd, J = 3,5 et 1 Hz : 1H) ; 7,32 (d, J = 2,5 Hz : 1H) ; 7,39 (dd, J = 9 et 2,5 Hz : 1H) ; 7,58 (dd, J = 5 et 1 Hz : 1H) ; 7,96 (d, J = 9 Hz : 1H) ; 8,69 (s large : 1H).

### 4-[3-(3-Fluoro-6-méthoxyquinolin-4-yl)-propyl]-pipéridin-4-carboxylate d'éthyle

A une solution de 0,5 g de 4-[3-(3-fluoro-6-méthoxyquinolin-4-yl)-propyl]-1-(*tert-*butyloxycarbonyl)-pipéridin-4-carboxylate d'éthyle dans 10 cm³ de dichlorométhane, on ajoute sous agitation et sous atmosphère inerte, à une température voisine de 5°C, 2 cm³ d'acide trifluoroacétique. Après 45 minutes, le mélange réactionnel est concentré à sec sous pression réduite (2 kPa) à une température voisine de 40°C. Le résidu est repris avec de l'éther diéthylique et avec une solution de bicarbonate de potassium saturée puis une solution de carbonate de potassium saturée. La phase organique est lavée avec 2 fois 5 cm³ d'eau, puis avec une solution saturée de chlorure de sodium, séchée sur sulfate de magnésium, filtrée puis concentrée à sec sous pression réduite (2kPa) à une température voisine de 40°C. On obtient 0,26 g de 4-[3-(3-fluoro-6-méthoxyquinolin-4-yl)-propyl]-pipéridin-4-carboxylate d'éthyle, sous forme d'une huile épaisse
Spectre de R.M.N. ¹H (300 MHz, (CD₃)₂SO d6, δ en ppm) : 1,02 (t, J = 7 Hz : 3H) ; 1,28 (mt : 2H) ; de 1,45 à 1,70 (mt : 4H) ; 1,90 (d large, J = 13,5 Hz : 2H) ; 2,46 (t large, J = 12 Hz : 2H) ; 2,79 (d mt, J = 12 Hz : 2H) ; 3,06 (t large, J = 6 Hz : 2H) ; 3,95 (s : 3H) ; 3,98 (q, J = 7 Hz : 2H) ; 7,35 (d, J = 2,5 Hz : 1H) ; 7,40 (dd, J = 9 et 2,5 Hz : 1H) ; 7,97 (d, J = 9 Hz : 1H) ; 8,70 (d, J = 1 Hz : 1H).

### 4-[3-(3-Fluoro-6-méthoxyquinolin-4-yl)-propyl]-1-(tert-butyloxycarbonyl)-pipéridin-4-carboxylate d'éthyle

On refroidit à une température voisine de -30°C 1,4 g de 4-allyl-1-(*tert-*butyloxycarbonyl)-pipéridin-4-carboxylate d'éthyle et l'on ajoute sous agitation et sous atmosphère inerte 11 cm³ d'une solution 0,5 M de 9-borabicyclo-[3,3,1]-nonane dans le tétrahydrofurane. Après l'addition, la température du mélange est ramenée à environ 20°C. La solution obtenue est agitée pendant encore 4 heures, puis l'on ajoute, 0,09 g de chlorure de palladium diphénylphosphino ferrocène, 1,4 g de 4-iodo-3-fluoro-6-méthoxy-quinoléine et 2,5 g de phosphate de potassium tribasique. Après 16 heures d'agitation à une température voisine de 60°C, le mélange réactionnel est refroidi au voisinage de 20°C puis concentré à sec sous pression réduite (2 kPa) à une température voisine de 40°C. Le résidu obtenu est repris par de l'acétate d'éthyle et de l'eau, décanté, séché sur sulfate de magnésium, filtré, concentré à sec sous pression réduite (2 kPa) à une température voisine de 40°C. Le résidu obtenu est purifié par chromatographie, sous pression atmosphérique, sur une colonne de gel de silice (granulométrie 70-200µ ; diamètre 4,5 cm ; masse 125 g), en éluant par un mélange de dichlorométhane-acétate d'éthyle (98/2 en volumes), et en recueillant des fractions de 20 cm³. Les fractions 98 à 170 sont réunies puis concentrées à sec dans les conditions de ci-dessus. On obtient 1,5 g de 4-[3-(3-fluoro-6-méthoxyquinolin-4-yl)propyl]-1-(*tert*-butyloxycarbonyl)-pipéridin-4-carboxylate d'éthyle, sous forme d'une huile épaisse de couleur brune.
Spectre de R.M.N. ¹H (300 MHz, (CD₃)₂SO d6, δ en ppm) : 1,02 (t, J = 7 Hz : 3H) ; 1,30 (mt : 2H) ; 1,39 (s : 9H) ; de 1,45 à 1,70 (mt : 4H) ; 1,92 (d large, J = 13,5 Hz : 2H) ; 2,81 (mt : 2H) ; 3,05 (t large, J = 6,5 Hz : 2H) ; 3,69 (d large, J = 13,5 Hz : 2H) ; 3,95 (s : 3H) ; 4,00 (q, J = 7 Hz : 2H) ; 7,34 (d, J = 2,5 Hz : 1 H) ; 7,40 (dd, J = 9 et 2,5 Hz : 1H) ; 7,97 (d, J = 9 Hz : 1H) ; 8,70 (d, J = 1 Hz : 1H).

### 4-Iodo-3-fluoro-6-méthoxy-quinoléine

On refroidit à une température voisine de -75°C 1,8 cm³ de diisopropylamine dans 80 cm³ de tétrahydrofurane et l'on ajoute sous agitation et sous atmosphère inerte 7,7 cm³ d'une solution 1,6 M de buthyllithium dans l'hexane. Après une agitation de 20 minutes à une température voisine de -75°C on ajoute une solution de 2,2 g de 3-fluoro-6-méthoxy-quinoléine dans 20 cm³ de tétrahydrofurane. La solution obtenue est agitée pendant encore 4 heures, puis l'on ajoute une solution de 3,3 g d'iode bisublimé dans 10 cm³ de tétrahydrofurane. Après 2 heures d'agitation à une température voisine de 20°C, le mélange réactionnel est hydrolysé par 200 cm³ d'un mélange tétrahydrofurane-eau 90/10 puis 100 cm³ d'une solution saturée de chlorure de sodium et 150 cm³ d'acétate d'éthyle. Le mélange est lavé avec 3 fois 80 cm³ d'une solution saturée de chlorure de sodium séché sur du sulfate de magnésium, filtré, concentré à sec sous pression réduite (2 kPa) à une température voisine de 40°C. Le résidu obtenu est purifié par chromatographie sous pression atmosphérique, sur une colonne de gel de silice (granulométrie 70-200 µ ; diamètre 5 cm ; hauteur 35 cm), en éluant avec un mélange d'éther de pétrole et d'acétate d'éthyle 90/10. Les fractions 66 à 95 sont réunies puis concentrées à sec sous pression réduite (5 kPa) à une température voisine de 40°C. On obtient 0,9 g de 4-iodo-3-fluoro-6-méthoxy quinoléine sous forme d'un solide crème
Spectre de R.M.N. ¹H (300 MHz, (CD₃)₂SO d6, δ en ppm) : 4,00 (s : 3H) ; 7,31 (d, J = 2,5 Hz : 1H) ; 7,47 (dd, J = 9 et 2,5 Hz : 1H) ; 8,01 (d, J = 9 Hz : 1H) ; 8,64 (s : 1H).

### 3-Fluoro-6-méthoxy-quinoléine

Un mélange de 1,35 g de 4-chloro-3-fluoro-6-méthoxy-quinoléine, 1,1 cm³ de triéthylamine et 100 mg de palladium sur charbon dans 23 cm³ de méthanol est agité à une température voisine de 20°C sous pression de 2 bars d'hydrogène pendant 18 heures. Le mélange réactionnel est filtré puis concentré à sec sous pression réduite (2 kPa) à une température voisine de 40°C. Le résidu obtenu est purifié par chromatographie sous pression atmosphérique, sur une colonne de gel de silice (granulométrie 70-200 µ ; diamètre 3 cm ; masse 40 g), en éluant par dichlorométhane-acétate d'éthyle (95/5 en volumes). Les fractions contenant le produit sont réunies puis concentrées à sec selon les conditions de ci-dessus. On obtient 1 g de 3-fluoro-6-méthoxy-quinoléine.
Spectre de R.M.N. ¹H (300 MHz, (CD₃)₂SO d6, δ en ppm) : 3,92 (s : 3H) ; 7,40 (mt : 2H) ; 8,07 (d, J = 9 Hz : 1H) ; 8,04 (dd, J = 10 et 3 Hz : 1H) ; 8,77 (d, J = 3 Hz : 1H).

### 4-Chloro-3-fluoro-6-méthoxy-quinoléine

On refroidit à une température voisine de -75°C 1,3 cm³ de diisopropylamine dans 50 cm³ de tétrahydrofurane et l'on ajoute sous agitation et sous atmosphère inerte 5,8 cm³ d'une solution 1,6 M de buthyllithium dans l'hexane. Après une agitation de 20 minutes à une température voisine de -75°C on ajoute une solution de 1,2 g de 4-chloro-6-méthoxy-quinoléine dans 20 cm³ de tétrahydrofurane. La solution obtenue est agitée pendant encore 4 heures, puis l'on ajoute une solution de 2,9 g de N-fluorobenzène sulfonimide dans 10 cm³ de tétrahydrofurane. Après 2 heures d'agitation à une température voisine de 20°C, le mélange réactionnel est hydrolysé par 200 cm³ d'un mélange tétrahydrofurane-eau 90/10 puis 100 cm³ d'une solution saturée de chlorure de sodium et 150 cm³ d'acétate d'éthyle. Le mélange est lavé avec 3 fois 80 cm³ d'une solution saturée de chlorure de sodium séché sur du sulfate de magnésium, filtré, concentré à sec sous pression réduite (2 kPa) à une température voisine de 40°C. Le résidu obtenu est purifié par chromatographie sous pression atmosphérique, sur une colonne de gel de silice (granulométrie 70-200 µ ; diamètre 4 cm ; masse 100 g), en éluant avec du dichlorométhane. Les fractions contenant le produit sont réunies puis concentrées à sec sous pression réduite (5 kPa) à une température voisine de 40°C. On obtient 0,4 g de 4-chloro-3-fluoro-6-méthoxy quinoléine.
Spectre de R.M.N. ¹H (300 MHz, (CD₃)₂SO d6, δ en ppm) : 4,01 (s : 3H) ; 7,43 (d, J = 2,5 Hz : 1H) ; 7,52 (dd, J = 9 et 2,5 Hz : 1H) ; 8,07 (d, J = 9 Hz : 1H) ; 8,91 (d, J = 1 Hz : 1H).

### 4-Chloro-6-méthoxy-quinoléine

Un mélange de 12 g de 4-hydroxy-6-méthoxy-quinoléine dans 50 cm³ d'oxychlorure de phosphore est porté à une température voisine de 100°C, sous agitation et sous atmosphère inerte pendant 2 heures. Après refroidissement à environ 20°C, le mélange réactionnel est concentré à sec sous pression réduite (2 kPa) à une température voisine de 40°C puis hydrolysé avec de la glace puis amené à pH = 10 à l'aide d'une solution de soude 5 N. Le mélange est extrait avec de l'acétate d'éthyle, séché sur du sulfate de magnésium, filtré, concentré à sec sous pression réduite (2 kPa) à une température voisine de 40°C. Le résidu obtenu est chromatographié, sous pression atmosphérique, sur une colonne de gel de silice (granulométrie 70-200 µ), en éluant par un mélange de dichlorométhane-méthanol (85/15 en volumes). Les fractions contenant le produit sont réunies puis concentrées à sec selon les conditions ci-dessus. On obtient 12 g 4-chloro-6-méthoxy-quinoléine sous forme d'un solide blanc fondant à 82°C.
Spectre de R.M.N. ¹H (300 MHz, (CD₃)₂SO d6, δ en ppm) : 3,958 (s : 3H) ; 7,45 (d, J = 2,5 Hz : 1H) ; 7,53 (dd, J = 9 et 2,5 Hz : 1H) ; 7,76 (d, J = 4,5 Hz : 1H) ; 8,04 (d, J = 9 Hz : 1H) ; 8,70 (d, J = 4,5 Hz : 1H).

La 4-allyl-1-(*tert*-butyloxycarbonyl)-pipéridine-4-carboxylate d'éthyle a été préparé dans l'exemple 1.

La 4-hydroxy-6-méthoxy-quinoléine a été préparé comme décrit dans l'exemple 1.

### Exemple 12

### Acide-4-[3-(3-fluoro-6-méthoxyquinolin-4-yl)-propyl]-1-[2-(3,5-difluoro-6-phénoxy)-éthyl]-pipéridin-4-carboxylique

Un mélange de 0,4 g de 4-[3-(3-fluoro-6-méthoxyquinolin-4-yl)-propyl]-1-[2-(3,5-difluoro-6-phénoxy)-éthyl]-pipéridin-4-carboxylate d'éthyle dans 6 cm³ d'acide chlorhydrique aqueux 5 N et 10 cm³ de dioxanne est porté à une température voisine de 100°C, sous agitation et sous atmosphère inerte pendant 20 heures. Après refroidissement à environ 20°C, le mélange réactionnel est concentré à sec sous pression réduite (2 kPa) à une température voisine de 40°C. Le résidu obtenu est chromatographié, à pression atmosphérique, sur une colonne de gel de silice (granulométrie 70-200 µ ; diamètre 1,5 cm ; masse 50 g), en éluant par un mélange de chloroforme-méthanol-ammoniaque (12/3/0,5 en volumes) et en recueillant des fractions de 15 cm³. Les fractions 10 à 15 sont réunies puis concentrées à sec selon les conditions de ci-dessus. On obtient 0,2 g d'acide 4-[3-(3-fluoro-6-méthoxyquinolin-4-yl)propyl]-1-[2-(3,5-difluoro-6-phénoxy)-éthyl]-pipéridin-4-carboxylique, sous forme d'un solide blanc fondant à 175°C.
Spectre de R.M.N. ¹H (300 MHz, (CD₃)₂SO d6, δ en ppm) : 1,33 (t très large, J = 13 Hz : 2H) ; de 1,50 à 1,70 (mt : 4H) ; 1,95 (d large, J = 13 Hz : 2H) ; 2,05 (t large, J = 11,5 Hz : 2H) ; 2,60 (t, J = 6 Hz : 2H) ; 2,69 (d large, J = 11,5 Hz : 2H) ; 3,04 (mt : 2H) ; 3,96 (s : 3H) ; 4,06 (t, J = 6 Hz : 2H) ; de 6,65 à 6,85 (mt : 3H) ; 7,34 (d, J = 2,5 Hz : 1H) ; 7,39 (dd, J = 9 et 2,5 Hz : 1H) ; 7,96 (d, J = 9 Hz : 1H) ; 8,69 (s large : 1H).

### 4-[3-(3-Fluoro-6-méthoxyquinolin-4-yl)-propyl]-1-[2-(3,5-difluoro-6-phénoxy)-éthyl]-pipéridin-4-carboxylate d'éthyle

Un mélange de 0,4 g de 4-[3-(3-fluoro-6-méthoxyquinolin-4-yl)-propyl]-pipéridin-4-carboxylate d'éthyle, 0,3 g de 1-(2-bromoéthoxy)-3,5-diflurobenzène, 0,18 g d'iodure de potassium et 0,74 g de carbonate de potassium dans 10 cm³ d'acétonitrile est chauffé pendant 18 heures à une température voisine de 75°C, sous agitation et sous atmosphère inerte. Après refroidissement à une température voisine de 20°C, le mélange réactionnel est concentré à sec sous pression réduite (5 kPa) à une température voisine de 40°C. Le résidu obtenu est repris par de l'acétate d'éthyle et de l'eau. La phase organique est lavée avec de l'eau et une solution saturée de chlorure de sodium, séchée sur du sulfate de magnésium, filtrée, concentrée à sec dans les conditions ci-dessus. Le résidu est purifié par chromatographie sous pression atmosphérique, sur une colonne de gel de silice (granulométrie 70-200 µ ; diamètre 1,5 cm ; masse 35 g), en éluant par du dichlorométhane. Les fractions 7 à 11 sont réunies puis concentrées à sec selon les conditions de ci-dessus. On obtient 0,4 g de 4-[3-(3-fluoro-6-méthoxyquinolin-4-yl)-propyl]-1-[2-(3,5-difluoro-6-phénoxy)-éthyl]-pipéridin-4-carboxylate d'éthyle sous forme d'une huile épaisse incolore.
Spectre de R.M.N. ¹H (300 MHz, (CD₃)₂SO d6, δ en ppm) : 1,00 (t, J = 7 Hz : 3H) ; 1,38 (t très large, J = 12 Hz : 2H) ; de 1,45 à 1,70 (mt : 4H) ; de 1,90 à 2,10 (mt : 4H) ; 2,60 (t, J = 6 Hz : 2H) ; 2,69 (d très large, J = 12 Hz : 2H) ; 3,05 (t très large, J = 6,5 Hz : 2H) ; 3,95 (s : 3H) ; 3,98 (q, J = 7 Hz : 2H) ; 4,06 (t, J = 6 Hz : 2H) ; de 6,65 à 6,85 (mt : 3H) ; 7,34 (d, J = 2,5 Hz : 1H) ; 7,40 (dd, J = 9 et 2,5 Hz : 1H) ; 7,97 (d, J = 9 Hz : 1H) ; 8,70 (s large : 1H).

Le 4-allyl-1-(*tert*-butyloxycarbonyl)-pipéridin-4-carboxylate d'éthyle a été préparé dans l'exemple 1.

Le 1-(2-bromoéthoxy)-3,5-difluorobenzène peut être obtenu par application de la méthode décrite dans l'exemple 16.

### Exemple 13

### Dichlorhydrate de l'acide 4-[3-(3-chloro-6-méthoxyquinolin-4-yl)-propyl]-1-[2-(2,3,5-trifluoro-6-phénoxy)-éthyl]-pipéridin-4-carboxylique

Un mélange de 1 g de 4-[3-(3-chloro-6-méthoxy-quinolin-4-yl)-propyl]-1-[2-(2,3,5-trifluoro-6-phénoxy)-éthyl]-pipéridin-4-carboxylate de benzyle dans 50 cm³ d'acide chlorhydrique aqueux 5 N est porté à une température voisine de 100°C, sous agitation pendant 20 heures. Après refroidissement à environ 20°C, le mélange réactionnel est concentré à sec sous pression réduite (2 kPa) à une température voisine de 40°C. Le résidu obtenu est repris avec 50 cm³ d'acétone, filtré, lavé avec 3 fois 15 cm³ puis séché au dessicateur sous pression réduite (0,1 kPa) à une température voisine de 40°C. On obtient 0,88 g, dichlorhydrate de l'acide 4-[3-(3-chloro-6-méthoxyquinolin-4-yl)-propyl]-1-[2-(2,3,5-trifluoro-6-phénoxy)-éthyl]-pipéridin-4-carboxylique sous forme d'un solide blanc fondant à 170°C.
Spectre de R.M.N.¹H (300 MHz, (CD₃)₂SO d6, δ en ppm) : de 1,45 à 2,30 (mt : 8H) ; de 2,85 à 3,70 (mt : 8H) ; 3,97 (s : 3H) ; 4,50 (mt : 2H) ; 7,15 (mt : 2H) ; 7,40 (d, J = 3 Hz : 1H) ; 7,46 (dd, J = 9 et 3 Hz : 1H) ; 7,97 (d, J = 9 Hz : 1H) ; 8,69 (s : 1 H) ; 10,07 (mf : 1H) ; de 12,50 à 13,10 (mf étalé : 1H).

### 4-[3-(3-Chloro-6-méthoxyquinolin-4-yl)-propyl]-1-[2-(2,3,5-trifluoro-6-phénoxy)-éthyl]-pipéridin-4-carboxylate de benzyle

Un mélange de 1,4 g de chlorhydrate de 4-[3-(3-chloro-6-méthoxyquinolin-4-yl)-propyl]-pipéridin-4-carboxylate de benzyle, 0,9 g de 1-(2-bromoéthoxy)-2,3,5-trifluorobenzène, 0,6 g d'iodure de potassium et 2 g de carbonate de potassium dans 100 cm³ d'acétonitrile est chauffé pendant 18 heures à une température voisine de 75°C, sous agitation et sous atmosphère inerte. Après refroidissement à une température voisine de 20°C, le mélange réactionnel est filtré, lavé avec 3 fois 30 cm³ d'acétonitrile. Le filtrat est concentré à sec sous pression réduite (2 kPa) à une température voisine de 40°C. Le résidu obtenu est purifié par chromatographie sous pression atmosphérique, sur une colonne de gel de silice (granulométrie 20-45 µ ; diamètre 3 cm ; hauteur : 27 cm), en éluant par de l'acétate d'éthyle et en recueillant des fractions de 50 cm³. Les fractions 9 à 23 sont réunies puis concentrées à sec selon les conditions ci-dessus. On obtient 1,27 g de 4-[3-(3-chloro-6-méthoxyquinolin-4-yl)-propyl]-1-[2-(2,3,5-trifluoro-6-phénoxy)-éthyl]-pipéridin-4-carboxylate de benzyle sous forme d'une huile épaisse jaune.
Spectre de R.M.N.¹H (300 MHz, (CD₃)₂SO d6, δ en ppm) : de 1,30 à 1,60 (mt : 4H) ; 1,72 (mt : 2H) ; de 1,95 à 2,15 (mt : 4H) ; 2,64 (t, J = 5,5 Hz : 2H) ; 2,71 (dmf, J = 12 Hz : 2H) ; 3,13 (t large, J = 7,5 Hz : 2H) ; 3,93 (s : 3H) ; 4,16 (t, J = 5,5 Hz : 2H) ; 5,04 (s : 2H) ; de 6,95 à 7,15 (mt : 2H) ; de 7,20 à 7,30 (mt : 5H) ; 7,34 (d, J = 3 Hz : 1H) ; 7,45 (dd, J = 9 et 3 Hz : 1H) ; 7,97 (d, J = 9 Hz : 1H) ; 8,67 (s : 1H).

### Chlorhydrate de 4-[3-(3-ehloro-6-méthoxyquinolin-4-yl)-propyl]-pipéridin-4-carboxylate de benzyle

Un mélange de 17,4 g de 1-*tert*-butyloxycarbonyl-4-[3-(3-chloro-6-méthoxyquinolin-4-yl)-propyl]-pipéridin-4-carboxylate de benzyle dans 75 cm³ d'acide chlorhydrique 5 N en solution dans le dioxanne et 200 cm³ de dioxanne est agité à une température voisine de 20°C pendant 20 heures. On ajoute au mélange réactionnel 200 cm³ d'éther diéthylique. On filtre le précipité formé pour obtenir 14,26 g de chlorhydrate de 4-[3-(3-fluoro-6-méthoxyquinolin-4-yl)-propyl]-pipéridin-4-carboxylate de benzyle.
Spectre de R.M.N.¹H (300 MHz, (CD₃)₂SO d6, δ en ppm) : 1,49 (mt : 2H) ; de 1,65 à 1,85 (mt : 4H) ; 2,11 (dmf, J = 14 Hz : 2H) ; 2,78 (mt : 2H) ; de 3,10 à 3,25 (mt : 2H); 3,20 (t large, J = 7,5 Hz : 2H) ; 3,96 (s : 3H) ; 5,09 (s : 2H) ; 7,28 (mt : 5H) ; 7,43 (d, J = 3 Hz : 1H) ; 7,53 (dd, J = 9 et 3 Hz : 1H) ; 8,06 (d, J = 9 Hz : 1H) ; 8,80 (s : 1H) ; de 9,05 à 9,30 (mf : 2H).

### 1-(2-Bromoéthoxy)-2,3,5-trifluorobenzène

Un mélange de 11,4 g de 2,3,5 trifluorophénol, 40,5 cm³ de dibromo-1,2-éthane et 15,3 g de carbonate de potassium dans 200 cm³ d'acétonitrile est chauffé pendant 18 heures à une température voisine de 75°C, sous agitation et sous atmosphère inerte. Après refroidissement à une température voisine de 20°C, le mélange réactionnel est filtré, lavé avec 4 fois 50 cm³ d'acétonitrile. Le filtrat est concentré à sec sous pression réduite (2 kPa) à une température voisine de 40°C. Le résidu obtenu est repris par 150 cm³ d'éther de pétrole (40-65°C), filtré, lavé par 3 fois 30 cm³ d'éther de pétrole (40-65°C). Le filtrat est concentré à sec sous pression réduite (2 kPa) à une température voisine de 40°C. Le résidu obtenu est purifié par chromatographie sous pression atmosphérique, sur une colonne de gel de silice (granulométrie 20-4S µ ; diamètre 5 cm ; hauteur : 33 cm), en éluant par de l'éther de pétrole (40-65°C) et en recueillant des fractions de 100 cm³. Les fractions 28 à 63 sont réunies puis concentrées à sec selon les conditions ci-dessus. On obtient 16,15 g de 1-(2-bromoéthoxy)-2,3,5-trifluorobenzène sous forme d'une huile épaisse incolore.
Spectre de R.M.N. ¹H (300 MHz, (CD₃)₂SO d6, δ en ppm) : 3,83 (t large, J = 5,5 Hz : 2H) ; 4,45 (t large, J = 5,5 Hz : 2H) ; de 7,00 à 7,15 (mt : 2H).

Le 4-allyl-1-(*tert-*butyioxycarbonyl)-pipéridin-4-carboxylate de benzyle a été préparé dans l'exemple 1.

### Exemple 14

### Acide 4-[3-(3-chloro-6-méthoxyquinolin-4-yl)-propyl]-1-[2-(2,5-difluoro-6-phénylthio)-éthyl]-pipéridin-4-carboxylique

Un mélange de 1,3 g de 4-[3-(3-chloro-6-méthoxyquinolin-4-yl)-propyl]-1-[2-(2,5-difluoro-6- -pipéridin-4-carboxylate de benzyle dans 15 cm³ d'acide chlorhydrique aqueux 5 N et 15 cm³ de dioxanne est porté à une température voisine de 100°C, sous agitation et sous atmosphère inerte pendant 20 heures. Après refroidissement à environ 20°C, le mélange réactionnel est évaporé sous pression réduite (2 kPa) à une température voisine de 40°C. Le résidu obtenu est repris avec 4 fois du toluène en évaporant entre chaque lavage. L'huile obtenue est reprise par un mélange de chloroforme-méthanol-ammoniaque (12/3/0,5 en volumes), décantée, lavée avec de l'eau puis laissée cristalliser pendant 20 heures. Le solide obtenu est filtré puis séché au dessicateur sous pression réduite (0,1 kPa) à une température voisine de 40°C. On obtient 0,6 g d'acide-4-[3-(3-chloro-6-méthoxyquinolin-4-yl)-propyl]-1-[2-(2,5-difluoro-6-phénylthio)-éthyl]-pipéridin-4-carboxylique sous forme d'un solide cristallin blanc fondant à 205°C.
Spectre de R.M.N.¹H (400 MHz, (CD₃)₂SO d6, δ en ppm) : 1,39 (mf : 2H); 1,55 (mt: 2H) ; 1,70 (mt : 2H) ; de 1,90 à 2,20 (mt : 4H) ; de 2,40 à 2,85 (mf étalé : 4H) ; de 3,05 à 3,25 (mt : 2H) ; 3,17 (t large, J = 7,5 Hz : 2H) ; 3,96 (s : 3H) ; 7,07 (mt : 1H) ; 7,26 (mt : 1H) ; 7,33 (mt : 1H) ; 7,38 (d, J = 2,5 Hz : 1H) ; 7,46 (dd, J = 9 et 2,5 Hz : 1H) ; 7,97 (d, J = 9 Hz : 1H) ; 8,68 (s : 1H) ; de 12,00 à 12,50 (mf : 1H) .

### 4-[3-(3-Chloro-6-méthoxyquinolin-4-yl)-propyl]-1-[2-(2,5-difluoro-6-phénylthio)-éthyl]-pipéridin-4-carboxylate de benzyle

Un mélange de 1,4 g de chlorhydrate de 4-[3-(3-chloro-6-méthoxyquinolin-4-yl)-propyl]-pipéridin-4-carboxylate de benzyle, 0,9 g de 1-(2-bromo-éthylthio)-2,5-difluorobenzène, 0,6 g d'iodure de potassium et 2 g de carbonate de potassium dans 100 cm³ d'acétonitrile est chauffé pendant 18 heures à une température voisine de 75°C, sous agitation et sous atmosphère inerte. Après refroidissement à une température voisine de 20°C, le mélange réactionnel est concentré à sec sous pression réduite (2 kPa) à une température voisine de 40°C. Le résidu obtenu est repris par de l'eau, extrait par de l'acétate d'éthyle. La phase organique est lavée avec une solution de chlorure de sodium saturée, séchée sur sulfate de magnésium puis concentrée à sec comme ci-dessus. Le résidu obtenu est purifié par chromatographie sous pression atmosphérique, sur une colonne de gel de silice (granulométrie 70-200 µ ; diamètre 2,5 cm ; masse : 50 g), en éluant par un mélange d'acétate d'éthyle et de dichlorométhane (05/95 en volulmes) et en recueillant des fractions de 15 cm³. Les fractions 21 à 100 sont réunies puis concentrées à sec selon les conditions ci-dessus. On obtient 1,35 g de 4-[3-(3-chloro-6-méthoxyquinolin-4-yl)-propyl]-I-[2-(2,5-difluoro-6-phénylthio)-éthyl]-pipéridin-4-carboxylate de benzyle sous forme d'un solide blanc fondant à 95°C.
Spectre de R.M.N.¹H (300 MHz, (CD₃)₂SO d6, δ en ppm) : de 1,30 à 1,60 (mt : 4H) ; 1,71 (mt : 2H) ; de 1,85 à 2,05 (mt : 4H) ; de 2,40 à 2,60 (mt : 2H) ; 2,66 (dmf, J = 12 Hz : 2H) ; de 3,05 à 3,20 (mt : 2H) ; 3,11 (t large, J = 7,5 Hz : 2H) ; 3,92 (s : 3H) ; 5,03 (s : 2H) ; 7,04 (mt : 1H) ; de 7,15 à 7,35 (mt : 7H) ; 7,35 (d, J = 3 Hz : 1H) ; 7,44 (dd, J = 9 et 3 Hz : 1H) ; 7,95 (d, J = 9 Hz : 1H) ; 8,66 (s : 1H).

### 1-(2-Bromo-éthylthio)-2,5-difluorobenzène

Un mélange de 5,8 g de S-(2,5)-difluoro-phényl-diméthyl-thio-carbamate dans 80 cm³ d'une solution d'hydroxyde de potassium à 10 % dans le méthanol est chauffé pendant 1 heure à une température voisine de 100°C, sous agitation et sous atmosphère inerte. Après refroidissement à une température voisine de 20°C, le mélange réactionnel est concentré à sec sous pression réduite (2 kPa) à une température voisine de 40°C. Le résidu obtenu est repris par de l'eau, extrait par de l'éther diéthylique puis acidifié avec 40 cm³ d'acide chlorhydrique 5N. La phase aqueuse est extraite par 2 fois 30 cm³ d'éther diéthylique. Les phases organiques sont réunies, lavées avec une solution saturée de chlorure de sodium, séchées sur sulfate de sodium, filtrées puis concentrées à sec comme ci-dessus. Le résidu obtenu est repris par 30 cm³ de 1,2 dibromoéthane et 0,5 g d'aliquat 336 puis on coule 20 cm³ d'une solution froide de soude. La solution obtenue est agitée pendant encore 18 heures à une température voisine de 20°C puis lavé par 2 fois 15 cm³ d'eau. Les phases organiques sont réunies, lavées avec une solution saturée de chlorure de sodium, séchées sur sulfate de sodium, filtrées puis concentrées à sec comme ci-dessus. Le résidu obtenu est purifié par chromatographie sous pression atmosphérique, sur une colonne de gel de silice (granulométrie 70-200 µ ; diamètre 2,5 cm ; masse : 75 g), en éluant par de l'éther de pétrole et en recueillant des fractions de 50 cm³. Les fractions 4 à 13 sont réunies puis concentrées à sec selon les conditions ci-dessus. On obtient de 4,4 g de 1-(2-bromo-éthylthio)-2,5-difluorobenzène sous forme d'une huile fluide incolore.
Spectre de R.M.N.¹H (300 MHz, (CD₃)₂SO d6, δ en ppm) : 3,50 (mt : 2H) ; 3,66 (mt: 2H) ; 7,15 (mt : 1H) ; 7,32 (t dédoublé, J = 9 et 4,5 Hz : 1 H) ; 7,42 (ddd, J = 9 - 6,5 et 3 Hz 1H).

### S-(2,5)-Difluoro-phénylthiocarbamate de diméthyle

2 g de O-(2,5)-difluoro-phénylthiocarbamate de diméthyle sont chauffés à une température voisine de 235°C pendant 40 minutes. Le résidu obtenu est purifié par chromatographie sous pression atmosphérique, sur une colonne de gel de silice (granulométrie 70-200 µ ; diamètre 3 cm ; masse : 25 g), en éluant par un mélange d'éther de pétrole et de dichlorométhane (50/50 en volumes) et en recueillant des fractions de 15 cm³. Les fractions 6 à 11 sont réunies puis concentrées à sec sous pression réduite (2 kPa) à une température voisine de 40°C. On obtient 1,25 g de S-2,5)-difluoro-phénylthiocarbamate de diméthyle sous forme d'un solide blanc fondant à 96°C.
Spectre de R.M.N. ¹H (300 MHz, (CD₃)₂SO d6, δ en ppm) : 2,96 (mf : 3H) ; 3,08 (mf : 3H) ; de 7,35 à 7,50 (mt : 3H).

### O-(2,5)-Difluoro-phénylthiocarbamate de diméthyle

A une solution de 7,5 g de 2,5-difluorophénol dans 120 cm³ de diméthylformamide on ajoute sous agitation 14,1 g de chlorure de diméthyl-thiocarbamate et 13 g de 1,4-diaza-(2,2,2)-tricyclo octane. Après 1 heure d'agitation à une température voisine de 20°C le mélange réactionnel est repris par 1 dm³ d'eau et 100 cm³ d'acide chlorhydrique concentré à sec, extrait par 400 cm³ d'éther diéthylique. La phase organique est séchée sur sulfate de sodium, filtrée puis concentrée à sec sous pression réduite (2 kPa) à une température voisine de 40°C. Le résidu obtenu est purifié par chromatographie sous pression atmosphérique, sur une colonne de gel de silice (granulométrie 70-200 µ ; diamètre 10 cm ; masse : 400 g), en éluant par un mélange d'éther de pétrole et de dichlorométhane (50/50 en volumes). Les fractions contenant le produit attendu sont réunies puis concentrées à sec sous pression réduite (5 kPa) à une température voisine de 40°C. On obtient 12,4 g de O-(2,5)-Difluorophénylthiocarbamate de diméthyle sous forme d'un solide fondant à 62°C.
Spectre de R.M.N.¹H (300 MHz, (CD₃)₂SO d6, δ en ppm) : 3,35 (s : 3H) ; 3,40 (s : 3H) ; de 7,15 à 7,30 (mt : 2H) ; 7,42 (t dédoublé, J = 9,5 et 5,5 Hz : 1H).

Le chlorhydrate de 4-[3-(3-chloro-6-méthoxyquinolin-4-yl)-propyl]-pipéridin-4-carboxylate de benzyle a été préparé dans l'exemple 13.

### Exemple 15

### Dichlorhydrate de l'acide 4-[3-(3-chloro-6-méthoxyquinolin-4-yl)-propyl]-1-(2-(2,6-difluorophénoxy)-éthyl]-pipéridin-4-carboxylique

Un mélange de 1 g de 4-[3-(3-chloro-6-méthoxyquinolin-4-yl)-propyl]-I-[2-(2,6-difluorophénoxy)-éthyl]-pipéridin-4-carboxylate de benzyle dans 50 cm³ d'acide chlorhydrique aqueux 5 N est porté à une température voisine de 100°C, sous agitation et sous atmosphère inerte pendant 20 heures. Après refroidissement à environ 20°C, le mélange réactionnel est concentré à sec sous pression réduite (2 kPa) à une température voisine de 40°C. Le résidu obtenu est repris par 50 cm³ d'acétone puis agité pendant 1 heure à une température voisine de 20°C. Le mélange est filtré, lavé avec 3 fois 15 cm³ d'acétone puis séché au dessicateur sous pression réduite (0,1 kPa) à une température voisine de 40°C. On obtient 0,86 g de dichlorhydrate d'acide 4-[3-(3-chloro-6-méthoxyquinolin-4-yl)-propyl]-1-[2-(2,6-difluorophénoxy)-éthyl]-pipéridin-4-carboxylique sous forme d'un solide blanc fondant à 218°C.
Spectre de R.M.N.¹H (300 MHz, (CD₃)₂SO d6, δ en ppm): 1,59 (mt : 2H) ; 1,75 (mt: 2H) ; 1,82 (t trés large, J = 14 Hz : 2H) ; 2,22 (d large, J = 14 Hz : 2H) ; 2,98 (mt : 2H) ; 3,22 (t large, J = 7,5 Hz : 2H) ; de 3,45 à 3,70 (mt : 4H) ; 3,98 (s : 3H) ; 4,51 (t, J = 5 Hz : 2H) ; de 7,15 à 7,25 (mt : 3H) ; 7,42 (d, J = 3 Hz : 1H) ; 7,48 (dd, J = 9 et 3 Hz : 1H) ; 7,99 (d, J = 9 Hz : 1H) ; 8,72 (s : 1H) ; 10,66 (mf : 1H).

### 4-[3-(3-Chloro-6-méthoxyquinolin-4-yl)-propyl]-1-[2-(2,6-difluorophénoxy)-éthyl]-pipéridin-4-carboxytate de benzyle

Un mélange de 1,4 g de chlorhydrate de 4-[3-(3-chloro-6-méthoxyquinolin-4-yl)-propyl]-pipéridin-4-carboxylate de benzyle, 0,82 g de 1-(2-bromo-éthoxy)-2,6-difluorobenzène, 0,6 g d'iodure de potassium et 2 g de carbonate de potassium dans 100 cm³ d'acétonitrile est chauffé pendant 18 heures à une température voisine de 75°C, sous agitation et sous atmosphère inerte. Après refroidissement à une température voisine de 20°C, le mélange réactionnel est filtré, lavé avec 3 fois 30 cm³ d'acétonitrile. Le filtrat est concentré à sec sous pression réduite (2 kPa) à une température voisine de 40°C. Le résidu obtenu est purifié par chromatographie sous pression atmosphérique, sur une colonne de gel de silice (granulométrie 20-45 µ ; diamètre 3 cm ; hauteur : 27 cm), en éluant par de l'acétate d'éthyle et en recueillant des fractions de 50 cm³. Les fractions 7 à 17 sont réunies puis concentrées à sec selon les conditions ci-dessus. On obtient 1,65 g de 4-[3-(3-chloro-6-méthoxyquinolin-4-yl)-propyl]-1-[2-(2,6-difluorophénoxy)-éthyl]-pipéridin-4-carboxylate de benzyle sous forme d'une huile épaisse jaune.
Spectre de R.M.N. ¹H (300 MHz, (CD₃)₂SO d6, δ en ppm) : de 1,25 à 1,55 (mt : 4H) ; 1,70 (mt : 2H) ; de 1,90 à 2,10 (mt : 4H) ; 2,59 (t, J = 6 Hz : 2H) ; 2,66 (dmf, J = 12 Hz : 2H) ; 3,13 (t large, J = 7,5 Hz : 2H) ; 3,93 (s : 3H) ; 4,14 (t, J = 6 Hz : 2H) ; 5,03 (s : 2H) ; de 7,05 à 7,20 (mt : 3H) ; 7,35 (mt : 5H) ; 7,35 (d, J = 3 Hz : 1H) ; 7,46 (dd, J = 9 et 3 Hz : 1H) ; 7,97 (d, J = 9 Hz : 1H) ; 8,67 (s : 1H).

### 1-(2-Bromo-éthoxy)-2,6-difluorobenzène

Un mélange de 10 g de 2,6-difluorophénol, 40,5 cm³ de dibromo-1,2-éthane et 15,3 g de carbonate de potassium dans 300 cm³ d'acétonitrile est chauffé pendant 18 heures à une température voisine de 75°C, sous agitation et sous atmosphère inerte. Après refroidissement à une température voisine de 20°C, le mélange réactionnel est filtré, lavé avec 3 fois 30 cm³ d'acétonitrile. Le filtrat est concentré à sec sous pression réduite (2 kPa) à une température voisine de 40°C. Le résidu obtenu est repris par 200 cm³ d'éther de pétrole (40-65°C), filtré, lavé par 3 fois 30 cm³ d'éther de pétrole (40-65°C). Le filtrat est concentré à sec sous pression réduite (2 kPa) à une température voisine de 40°C. Le résidu obtenu est purifié par chromatographie sous pression atmosphérique, sur une colonne de gel de silice (granulométrie 20-45 µ ; diamètre 7 cm ; hauteur : 28 cm), en éluant par de l'éther de pétrole (40-65°C) et en recueillant des fractions de 100 cm³. Les fractions 22 à 58 sont réunies puis concentrées à sec selon les conditions ci-dessus. On obtient 14,5 g de 1-(2-bromo-éthoxy)-2,6-difluorobenzène sous forme d'une huile épaisse incolore.
Spectre de R.M.N.¹H (300 MHz, (CD₃)₂SO d6, δ en ppm) : 3,76 (t large, J = 6 Hz : 2H) ; 4,42 (t large, J = 6 Hz : 2H) ; de 7,10 à 7,25 (mt : 3H).

Le chlorhydrate de 4-[3-(3-chloro-6-méthoxyquinolin-4-yl)-propyl]-pipéridin-4-carboxylate de benzyle a été préparé dans l'exemple 13.

### Exemple 16

### Dichlorhydrate de l'acide 4-[3-(3-chloro-6-méthoxyquinolin-4-yl)-propyl]-1-[2-(2,5-difluorophénoxy)-éthyl]-pipéridin-4-carboxylique

Un mélange de 1 g de 4-[3-(3-chloro-6-méthoxyquinolin-4-yl)-propyl]-1-[2-(2,5-difluorophénoxy)-éthyl]-pipéridin-4-carboxylate de benzyle dans 50 cm³ d'acide chlorhydrique 5 N est porté à une température voisine de 100°C, sous agitation et sous atmosphère inerte pendant 20 heures. Après refroidissement à environ 20°C, le mélange réactionnel est concentré à sec sous pression réduite (2 kPa) à une température voisine de 40°C. Le résidu obtenu est repris par 50 cm³ d'acétone puis agité pendant 1 heure à une température voisine de 20°C. Le mélange est filtré, lavé avec 3 fois 15 cm³ d'acétone puis séché au dessicateur sous pression réduite (0,1 kPa) à une température voisine de 40°C. On obtient 0,8 g de dichlorhydrate d'acide 4-[3-(3-chloro-6-méthoxyquinolin-4-yl)-propyl]-1-[2-(2,5-difluorophénoxy)-éthyl]-pipéridin-4-carboxylique sous forme d'un solide blanc fondant à 180°C.
Spectre de R.M.N.¹H (400 MHz, (CD₃)₂S0 d6, δ en ppm) : 1,58 (mt : 2H) ; 1,72 (mt: 2H) ; 1,84 (t très large, J = 14 Hz : 2H) ; 2,19 (d large, J = 14 Hz : 2H) ; 2,95 (mt : 2H) ; 3,22 (t large, J = 7,5 Hz : 2H) ; 3,53 (mt : 4H) ; 3,98 (s : 3H) ; 4,52 (t, J = 5 Hz : 2H) ; 6,84 (mt : 1H) ; 7,22 (ddd, J = 9 - 7 et 3 Hz : 1H) ; 7,30 (ddd, J = 10,5 - 9 et 4,5 Hz : 1H) ; 7,42 (d, J = 2,5 Hz : 1H) ; 7,49 (dd, J = 9 et 2,5 Hz : 1H) ; 8,00 (d, J = 9 Hz: 1 H) ; 8,74 (s : 1H) ; 10,97 (mf : 1H) .

### 4-[3-(3-Chloro-6-méthoxyquinolin-4-yl)-propyl]-1-[2-(2,5-difluorophénoxy)-éthyl]-pipéridin-4-carboxylate de benzyle

Un mélange de 1,4 g de chlorhydrate de 4-[3-(3-chloro-6-méthoxyquinolin-4-yl)-propyl]-pipéridin-4-carboxylate de benzyle, 0,82 g de 1-(2-bromo-éthoxy)-2,5-difluorobenzène, 0,6 g d'iodure de potassium et 2 g de carbonate de potassium dans 100 cm³ d'acétonitrile est chauffé pendant 18 heures à une température voisine de 75°C, sous agitation et sous atmosphère inerte. Après refroidissement à une température voisine de 20°C, le mélange réactionnel est filtré, lavé avec 3 fois 30 cm³ d'acétonitrile. Le filtrat est concentré à sec sous pression réduite (2 kPa) à une température voisine de 40°C. Le résidu obtenu est purifié par chromatographie sous pression atmosphérique, sur une colonne de gel de silice (granulométrie 20-45 µ ; diamètre 2,4 cm ; hauteur : 26 cm), en éluant par de l'acétate d'éthyle et en recueillant des fractions de 30 cm³. Les fractions 7 à 14 sont réunies puis concentrées à sec dans les conditions de ci-dessus. On obtient 1,6 g de 4-[3-(3-chloro-6-méthoxyquinolin-4-yl)-propyl]-1-[2-(2,5-difluorophénoxy)-éthyl]-pipéridin-4-carboxylate de benzyle sous forme d'une huile épaisse jaune.
Spectre de R.M.N.¹H (300 MHz, (CD₃)₂SO d6, δ en ppm) : de 1,35 à 1,60 (mt : 4H) ; 1,73 (mt : 2H) ; de 1,95 à 2,15 (mt : 4H) ; 2,63 (t, J = 5,5 Hz : 2H) ; 2,72 (dmf, J = 12 Hz : 2H) ; 3,14 (t large, J = 7,5 Hz : 2H) ; 3,92 (s : 3H) ; 4,12 (t, J = 5,5Hz : 2H) ; 5,04 (s : 2H) ; 6,75 (t détriplé, J = 9 et 3 Hz : 1H) ; 7,13 (ddd, J = 10,5 - 7,5 et 3 Hz : 1H) ; de 7,20 à 7,30 (mt : 6H) ; 7,34 (d, J = 3 Hz : 1H) ; 7,45 (dd, J = 9 et 3 Hz : 1H) ; 7,97 (d, J = 9 Hz : 1H) ; 8,67 (s : 1H).

### 1-(2-Bromo-éthoxy)-2,5-difluorobenzène

Un mélange de 10 g de 2,5-difluorophénol, 40,5 cm³ de dibromo-1,2-éthane et 15,3 g de carbonate de potassium dans 200 cm³ d'acétonitrile est chauffé pendant 18 heures à une température voisine de 75°C, sous agitation et sous atmosphère inerte. Après refroidissement à une température voisine de 20°C, le mélange réactionnel est filtré, lavé avec 4 fois 50 cm³ d'acétonitrile. Le filtrat est concentré à sec sous pression réduite (2 kPa) à une température voisine de 40°C. Le résidu obtenu est repris par 150 cm³ d'éther de pétrole (40-65°C), filtré, lavé par 3 fois 30 cm³ d'éther de pétrole (40-65°C). Le filtrat est concentré à sec sous pression réduite (2 kPa) à une température voisine de 40°C. Le résidu obtenu est purifié par chromatographie sous pression atmosphérique, sur une colonne de gel de silice (granulométrie 20-45 µ ; diamètre 7 cm ; hauteur : 49 cm), en éluant par de l'éther de pétrole (40-65°C) et en recueillant des fractions de 100 cm³. Les fractions 29 à 88 sont réunies puis concentrées à sec dans les conditions de ci-dessus. On obtient 13,5 g de 1-(2-bromo-éthoxy)-2,5-difluorobenzène sous forme d'une huile fluide incolore.
Spectre de R.M.N.¹H (300 MHz, (CD₃)₂SO d6, δ en ppm) : 3,85 (t large, J = 6 Hz : 2H) ; 4,44 (t large, J = 6 Hz : 2H) ; 6,82 (mt : 1H) ; 7,18 (ddd, J = 9 - 7 et 3 Hz : 1 H) ; 7,29 (ddd, J = 11 - 9 et 5 Hz : 1H).

Le chlorhydrate de 4-[3-(3-chloro-6-méthoxyquinolin-4-yl)-propyl]-pipéridin-4-carboxylate de benzyle a éte préparé dans l'exemple 13.

### Exemple 17

### Dichlorhydrate de l'acide 4-[3-(3-chloro-6-méthoxyquinolin-4-yl)-propyl]-1-[2-(2,3-difluorophénoxy)-éthyl]-pipéridin-4-carboxylique

Un mélange de 1 g de 4-[3-(3-chloro-6-méthoxyquinolin-4-yl)-propyl]-1-[2-(2,3-difludrophénoxy)-éthyl]-pipén'din-4-carboxylate de benzyle dans 50 cm³ d'acide chlorhydrique aqueux 5 N est porté à une température voisine de 100°C, sous agitation et sous atmosphère inerte pendant 20 heures. Après refroidissement à environ 20°C, le mélange réactionnel est concentré à sec sous pression réduite (2 kPa) à une température voisine de 40°C. Le résidu obtenu est repris par 50 cm³ d'acétone puis agité pendant 1 heure à une température voisine de 20°C. Le mélange est filtré, lavé avec 3 fois 15 cm³ d'acétone puis séché au dessicateur sous pression réduite (0,1 kPa) à une température voisine de 40°C. On obtient 0,9 g de dichlorhydrate d'acide 4-[3-(3-chloro-6-méthoxyquinolin-4-yl)-propyl]-1-[2-(2,3-difluorophénoxy)-éthyl]-pipéridin-4-carboxylique sous forme d'un solide blanc fondant à 259°C.
Spectre de R.M.N.¹H (400 MHz, (CD₃)₂SO d6, δen ppm) : de 1,45 à 1,65 (mt : 2H) ; 1,73 (mt : 2H) ; 1,81 (t très large, J = 14 Hz : 2H) ; 2,20 (d large, J = 14 Hz : 2H) ; 2,97 (mt : 2H) ; 3,22 (t large, J = 7,5 Hz : 2H) ; de 3,45 à 3,60 (mt : 4H); 3,98 (s : 3H) ; 4,54 (t, J = 5 Hz : 2H) ; 7,09 (mt : 2H) ; 7,20 (mt : 1H) ; 7,42 (d, J = 2,5 Hz : 1H) ; 7,48 (dd, J = 9 et 2,5 Hz : 1H) ; 7,99 (d, J = 9 Hz : 1H) ; 8,72 (s : 1H) ; 10,70 (mf : 1H) ; de 12,40 à 13,30 (mf étalé : 1H).

### 4-[3-(3-Chloro-6-méthoxyquinolin-4-yl)-propyl]-1-[2-(2,3-difluorophénoxy)-éthyl]-pipéridin-4-carboxylate de benzyle

Un mélange de 1,4 g de chlorhydrate de 4-[3-(3-chloro-6-méthoxyquinolin-4-yl)-propyl]-pipéridin-4-carboxylate de benzyle, 0,82 g de 1-(2-bromo-éthoxy)-2,3-difluorobenzène, 0,6 g d'iodure de potassium et 2 g de carbonate de potassium dans 100 cm³ d'acétonitrile est chauffé pendant 18 heures à une température voisine de 75°C, sous agitation et sous atmosphère inerte. Après refroidissement à une température voisine de 20°C, le mélange réactionnel est filtré, lavé avec 3 fois 30 cm³ d'acétonitrile. Le filtrat est concentré à sec sous pression réduite (2 kPa) à une température voisine de 40°C. Le résidu obtenu est purifié par chromatographie sous pression atmosphérique, sur une colonne de gel de silice (granulométrie 20-45 µ; diamètre 2,7 cm ; hauteur : 32 cm), en éluant par de l'acétate d'éthyle et en recueillant des fractions de 30 cm³. Les fractions 7 à 21 sont réunies puis concentrées à sec dans les conditions de ci-dessus. On obtient 1,6 g de 4-[3-(3-chloro-6-méthoxyquinolin-4-yl)-propyl]-1-[2-(2,3-difluorophénoxy)-éthyl]-pipéridin-4-carboxylate de benzyle sous forme d'une huile épaisse jaune.
Spectre de R.M.N. ¹H (300 MHz, (CD₃)₂SO d6, δ en ppm): de 1,35 à 1,55 (mt : 4H) ; 1,72 (mt : 2H) ; de 1,95 à 2,15 (mt : 4H) ; 2,64 (t, J = 6 Hz : 2H) ; 2,72 (dmf, J = 12 Hz : 2H) ; 3,13 (t large, J = 7,5 Hz : 2H) ; 3,93 (s : 3H) ; 4,15 (t, J = 6 Hz : 2H) ; 5,04 (s : 2H) ; de 6,90 à 7,20 (mts : 3H) ; 7,25 (mt : 5H) ; 7,35 (d, J = 3 Hz : 1H) ; 7,46 (dd, J = 9 et 3 Hz : 1H) ; 7,97 (d, J = 9 Hz : 1H) ; 8,67 (s : 1H).

### 1-(2-Bromo-éthoxy)-2,3-difluorobenzène

Un mélange de 10 g de 2,3-difluorophénol, 40,5 cm³ de dibromo-1,2-éthane et 15,3 g de carbonate de potassium dans 200 cm³ d'acétonitrile est chauffé pendant 48 heures à une température voisine de 75°C, sous agitation et sous atmosphère inerte. Après refroidissement à une température voisine de 20°C, le mélange réactionnel est filtré, lavé avec 6 fois 30 cm³ d'acétonitrile. Le filtrat est concentré à sec sous pression réduite (2 kPa) à une température voisine de 40°C. Le résidu obtenu est purifié par chromatographie sous pression atmosphérique, sur une colonne de gel de silice (granulométrie 20-45 µ ; diamètre 7 cm ; hauteur : 42 cm), en éluant par un mélange de dichlorométhane et d'acétate d'éthyle (90/10 en volumes) et en recueillant des fractions de 100 cm³. Les fractions 4 à 10 sont réunies puis concentrées à sec dans les conditions de ci-dessus. Le résidu obtenu est purifié par chromatographie sous pression atmosphérique, sur une colonne de gel de silice (granulométrie 20-45 µ; diamètre 5 cm ; hauteur : 30 cm), en éluant par de l'éther de pétrole (40-65°C) et en recueillant des fractions de 100 cm³. Les fractions 34 à 82 sont réunies puis concentrées à sec selon les conditions ci-dessus. On obtient 13,6 g de 1-(2-bromo-éthoxy)-2,3-difluorobenzène sous forme d'une huile fluide incolore.
Spectre de R.M.N. ¹H (300 MHz, (CD₃)₂SO d6, δ en ppm) : 3,85 (t large, J = 6 Hz : 2H) ; 4,44 (t large, J = 6 Hz : 2H) ; 6,82 (mt : 1H) ; 7,18 (ddd, J = 9 - 7 et 3 Hz : 1H) ; 7,29 (ddd, J = 11 - 9 et 5 Hz : 1 H).

Le chlorhydrate de 4-[3-(3-chloro-6-méthoxyquinolin-4-yl)-propyl]-pipéridin-4-carboxylate de benzyle a éte préparé dans l'exemple 13.

### Exemple 18

### Acide 4-[3-(3-chloro-6-méthoxyquinolin-4-yl)-propyl]-1-(2-thiazol-2-thioéthyl)-pipéridin-4-carboxylique.

Un mélange de 0,3 g de 4-[3-(3-chloro-6-méthoxyquinolin-4-yl)-propyl]-1-(2-thiazol-2-thioéthyl)-pipéridin-4-carboxylate d'éthyle dans 6 cm³ d'acide chlorhydrique aqueux 5 N est porté à une température voisine de 100°C, sous agitation et sous atmosphère inerte pendant 6 heures. Après refroidissement et 16 heures d'agitation à une température voisine de 20°C, le mélange réactionnel est concentré à sec sous pression réduite (2 kPa) à une température voisine de 40°C. Le résidu obtenu est repris par 10 cm³ de dichlorométhane à 10 % de méthanol puis concentré, à sec sous pression réduite (2 kPa) à une température voisine de 40°C. Le résidu obtenu est purifié par chromatographie sous pression atmosphérique, sur une colonne de gel de silice (granulométrie 40-60 µ ; diamètre 2,5 cm ; hauteur : 30 cm), en éluant par un mélange de dichlorométhane-méthanol-ammoniaque (89/10/1 en volumes). Les fractions 18 à 41 sont réunies puis concentrées à sec dans les conditions de ci-dessus. Le résidu obtenu est repris par 5 cm³ d'éther isopropylique, filtré et séché au dessicateur sous pression réduite (0,1 kPa) à une température voisine de 40°C. On obtient 0,24 g d'acide 4-[3-(3-chloro-6-méthoxyquinolin-4-yl)-propyl]-1-(2-thiazol-2-thioéthyl)-pipéridin-4-carboxylique sous forme d'un solide blanc fondant 200°C.
Spectre de R.M.N.¹H (300 MHz, (CD₃)₂SO d6, δ en ppm) : 1,34 (mt : 2H) ; 1,55 (mt: 2H) ; 1,69 (mt : 2H) ; de 1,85 à 2,15 (mt : 4H) ; 2,59 (t large, J = 7 Hz: 2H) ; 2,67 (dmf, J = 12 Hz : 2H); 3,17 (t large, J = 6 Hz: 2H) ; de 3,20 à 3,50 (mt : 2H); 3,96 (s : 3H) ; 7,38 (s large : 1H) ; 7,45 (dd large, J = 9 et 2,5 Hz : 1H) ; 7,63 (d, J = 3 Hz : 1H) ; 7,71 (d, J = 3 Hz : 1H) ; 7,96 (d, J = 9 Hz : 1H) ; 8,68 (s : 1H) ; de 11,90 à 12,55 (mf très étalé: 1H).

### 4-[3-(3-Chloro-6-méthoxyquinolin-4-yl)-propyl]-1-(2-thiazol-2-thioéthyl)-pipéridin-4-carboxylate d'éthyle

A une solution de 0,9 g de 4-[3-(3-chloro-6-méthoxyquinolin-4-yl)-propyl]-1-[2-chloroéthyl]-pipéridin-4-carboxylate d'éthyle dans 50 cm³ d'acétonitrile on ajoute à une température voisine de 20°C, sous agitation et sous atmosphère inerte, 0,28 g de 2 mercaptothiazole et 0,33 g de carbonate de potassium et 0,39 g d'iodure de potassium. Après chauffage pendant 20 heures à une température voisine de 80°C, le mélange réactionnel est refroidi à environ 20°C, filtré, concentré à sec sous pression réduite (2 kPa) à une température voisine de 50°C. Le résidu obtenu est purifié par chromatographie sous une pression d'azote de 100 kPa, sur une colonne de gel de silice (granulométrie 40-60 µ ; diamètre 3,5 cm ; hauteur 35 cm), en éluant par un mélange de dichlorométhane-méthanol (95/0,5 en volumes) et en recueillant des fractions de 40 cm³. Les fractions contenant le produit sont réunies puis purifié par chromatographie sous une pression d'azote de 100 kPa, sur une colonne de gel de silice (granulométrie 40-60 µ ; diamètre 3,5 cm ; hauteur 30 cm), en éluant par un mélange de acétate d'éthyle-cyclohexane (75/25 en volumes) et en recueillant des fractions de 30 cm³. Les fractions 23 à 40 sont réunies puis concentrées à sec comme précédemment. On obtient 0,75 g de 4-[3-(3-chloro-6-méthoxyquinolin-4-yl)propyl]-1-(2-thiazol-2-thioéthyl)pipéridin-4-carboxylate d'éthyle.
Spectre RMN : ¹H (300 MHz, (CD₃)₂SO d6, d en ppm) : 1,05 (t, J = 7 Hz : 3H) ; 1,38 (mt : 2H); 1,52 (mt : 2H); 1,68 (mt : 2H); de 1,90 à 2,05 (mt : 4H) ; 2,59 (t, J = 7 Hz : 2H); 2,68 (d large, J = 12 Hz : 2H) ; 3,17 (t large, J = 7,5 Hz : 2H); de 3,25 à 3,40 (mt : 2H) ; 3,97 (s : 3H) ; 4,00 (q, J = 7 Hz : 2H); 7,38 (d, J = 2,5 Hz: 1H) ; 7,46 (dd, J = 9 et 2,5 Hz : 1H) ; 7,63 (d, J = 3,5 Hz : 1H) ; 7,71 (d, J = 3,5 Hz: 1H); 7,96 (d, J = 9 Hz : 1H) ; 8,68 (s : 1H).

### 4-[3-(3-Chloro-6-méthoxyquinolin-4-yl)propyl]-1-[2-chloroéthyl]pipéridin-4-carboxylate d'éthyle

A une solution de 1,8 g de 4-[3-(3-Chloro-6-méthoxyquinolin-4-yl)propyl]-1-[2-hydroxyéthyl]pipéridin-4-carboxylate d'éthyle dans 40 cm3 de dichlorométhane, on ajoute sous agitation, à une température voisine de 20°C, 1,21 cm3 de chlorure de thionyle. Après 48 heures d'agitation à une température voisine de 20°C, le mélange réactionnel est concentré sous pression réduite (1 kPa) à une température voisine de 50°C. Le résidu est repris par 50 cm3 d'eau et 50 cm3 d'acétate d'éthyle sous vive agitation à une température voisine de 20°C puis on ajoute 10 g de carbonate de potassium. La phase organique est lavée par 25 cm3 d'eau puis par 25 cm3 d'une solution saturée de chlorure de sodium, séchés sur sulfate de magnésium, filtrés, concentrés sous pression réduire (1 kPa) à une température voisine de 50°C. On obtient 1,8 g de 4-[3-(3-Chloro-6-méthoxyquinolin-4-yl)propyl]-1-[2-chloroéthyl] pipéridin-4-carboxylate d'éthyle.
Spectre infra rouge (CCl₄) : 2957 ; 1726 ; 1622 ; 1503 ; 1230 ; 1116 ; 1029 et 833 cm⁻¹

### 4-[3-(3-Chloro-6-méthoxyquinolin-4-yl)propyl]-1-[2-hydroxyéthyl]pipéridin-4-carboxylate d'éthyle

A une solution de 1,17 cm3 de 2 iodoéthanol dans 80 cm3 d'acétonitrile, on ajoute à une température voisine de 20°C, sous agitation et sous atmosphère inerte, 3,9 g de 4-[3-(3-chloro-6-méthoxyquinolin-4-yl)propyl]pipéridin-4-carboxylate d'éthyle et 2,07 g de carbonate de potassium. Après chauffage pendant 23 heures à une température voisine de 80°C, on ajoute 1,17 cm3 de 2 iodoéthanol supplémentaire. Après chauffage pendant 40 heures à une température voisine de 80°C le mélange réactionnel est refroidi à environ 20°C, filtré, concentré sous pression réduite (1 kPa) à une température voisine de 50°C. Le résidu obtenu est purifié par chromatographie sous une pression d'azote de 100 kPa, sur une colonne de gel de silice (granulométrie 40-60 µ ; diamètre 4 cm ; hauteur 60 cm), en éluant par un mélange de dichlorométhane-méthanol-ammoniaque (89/10/1 en volumes) et en recueillant des fractions de 50 cm3. Les fractions 14 à 17 sont réunies puis concentrées comme précédemment. On obtient 1,9 g de 4-[3-(3-Chloro-6-méthoxyquinolin-4-yl)propyl]-1-[2-hydroxyéthyl]pipéridin-4-carboxylate d'éthyle.
Spectre infra-rouge (CCl₄) : 2439 ; 2952 ; 1726 ; 1622; 1503 ; 1230 ; 1116 ; 1029 et 833 cm⁻¹.

### Exemple 19

### Dichlorhydrate de 4-[3-(R,S)-hydroxy-3-(3-chloro-6-méthoxy-quinolin-4-yl)-propyl]-1-[2-cyclopentylthio-éthyl)-piperidin-4-yl]méthanol

Un mélange de 0,6 g Dichlorhydrate de {4-[3-(3-chloro-6-méthoxy-quinolin-4-yl)-3-(R,S)-hydroxy-propyl]-piperidin-4-yl}méthanol, 0,24 g de 1-bromo-2-(cyclopentylthio)-éthyle, 0,22 g d'iodure de potassium et 0,9 g de carbonate de potassium dans 25 cm³ d'acétonitrile est chauffé sous agitation pendant 20 heures à une température voisine de 75°C. Après refroidissement à une température voisine de 20°C, le mélange réactionnel est concentré à sec sous pression réduite (2 kPa) à une température voisine de 40°C. Le résidu obtenu est repris par 20 cm³ d'eau puis extrait par 3 fois 20 cm³ d'acétate d'éthyle. Les phases organiques sont réunies, séchées sur sulfate de magnésium, filtrées puis concentrées à sec comme ci-dessus. Le résidu obtenu est purifié par chromatographie sous une pression d'azote de 50 kPa, sur une colonne de gel de silice (granulométrie 20-45 µ ; diamètre 3 cm ; hauteur : 18 cm), en éluant par un mélange de dichlorométhane-méthanol (90/10 en volumes). Les fractions contenant le produit attendu sont réunies puis concentrées à à sec dans les conditions ci-dessus. Le résidu obtenu est repris dans 5 cm³ d'acétone puis acidifié avec 3 cm³ d'une solution d'acide chlorhydrique N dans l'éther diéthylique. La suspension obtenue est diluée avec 20 cm³ d'éther diéthylique, agitée à une température voisine de 20°C pendant 3 heures, filtrée, lavée par de l'éther diéthylique puis séché au dessicateur sous pression réduite (0,1 kPa) à une température voisine de 40°C. On obtient 0,4 g de dichlorhydrate de 4-[3-(R,S)-hydroxy-3-(3-chloro-6-méthoxy-quinolin-4-yl)-propyl]-1-[2-cyclopentylthio-éthyl)-piperidin-4-yl]méthanol sous forme d'un solide jaune pâle fondant à 120°C.
Spectre de R.M.N.¹H (400 MHz, (CD₃)₂SO d6 avec ajout de quelques gouttes de CD₃COOD d4 à une température de 373K , δ en ppm) : de 1,20 à 2,15 (mt : 16H) ; 2,93 (t large, J = 7,5 Hz : 2H) ; de 3,10 à 3,30 (mt : 7H) ; 3,33 (s large : 2H) ; 3,91 (s : 3H) ; 5,49 (mt : 1H) ; 7,41 (dd, J = 9 et 3 Hz : 1H) ; 7,93 (d, J = 9 Hz : 1H) ; 8,22 (d, J = 3 Hz : 1H) ; 8,60 (s : 1H).

### Dichlorhydrate de {4-[3-(3-chloro-6-méthoxy-quinolin-4-yl)-3-(R,S)-hydroxypropyl]-piperidin-4-yl} méthanol

Un mélange de 1,5 g de 4-(*tert*-butyl-diméthyl-silanyloxyméthyl)-4-[3-(3-chloro-6-méthoxy-quinolin-4-yl)-3-(R,S)-hydroxy-propyl]-pipéridin-1-carboxylate de *tert-*butyle dans 6,5 cm³ d'acide chlorhydrique 5 N en solution dans le dioxanne et 30 cm³ de dioxanne est agité à une température voisine de 20°C pendant 17 heures. On ajoute au mélange réactionnel 100 cm³ d'éther diéthylique. On filtre le précipité formé pour obtenir 1,24 g de dichlorhydrate de {4-[3-(3-chloro-6-méthoxy-quinolin-4-yl)-3-(R,S)-hydroxy-propyl]-piperidin-4-yl}méthanol sous forme d'un solide jaune pâle.
Spectre de R.M.N.¹H (300 MHz, (CD₃)₂SO d6, δ en ppm) : de 1,20 à 2,10 (mt : 8H) ; de 2,85 à 3,10 (mt : 4H) ; 3,26 (s large : 2H) ; 3,91 (s : 3H) ; 5,45 (mt : 1H) ; 7,47 (dd, J = 9 et 3 Hz : 1H) ; 7,98 (d, J = 9 Hz : 1H) ; 8,23 (d, J = 3 Hz : 1H) ; de 8,60 à 8,80 (mf : 2H) ; 8,69 (s : 1H) .

### 4-(tert-Butyl-diméthyl-silanyloxyméthyl)-4-[3-(3-chloro-6-méthoxy-quinolin-4-yl)-3-(R,S)-hydroxy-propyl]-pipéridin-1-carboxylate de tert-butyle

Une solution de 5,26 g de 4-(*tert*-butyl-diméthyl-silanyloxyméthyl)-4-[3-(3-chloro-6-méthoxy-quinolin-4-yl)-propyl]-pipéridin-1-carboxylate de *tert*-butyle dans 300 cm³ de diméthylsulfoxyde et de 75 cm³ de *tert*-butanol est agité sous atmosphère saturée en oxygène à une température voisine de 20°C. Après 5 minutes une solution de 2,1 g de *tert*-butylate de potassium dans 30 cm³ de *tert*-butanol est ajoutée au mélange réactionnel. Après 1 heure et demie de barbotage d'oxygène le mélange réactionnel est versé délicatement sur un mélange de 300 g de glace, 300 cm³ d'eau et 1,1 cm³ d'acide acétique. La phase aqueuse est extraite avec 3 fois 300 cm³ de dichlorométhane. Les phases organiques sont réunies, lavées par 2 fois 300 cm³ d'eau, séchées sur du sulfate de magnésium puis concentrées à sec sous pression réduite (2 kPa) à une température voisine de 40°C. Le résidu obtenu est purifié par chromatographie sous une pression d'azote de 50 kPa, sur une colonne de gel de silice (granulométrie 20-45 µ ; diamètre 4 cm ; hauteur : 27 cm), en éluant par un mélange de cyclohexane-acétate d'éthyle (50/50 en volumes). Les fractions contenant le produit attendu sont réunies puis concentrées à sec dans les conditions de ci-dessus. On obtient 3,4 g de 4-(*tert*-butyl-diméthyl-silanyloxyméthyl)-4-[3-(3-chloro-6-méthoxy-quinolin-4-yl)-3-hydroxy-propyl]-pipéridin-1-carboxylate de *tert*-butyle sous forme d'une meringue blanche.
- Spectre de masse :: EI m/z=578 M⁺
m/z=521 C₂₆H₃₈CIN₂O₅Si⁺
m/z=465 C₂₂H₃₀CIN₂O₅Si⁺
m/z=421 C₂₁H₃₀ClN₂O₃Si⁺ pic de base
m/z=57 C₄H₉⁺
DCI
m/z=579 MH⁺
m/z=523 C₂₆H₄₀ClN₂O₅Si⁺

### 4-(tert-Butyl-diméthyl-silanyloxyméthyl)-4-[3-(3-chloro-6-méthoxy-quinolin-4-yl)-propyl]-pipéridin-1-carboxylate de tert-butyle

Un mélange de 0,45 g de 4-[3-(3-chloro-6-méthoxy-quinolin-4-yl)-propyl]-4-hydroxyméthyl-piperidin-1-carboxylate de *tert*-butyle, 0,3 g de chlorure de *tert-*butyldiméthylsilyl, 0,17 cm³ de triéthylamine et 0,04 g de diméthyl-amino-pyridine dans 20 cm³ de dichlorométhane est agité sous atmosphère inerte à une température voisine de 20°C. Après 18 heures d'agitation on ajoute 0,3 g de chlorure de *tert-*butyldiméthylsilyl, 0,04 g de diméthyl-amino-pyridine et 0,2 cm³ de triéthylamine. Le mélange réactionnel est repris par 20 cm³ d'eau. La phase aqueuse est extraite avec 2 fois 20 cm³ de dichlorométhane. Les phases organiques sont réunies, lavées par 2 fois 300 cm³ d'une solution saturés de chlorure de sodium, séchées sur du sulfate de magnésium puis concentrées à sec sous pression réduite (2 kPa) à une température voisine de 40°C. Le résidu obtenu est purifié par chromatographie sous une pression d'azote de 50 kPa, sur une colonne de gel de silice (granulométrie 20-45 µ ; diamètre 2,5 cm ; hauteur : 15 cm), en éluant par un mélange de cyclohexane-acétate d'éthyle (70/30 en volumes). Les fractions contenant le produit attendu sont réunies puis concentrées à sec dans les conditions de ci-dessus. On obtient 0,48 g de 4-(tert-butyl-diméthyl-silanyloxyméthyl)-4-[3-(3-chloro-6-méthoxy-quinolin-4-yl)-propyl]-pipéridin-1-carboxylate de *tert*-butyle sous forme d'une laque incolore.
Spectre de R.M.N.¹H (300 MHz, (CD₃)₂SO d6, δ en ppm) : -0,04 (s : 6H) ; 0,76 (s : 9H) ; 1,29 (mt : 4H) ; 1,39 (s : 9H) ; 1,58 (mt : 4H) ; 3,19 (mt : 2H) ; de 3,20 à 3,45 (mt : 6H) ; 3,95 (s : 3H) ; 7,41 (d, J = 3 Hz : 1H) ; 7,45 (dd, J = 9 et 3 Hz : 1H) ; 7,95 (d, J = 9 Hz : 1H);8,67(s: 1H).

### 4-[3-(3-Chloro-6-méthoxy-quinolin-4-yl)-propyl]-4-hydroxyméthyl-piperidin-1-carboxylate de tert-butyle

A une solution de 12,3 g de 4-[3-(3-chloro-6-méthoxyquinolin-4-yl)-propyl]-1-(*tert-*butyloxycarbonyl)-pipéridin-4-carboxylate d'éthyle dans 250 cm³ de tétrahydrofurane on ajoute par petites portions sous agitation et sous atmosphère inerte, à une température voisine de -20°C 1,05 g d'hydrure d'aluminium et de lithium. Après 2 heures d'agitation à une température voisine de 20°C, on ajoute au mélange réactionnel à une température voisine de -20°C 0,5 g d'hydrure d'aluminium et de lithium. Après 1 heure et demie le mélange réactionnel est refroidi à 0°C puis est hydrolysé par une addition successive de 1,8 cm³ d'eau, 1,3 cm³ de soude 5N et 5,7 cm³ d'eau. Après 30 minutes d'agitation à une température voisine de 20°C le mélange réactionnel est filtré puis lavé avec 400 cm³ d'acétate d'éthyle. Le filtrat est lavé avec 200 cm³ d'une solution saturée de chlorure de sodium, séché sur sulfate de magnésium puis concentré à sec sous pression réduite (2 kPa) à une température voisine de 40°C. On obtient 10,9 g de 4-[3-(3-chloro-6-méthoxy-quinolin-4-yl)-propyl]-4-hydroxyméthyl-pipéridin-1-carboxylate de *tert-*butyle.
Spectre de R.M.N.¹H (300 MHz, (CD₃)₂SO d6, δ en ppm) : 1,23 (mt : 2H) ; 1,32 (mt: 2H) ; 1,39 (s : 9H) ; 1,58 (mt : 4H) ; de 3,10 à 3,40 (mt : 6H) ; 3,22 (d, J = 5 Hz : 2H) ; 3,96 (s : 3H) ; 4,50 (t, J = 5 Hz : 1H) ; 7,40 (d, J = 3 Hz : 1H) ; 7,45 (dd, J = 9 et 3 Hz : 1H) ; 7,96 (d, J = 9 Hz : 1H) ; 8,68 (s : 1H).

Le 4-[3-(3-chloro-6-méthoxyquinolin-4-yl)-propyl]-1-(*tert*-butyloxycarbonyl)-pipéridin-4-carboxylate d'éthyle a été préparé dans l'exemple 5.

### Exemple 20

### Acide 4-[3-(3-chloro-6-méthoxy-quinolin-4-yl)-3-(R,S)-fluoro-propyl]-1-(3-phényl-propyl)-pipéridin-4-carboxylique

Un mélange de 0,5 g de 4-[3-(3-chloro-6-méthoxy-quinolin-4-yl)-3-(R,S)-fluoro-propyl]-1-(3-phényl-propyl)-pipéridin-4-carboxylate de méthyle dans 8 cm³ d'acide chlorhydrique aqueux 5 N est porté à une température voisine de 100°C, sous agitation pendant 6 heures. Après 18 heures d'agitation à une température voisine 20°C, le mélange réactionnel est concentré à sec sous pression réduite (2 kPa) à une température voisine de 40°C. Le résidu obtenu est repris avec 10 cm³ de dichlorométhane, filtré. Le filtrat est concentré à sec comme ci-dessus, repris dans de l'éther diisopropylique puis séché au dessicateur sous pression réduite (0,1 kPa) à une température voisine de 40°C. Le résidu obtenu est purifié par chromatographie sous une pression d'azote de 60 kPa, sur une colonne de gel de silice (granulométrie 20-45 ; diamètre 2,5 cm ; hauteur: 40 cm), en éluant par un mélange de dichlorométhane-méthanol-ammoniaque (83/15/2). Les fractions 13 à 20 sont réunies puis concentrées à sec dans les conditions de ci-dessus. On obtient 0,24 g d'acide 4-[(3-chloro-6-méthoxy-quinolin-4-yl)-3-(R,S)-fluoro-propyl]-1-(3-phényl-propyl)-pipéridin-4-carboxylique, sous forme d'un solide blanc fondant à 240°C.
Spectre de R.M.N.¹H (300 MHz, (CD₃)₂SO d6, δ en ppm) : de 1,20 à 2,35 et de 2,40 à 2,70 (mts : 18H en totalité) ; 3,94 (s large : 3H) ; 6,36 (2 mts, J_{HF} = 48 Hz : 1H) ; de 7,10 à 7,35 (mt : 5H) ; 7,52 (d large, J = 9 Hz : 1H) ; 7,57 (s large : 1H) ; 8,02 (d large, J = 9 Hz : 1H) ; 8,75 (s large : 1 H).

### 4-[3-(3-Chloro-6-méthoxy-quinolin-4-yl)-3-(R,S)-fluoro-propyl]-1-(3-phényl-propyl)-pipéridin-4-carboxylate de méthyle

A une solution de 0,66 g de 4-[3-(3-chloro-6-méthoxy-quinolin-4-yl)-3-(R,S)-hydroxy-propyl]-l-(3-phényl-propyl)-pipéridin-4-carboxylate de méthyle dans 40 cm³ de dichlorométhane on ajoute sous agitation et sous atmosphère inerte, à une température voisine de -15°C 0,31 cm³ de trifluorure de diéthylaminosulfure. Après 3 heures d'agitation à une température voisine de 20°C, on ajoute au mélange réactionnel 0,31 cm³ de trifluorure de diéthylaminosulfure puis 20 cm³ d'une solution d'hydrogénocarbonate de sodium saturée. La phase aqueuse est extraite avec 20 cm³ de dichlorométhane. Les phases organiques sont réunies, lavées avec une solution saturée de chlorure de sodium, séché sur sulfate de magnésium puis concentré à sec sous pression réduite (2 kPa) à une température voisine de 40°C. Le résidu obtenu est purifié par chromatographie sous une pression d'azote de 100 kPa, sur une colonne de gel de silice (granulométrie 20-45 µ ; diamètre 3,5 cm ; hauteur : 35 cm), en éluant par un mélange de dichlorométhane-méthanol (97/3). Les fractions 15 à 21 sont réunies puis concentrées à sec selon les conditions ci-dessus. On obtient 0,5 g de 4-[3-(3-Chloro-6-méthoxy-quinolin-4-yl)-3-(R,S)-fluoro-propyl]-1-(3-phényl-propyl)-pipéridin-4-carboxylate de méthyle sous forme d'une huile brune.
Spectre de R.M.N.¹H (300 MHz, (CD₃)₂SO d6, δ en ppm) : de 1,30 à 2,35 et de 2,50 à 2,80 (mts : 18H en totalité) ; 3,54 (s : 3H) ; 3,93 (s : 3H) ; 6,36 (ddd, J_{HF} = 48 - 8,5 et 4,5 Hz : 1H) ; 7,19 (mt : 3H) ; 7,29 (t large, J = 7,5 Hz : 2H) ; 7,52 (mt : 2H) ; 8,04 (d, J = 9 Hz : 1H) ; 8,75 (s large : 1H).

### 4-[3-(3-Chloro-6-méthoxy-quinolin-4-yl)-3-(R,S)-hydroxy-propyl]-1-(3-phényl-propyl)-pipéridin-4-carboxylate de méthyle

Un mélange de 1 g de 4-[3-(3-chloro-6-méthoxy-quinolin-4-yl)-3-(R,S)-hydroxy-propyl]-pipéridin-4-carboxylate de méthyle, 0,58 g de 1-bromo-3-phénylpropane et 0,53 g de carbonate de potassium dans 50 cm³ d'acétonitrile est chauffé pendant 22 heures à une température voisine de 75°C, sous agitation et sous atmosphère inerte. Après refroidissement à une température voisine de 20°C, le mélange réactionnel est filtré, lavé avec de l'acétonitrile. Le filtrat est concentré à sec sous pression réduite (2 kPa) à une température voisine de 40°C. Le résidu obtenu est purifiée par chromatographie sous une pression d'azote de 100 kPa, sur une colonne de gel de silice (granulométrie 70-200 µ ; diamètre 3,5 cm ; hauteur 40 cm), en éluant par du dichlorométhane-méthanol (95/05). Les fractions 21 à 48 sont réunies puis concentrées à sec dans les conditions ci-dessus. On obtient 0,7 g 4-[3-(3-chloro-6-méthoxy-quinolin-4-yl)-3-(R,S)-hydroxy-propyl]-1-(3-phényl-propyl)-pipéridin-4-carboxylate de méthyle.
Spectre de R.M.N.¹H (300 MHz, (CD₃)₂SO d6, δ en ppm) : de 1,20 à 2,35 et de 2,45 à 2,75 (mts : 18H en totalité) ; 3,48 (s : 3H) ; 3,88 (s : 3H) ; 5,40 (mt : 1H) ; 6,07 (d, J = 4 Hz : 1H) ; de 7,20 à 7,25 (mt : 3H) ; 7,27 (t large, J = 7,5 Hz : 2H) ; 7,44 (dd, J = 9 et 3 Hz : 1H) ; 9,96 (d, J= 9 Hz : 1H) 8,14 (d, J = 3 Hz : 1H) ; 8,66 (s : 1H).

Le 4-[3-(3-chloro-6-méthoxy-quinolin-4-yl)-3-(R,S)-hydroxy-propyl]-pipéridin-4-carboxylate de méthyle a éte préparé dans l'exemple 49.

### Exemple 21

On prépare le 4-[3-(3-chloro-6-méthoxyquinolin-4-yl)-propyl]-1-[2-(3,5-difluoro-phénylamino)-éthyl]-pipéridine-4-carboxylate de benzyle, sous forme d'huile jaune.
Spectre de R.M.N. ¹H (300 MHz, (CD₃)₂SO d6, δ en ppm) : de 1,35 à 1,60 (mt : 4H) ; 1,74 (mt : 2H) ; de 1,95 à 2,10 (mt : 4H) ; 2,40 (t, J = 7 Hz : 2H) ; de 2,60 à 2,75 (mt : 2H) ; 3,07 (mt : 2H) ; 3,15 (t large, J = 7,5 Hz : 2H) ; 3,94 (s : 3H) ; 5,05 (s : 2H) ; 6,05 (t large, J = 5 Hz : 1H) ; de 6,15 à 6,30 (mt : 3H) ; 7,27 (mt : 5H) ; 7,36 (d, J = 2,5 Hz : 1H) ; 7,47 (dd, J = 9 et 2,5 Hz : 1H) ; 7,98 (d, J = 9 Hz : 1H) ; 8,68 (s : 1H).

### Acide-4-[3-(3-chloro-6-méthoxyquinolin-4-yl)-propyl]-1-[2-(3,5-difluoro-phénylamino)-éthyl]-pipéridine-4-carboxylique

Un mélange de 0,73 g de 4-[3-(3-chloro-6-méthoxyquinolin-4-yl)-propyl]-1-[2-(3,5-difluoro-phénylamino)-éthyl]-pipéridine-4-carboxylate de benzyle dans 12 cm³ d'acide chlorhydrique aqueux 5 N est porté à une température voisine de 100°C sous agitation pendant 3,5 heures. Après refroidissement à environ 20°C, le mélange réactionnel est évaporé sous pression réduite (2 kPa) à une température voisine de 50°C. Le résidu d'évaporation obtenu est repris dans 50 cm³ d'acétone puis est concentré à sec dans les mêmes conditions que précédemment. La meringue obtenue est purifiée par chromatographie sous une pression d'argon de 50 kPa, sur une colonne de gel de silice (granulométrie 20-45 µ ; diamètre 2,5 cm ; hauteur 38 cm), en éluant par un mélange de chloroforme-méthanol-ammoniaque (12/3/0,5 en volumes). Les fractions 31 à 46 sont réunies puis concentrées à sec selon les mêmes conditions que ci-dessus. La poudre obtenue est séchée à l'étuve sous pression réduite (0,1 kPa) à une température voisine de 40°C. On obtient 0,28 g d'acide 4-[3-(3-chloro-6-méthoxyquinolin-4-yl)-propyl]-1-[2-(3,5-difluoro-phénylamino)-éthyl]-pipéridine-4-carboxylique, sous forme d'un solide rose blanc fondant à 210°C.
Spectre de R.M.N. ¹H (300 MHz, (CD₃)₂SO d6, δ en ppm) : 1,45 (t large, J = 12,5 Hz : 2H) ; 1,56 (mt : 2H) ; 1,68 (mt : 2H) ; de 1,90 à 2,10 (mt : 4H) ; 2,41 (t, J = 6,5 Hz : 2H) ; 2,66 (d large, J = 12 Hz : 2H) ; 3,08 (mt : 2H) ; 3,18 (t large, J = 7,5 Hz : 2H) ; 3,97 (s : 3H) ; 6,08 (t large, J = 4,5 Hz : 1H) ; de 6,15 à 6,30 (mt : 3H) ; 7,38 (d, J = 2,5 Hz : 1H) ; 7,45 (dd, J = 9 et 2,5 Hz : 1H) ; 7,96 (d, J = 9 Hz : 1H) ; 8,68 (s : 1H).

### Exemple 22

### 4-[3-(R,S)-hydroxy-3-(3-chloro-6-méthoxyquinolin-4-yl)-propyl]-1-[2-(thien-3-yl)thioéthyl]-pipéridine-4-yl méthanol

Un mélange de 0,6 g de dichlorhydrate de {4-[3-(3-chloro-6-méthoxy-quinolin-4-yl)-3-(R,S)-hydroxy-propyl]-piperidin-4-yl}méthanol, 0,34 g de 2-(2-bromo-éthylthio)-thiophène, 0,95 g de carbonate de potassium et 0,23 g d'iodure de potassium dans 25 cm³ d'acétonitrile est chauffé pendant 17 heures à une température voisine de 80°C, sous agitation et sous atmosphère inerte. Après refroidissement à une température voisine de 20°C, le mélange réactionnel est dilué par 30 cm³ d'eau puis extrait par 2 fois 30 cm³ d'acétate d'éthyle. Les extraits organiques sont réunis, séchés sur sulfate de magnésium, filtrés puis concentrés à sec sous pression réduite (2 kPa) à une température voisine de 40°C. L'huile obtenue est purifiée par chromatographie sous une pression d'argon de 50 kPa, sur une colonne de gel de silice (granulométrie 20-45 µ; diamètre 3 cm ; hauteur 27 cm), en éluant par un mélange de dichlorométhane-méthanol (93/7 en volumes). Les fractions contenant le produit sont réunies puis concentrées à sec dans les conditions ci-dessus. L'huile obtenue est acidifiée par 2 cm³ d'éther chlorhydrique 1N, diluée par 20 cm³ d'éther diéthylique et agitée pendant 1 heure à une température voisine de 20°C. Le solide est filtré, lavé par de l'éther diéthylique puis séché à l'étuve sous pression réduite (10kPa) à une température d'environ 40°C. On obtient 0,28 g de chlorhydrate du 4-[3-(R,S)-hydroxy-3-(3-chloro-6-méthoxyquinolin-4-yl)-propyl]-1-[2-(thien-3-yl)thioéthyl]-pipéridine-4-yl méthanol sous forme d'un solide jaune fondant à 118°C en devenant pâteux.
Spectre de R.M.N. ¹H (400 MHz, (CD₃)₂SO d6, δ en ppm). Nous observons le mélange de deux diastéréoisomères dans les proportions aproximatives 50/50.
* de 1,35 à 2,10 (mt : 8H) ; de 2,85 à 3,60 (mt : 10H) ; 3,86 et 3,87 (2 s : 3H en totalité) ; 5,41 (mt : 1H) ; de 5,80 à 6,30 (mf étalé : 1H) ; 7,10 (mt : 1H) ; 7,29 (mt : 1H) ; 7,43 (dd, J = 9 et 3 Hz : 1H) ; 7,70 (mt : 1H) ; 7,94 (d, J = 9 Hz : 1H) ; 8,19 (s large : 1H) ; 8,64 (s : 1H) ; 9,48 (mf : 1H).

Le dichlorhydrate de {4-[3-(3-chloro-6-méthoxy-quinolin-4-yl)-3-(R,S)-hydroxy-propyl]-piperidin-4-yl}méthanol a été préparé dans l'exemple 19.

### Exemple 23

### 4-[3-(3-Chloro-6-méthoxy-quinolin-4-yl)-propyl]-1-(3-phényl-propyl)-pipéridine-4-carboxamide

A un mélange de 0,75 g d'acide 4-[3-(3-chloro-6-méthoxy-quinolin-4-yl)-propyl]-1-(3-phényl-propyl)-pipéridine-4-carboxylique sous forme de sel de sodium dans 20 cm³ de dichlorométhane, on ajoute 15,6 cm³ d'une solution d'ammoniaque à 0,5 N dans le dioxanne, 0,26 g de 1-hydroxybenzotriazole hydrate, 0,75 g de 1-[3-(diméthylamino)-propyl]-3-éthylcarbodiimide hydrochloride et 0,55 cm³ de triéthylamine. La suspension obtenue est agitée pendant 17 heures à une température voisine de 20°C. Le mélange réactionnel est dilué par 25 cm³ d'eau, agité puis décanté. La phase aqueuse est extraite par 2 fois 25 cm³ de dichlorométhane et les extraits organiques sont réunis, lavés par 25 cm³ de saumure, séchés sur sulfate de magnésium puis concentrés à sec sous pression réduite (2 kPa) à une température voisine de 40°C. Le résidu d'évaporation obtenu est purifié par chromatographie sous une pression d'argon de 50 kPa, sur une colonne de gel de silice (granulométrie 20-45 µ ; diamètre 3 cm ; hauteur 21 cm), en éluant par un mélange de dichlorométhane-méthanol (90/10 en volumes). Les fractions contenant le produit sont réunies puis concentrées à sec dans les conditions ci-dessus. On obtient 0,22 g de 4-[3-(3-chloro-6-méthoxy-quinolin-4-yl)-propyl]-1-(3-phényl-propyl)-pipéridine-4-carboxamide, sous forme d'un solide blanc fondant à 160°C.
Spectre de R.M.N. ¹H (400 MHz, (CD₃)₂SO d6, δ en ppm) : 1,33 (t large, J = 12 Hz : 2H) ; 1,53 (mt : 2H) ; 1,67 (mt : 4H) ; 1,98 (mt : 4H) ; 2,20 (t, J = 7,5 Hz: 2H) ; de 2,40 à 2,60 (mt : 4H) ; 3,15 (t, J = 7,5 Hz : 2H) ; 3,97 (s : 3H) ; 6,87 (s : 1H) ; 7,10 (s : 1H) ; 7,17 (mt: 1H) ; 7,20 (d large, J = 7,5 Hz : 2H) ; 7,27 (t large, J = 7,5 Hz : 2H) ; 7,37 (d, J = 2,5 Hz: 1H) ; 7,45 (dd, J = 9 et 2,5 Hz : 1H) ; 7,96 (d, J = 9 Hz: 1H) ; 8,68 (s : 1H).

Le dichlorhydrate de l'acide 4-[3-(3-chloro-6-méthoxy-quinolin-4-yl)-propyl]-1-(3-phényl-propyl)-pipéridine-4-carboxylique a été préparé dans l'exemple 4.

### Exemple 24

### Acide 4-[3-(3-chloro-6,7-diméthoxy-quinolin-4-yl)-propyl]-1-[2-(thiophène-2-ylthio)-éthyl]-pipéridine-4-carboxylique

A une solution de 1,47 g de 4-[3-(3-chloro-6,7-diméthoxy-quinolin-4-yl)-propyl]-1-[2-(thiophène-2-ylthio)-éthyl]-pipéridine-4-carboxylate d'éthyle dans 20 cm³ de dioxanne, on ajoute sous agitation et à une température voisine de 20°C, 26 cm³ de soude aqueuse 5N et 20 cm³ de méthanol, puis on porte l'ensemble à une température d'environ 70°C pendant 21 heures. Le mélange réactionnel est concentré à sec sous pression réduite (2 kPa) à une température voisine de 40°C. Le résidu obtenu est purifié par chromatographie sous une pression d'argon de 50 kPa, sur une colonne de gel de silice (granulométrie 40-63 µ ; masse 60g), en éluant par un mélange de dichlorométhane-méthanol-ammoniaque (12/3/0,5 en volumes). Les fractions 11 à 25 sont réunies puis concentrées à sec dans les conditions ci-dessus. On obtient 1,03 g d'acide 4-[3-(3-chloro-6,7-diméthoxy-quinolin-4-yl)-propyl]-1-[2-(thiophène-2-ylthio)-éthyl]-pipéridine-4-carboxylique sous forme d'un solide blanc fondant à 214°C.
Spectre de R.M.N. ¹H (300 MHz, (CD₃)₂SO d6, δ en ppm) : 1,27 (t très large, J = 12 Hz : 2H) ; de 1,45 à 1,75 (mt : 4H) ; de 1,85 à 2,10 (mt : 4H) ; 2,44 (mt : 2H) ; de 2,50 à 2,65 (mt : 2H) ; 2,89 (t large, J = 7,5 Hz : 2H) ; 3,13 (mt : 2H) ; 3,93 (s : 3H) ; 3,96 (s : 3H) ; 7,02 (dd, J = 5,5 et 3,5 Hz : 1H) ; 7,15 (dd, J = 3,5 et 1 Hz : 1H) ; 7,30 (s large: 1H) ; 7,38 (s large : 1H) ; 7,58 (dd, J = 5,5 et 1 Hz : 1H) ; 8,58 (s : 1H).

### 4-[3-(3-Chloro-6,7-diméthoxy-quinolin-4-yl)-propyl]-1-[2-(thiophène-2-ylthio)-éthyl]-pipéridine-4-carboxylate d'éthyle

Un mélange de 1,65 g de 4-[3-(3-chloro-6,7-diméthoxy-quinolin-4-yl)-pipéridine-4-carboxylate d'éthyle, 1,16 g de 2-(2-bromo-éthylthio)-thiophène, 1,63 g de carbonate de potassium et 0,685 g d'iodure de potassium dans 50 cm³ d'acétonitrile est agité pendant 23 heures à une température voisine de 20°C. La suspension est filtrée puis le filtrat est concentré à sec sous pression réduite (2 kPa) à une température voisine de 40°C. Le résidu d'évaporation est repris par 50 cm³ d'acétate d'éthyle, lavé par 2 fois 50 cm³ d'eau et par 40 cm³ d'une solution aqueuse saturée en chlorure de sodium, séché sur du sulfate de magnésium, filtré, concentré à sec dans les conditions ci-dessus. L'huile obtenue est purifiée par chromatographie sous pression d'argon de 50 kPa, sur une colonne de gel de silice (granulométrie 40-63 µ ; diamètre 4 cm) en éluant par un mélange de dichlorométhane-méthanol-acétonitrile (95/2,5/2,5 en volumes). Les fractions contenant le produit sont réunies puis concentrées à sec sous pression réduite (2 kPa) à une température voisine de 40°C. On obtient 1,47 g de 4-[3-(3-chloro-6,7-diméthoxy-quinolin-4-yl)-propyl]-1-[2-(thiophène-2-ylthio)-éthyl]-pipéridine-4-carboxylate d'éthyle, sous forme d'une huile.
Spectre de R.M.N. ¹H (300 MHz, (CD₃)₂SO d6, δ en ppm) : 1,05 (t, J = 7 Hz : 3H) ; 1,37 (t large, J = 12 Hz : 2H) ; 1,52 (mt : 2H) ; de 1,60 à 1,75 (mt : 2H) ; de 1,85 à 2,05 (mt : 4H) ; 2,46 (mt : 2H) ; 2,61 (d large, J = 12 Hz : 2H) ; 2,91 (mt : 2H) ; 3,15 (t large, J = 7,5 Hz : 2H) ; 3,95 (s : 3H) ; 3,97 (s : 3H) ; 4,00 (q, J = 7 Hz : 2H) ; 7,04 (dd, J = 5,5 et 3,5 Hz : 1H) ; 7,17 (dd, J = 3,5 et 1 Hz : 1H) ; 7,31 (s large : 1H) ; 7,40 (s large : 1H) ; 7,60 (dd, J = 5,5 et 1 Hz : 1H) ; 8,61 (s : 1H).

### 4-[3-(3-Chloro-6,7-diméthoxy-quinolin-4-yl)-pipéridine-4-carboxylate d'éthyle

A un mélange de 2,05 g de 4-[3-(3-chloro-6,7-diméthoxy-quinolin-4-yl)-propyl]-1-(*tert*-butyloxycarbonyl)-pipéridine-4-carboxylate d'éthyle dans 20 cm³ de dichlorométhane, on ajoute goutte à goutte 3 cm³ d'acide trifluoroacétique sous agitation et à une température voisine de 20°C. Au bout de 2 heures, le mélange réactionnel est concentré à sec sous pression réduite (2 kPa) à une température voisine de 40°C. Le résidu est repris par 20 cm³ d'eau et par 10 cm³ d'acétate d'éthyle puis extrait avec 2 fois 10 cm³ d'une solution aqueuse d'acide chlorhydrique 1N. La phase aqueuse est alcalinisée avec une solution aqueuse de soude aqueuse à 30 % jusqu'à ce que le pH soit environ de 10 puis extraite par 3 fois 30 cm³ d'acétate d'éthyle. Les extraits organiques sont réunis, séchés sur sulfate de magnésium, filtrés puis concentrés à sec comme ci-dessus. On obtient 1,65 g de 4-[3-(3-chloro-6,7-diméthoxy-quinolin-4-yl)-pipéridine-4-carboxylate d'éthyle, sous forme d'une huile jaune.
- Spectre de masse :: El m/z=420 M⁺ m/z=364 C₁₉H₂₃ClNO₄⁺
m/z=264 C₁₄H₁₅ClNO₂⁺ m/z=237 C₁₂H₁₂ClNO₂⁺pic de base
m/z=184 C₁₀H₁₈NO₂⁺

### 4-[3-(3-Chloro-6,7-diméthoxy-quinolin-4-yl)-propyl]-1-(tert-butyloxycarbonyl)pipéridine-4-carboxylate d'éthyle

On refroidit à une température voisine de 0°C, 21 cm³ d'une solution à 0,5 M de 9-borabicyclo-[3,3,1]-nonane et l'on ajoute goutte à goutte, sous agitation et sous atmosphère inerte, 2,61 g de 4-allyl-1-(*tert*-butyloxycarbonyl)-pipéridine-4-carboxylate d'éthyle dans 26 cm³ de tétrahydrofurane en maintenant la température aux environs de 0°C. Après l'addition, la température du mélange est ramenée à environ 20°C. La solution obtenue est agitée pendant encore 4 heures à cette température, puis l'on ajoute 37 cm³ de dioxanne, 0,19 g de chlorure de palladium diphénylphosphino ferrocène, 3,05 g de 4-bromo-3-fluoro-6-méthoxy-quinoléine et 5,58 g de phosphate de potassium tribasique. Après 24,75 heures d'agitation à une température voisine de 60°C, le mélange réactionnel est refroidi au voisinage de 20°C, filtré puis le filtrat est concentré à sec sous pression réduite (2 kPa) à une température voisine de 40°C. Le résidu obtenu est repris par 100 cm³ d'acétate d'éthyle et par 100 cm³ d'eau, décanté, puis par 100 cm³ d'une solution saturée de chlorure de sodium. La phase organique est séchée sur sulfate de magnésium, filtrée, concentrée à sec sous pression réduite (2 kPa) à une température voisine de 40°C. L'huile obtenue est purifiée par chromatographie, sous pression d'argon de 50 kPa, sur une colonne de gel de silice (granulométrie 40-63 µ ; volume 690 cm³), en éluant par un mélange de dichlorométhane-méthanol-acétonitrile (98/1/1 en volumes). Les fractions contenant le produit sont réunies puis concentrées à sec selon les mêmes conditions que ci-dessus. On obtient 2,16 g de 4-[3-(3-chloro-6,7-diméthoxy-quinolin-4-yl)-propyl]-1-(*tert*-butyloxycarbonyl)pipéridine-4-carboxylate d'éthyle, sous forme d'un gel jaune.
Spectre de R.M.N. ¹H (300 MHz, (CD₃)₂SO d6, δ en ppm) : 1,06 (t, J = 7 Hz : 3H) ; de 1,25 à 1,45 (mt : 2H) ; 1,39 (s : 9H) ; 1,53 (mt : 2H) ; 1,69 (mt : 2H) ; 1,93 (d large, J = 13,5 Hz : 2H) ; 2,82 (mf : 2H) ; 3,14 (t large, J = 7,5 Hz : 2H) ; 3,69 (d large, J = 13,5 Hz : 2H) ; 3,94 (s : 3H) ; 3,97 (s : 3H) ; 4,03 (q, J = 7 Hz : 2H) ; 7,31 (s large : 1H) ; 7,40 (s large : 1H) ; 8,61 (s : 1H).

### 4-Bromo-3-chloro-6,7-diméthoxy-quinoléine

A une solution de 5,39 g de 3-chloro-4-hydroxy-6,7-diméthoxy-quinoléine dans 270 cm³ d'acétonitrile, on ajoute sous agitation et à une température voisine de 20°C, 19 g de triphénylphosphine dibromure. Le mélange réactionnel est chauffé aux environs de 80°C pendant 8,25 heures. Après refroidissement à une température voisine de 20°C, l'insoluble est filtré. On obtient 5,67 g de 4-bromo-3-chloro-6,7-diméthoxy-quinoléine, sous forme d'un solide gris.
Spectre de masse : DCI m/z=302 MH⁺

### 3-Chloro-4-hydroxy-6,7-diméthoxy-quinoléine

A une solution de 6,7-diméthoxy-quinolin-4-ol dans 300 cm³ d'acide acétique, on ajoute sous agitation et à une température voisine de 20°C, 5,45 g de N-chlorosuccinimide. Le mélange réactionnel est chauffé à une température voisine de 50°C pendant 6 heures. Après refroidissement au voisinage de 20°C et agitation pendant 18 heures à cette même température, le mélange réactionnel est concentré à sec sous pression réduite (2 kPa) à une température voisine de 40°C. Au résidu d'évaporation est ajoutée goutte à goutte 100 cm³ d'une solution d'hydrogénocarbonate de sodium puis la suspension est agitée pendant 24 heures au voisinage de 20°C. L'insoluble est filtré puis séché à l'étuve sous pression réduite (20 Pa). On obtient 5,39 g de 3-chloro-4-hydroxy-6,7-diméthoxy-quinoléine, sous forme d'un solide vert foncé.
Spectre de masse: DCI m/z=240 MH⁺

### 6,7-Diméthoxy-4-hydroxy-quinoléine

Un mélange de 9,24 g d'acide 4-hydroxy-6,7-diméthoxy-quinoline-3-carboxylique dans 185 cm³ de diphényléther est porté, sous agitation mécanique, à une température voisine de 250°C pendant 1,3 heures. Après refroidissement du mélange réactionnel aux environs de 20°C, on ajoute 100 cm³ d'éther diisopropylique. La suspension est agitée pendant 8 heures, filtrée et le gâteau est lavé par 3 fois 100 cm³ d'éther düsopropylique. On obtient 7,96 g de 6,7-diméthoxy-quinolin-4-ol, sous forme d'un solide marron.
Spectre infra rouge (KBr) : 3242; 1605; 1548; 1500; 1273; 1239; 1220; 1075 et 822 cm⁻¹

### Acide 4-hydroxy-6,7-diméthoxy-quinoline-3-carboxylique

A un mélange de 21,1 g de 2-[(3,4-diméthoxy-phénylamino)-méthylène]-malonate de diéthyle dans 42 cm³ de nitrobenzène, on ajoute précautionneusement, sous agitation mécanique, 10,2 g de pentaoxyde de phosphore. Le mélange réactionnel est porté à une température voisine de 150°C pendant 4 heures. Après refroidissement aux environs de 20°C et agitation pendant 18 heures à cette même température, on verse goutte à goutte 14 cm³ d'eau puis le mélange réactionnel est chauffé au voisinage de 110°C pendant 7 heures. Après refroidissement à une température voisine de 20°C, on ajoute 42 cm³ d'acétate d'éthyle. La suspension est agitée dans ces conditions pendant 18 heures puis décantée, filtrée et le gâteau est lavé par 3 fois 40 cm³ d'acétate d'éthyle, 40 cm³ d'éthanol et 40 cm³ d'eau. On obtient 9,25 g d'acide 4-hydroxy-6,7-diméthoxy-quinoline-3-carboxylique, sous forme d'un solide crème.
Spectre infra rouge (KBr) : 2842; 1673; 1631; 1590; 1503; 1437; 1279; 1220; 1006; 807 ; 586 et 357 cm⁻¹.

### 2-[(3,4-Diméthoxy-phénylamino)-méthylène]-malonate de diéthyle

Un mélange de 10 g de 3,4 diméthoxyaniline dans 13,2 cm³ d'éthoxyméthylènemalonate de diéthyle est agité pendant 1,5 heures à une température voisine de 110°C. Après refroidissement au voisinage de 20°C, le mélange réactionnel est concentré à sec sous pression réduite (2 kPa) à une température voisine de 50°C. On obtient 21,1 g de 2-[(3,4-diméthoxy-phénylamino)-méthylène]-malonate de diéthyle, sous forme d'un solide marron.

### Spectre de masse : DCI m/z=324 MH⁺

La 4-allyl-1-(*tert*-butyloxycarbonyl)-pipéridine-4-carboxylate d'éthyle a été préparé dans l'exemple 1.

### Exemple 25

### Monochlorhydrate de l'acide 4-[3-(3-chloro-6-méthoxy-quinolin-4-yl)-3-(R,S)-hydroxy-propyl]-1-[2-(2,5-difluoro-phénylthio)-éthyl]-pipéridine-4-carboxylique

A une solution de 0,6 g de (R,S)-3-(3-chloro-6-méthoxy-quinolin-4-yl)-9-[2-(2,5-difluoro-phénylthio)-éthyl]-2-oxa-9-aza-spiro[5.5]undecan-1-one dans 50 cm³ de d'acétone, on ajoute sous agitation et à une température voisine de 20°C, 5 cm³ de soude aqueuse 5N. Au bout de 2 heures, le mélange réactionnel est concentré à sec sous pression réduite (2 kPa) à une température voisine de 40°C. Le résidu d'évaporation est trituré avec 10 cm³ d'éther diéthylique, filtré et le gâteau est lavé par de l'éther diéthylique puis séché à l'étuve sous pression réduite (10 Pa) à une température voisine de 50°C. Le solide est repris par de l'eau et acidifié par une solution aqueuse d'acide chlorhydrique 1N jusqu'à ce que le pH soit égal à 3. On ajoute à une température voisine de 20°C du dichlorométhane avec une agitation assez forte puis le solide est filtré. On obtient 0,32 g de monochlorhydrayte de l'acide 4-[3-(3-chloro-6-méthoxy-quinolin-4-yl)-3-(R,S)-hydroxy-propyl]-1-[2-(2,5-difluoro-phénylthio)-éthyl]-pipéridine-4-carboxylique, sous forme d'un solide blanc.
Spectre de R.M.N. ¹H (300 MHz, (CD₃)₂SO d6, δ en ppm) : de 1,30 à 2,35 (mt : 8H) ; 2,84 (mf : 2H) ; de 3,05 à 3,70 (mt : 6H) ; 3,90 (s large : 3H) ; 5,43 (mf : 1H) ; 6,16 (mf: 1H) ; 7,14 (mf : 1H) ; 7,33 (mf : 1H) ; 7,46 (mt : 2H) ; 7,96 (d large, J = 9 Hz : 1H) ; 8,16 (s large : 1H) ; 8,67 (s large : 1H) ; de 10,20 à 10,60 (mf étalé : 1H).

### 3-(3-Chloro-6-méthoxy-quinolin-4-yl)-9-[2-(2,5-difluoro-phénylthio)-éthyl]-2-oxa-9-aza-spiro[5.5]undecan-1-one

Un mélange de 1,75 g de dichlorhydrate de 4-[3-(3-chloro-6-méthoxyquinolin-4-yl)-(R,S)-propyl]-pipéridine-4-carboxylate d'éthyle, 0,95 g de 2-(2-bromo-éthylthio)-1,4-difluoro-benzène, 0,62 g d'iodure de potassium et 3,11 g de carbonate de potassium dans 30 cm³ d'acétonitrile et 20 cm³ de dimétylformamide est chauffé sous agitation pendant 18 heures à une température voisine de 85°C. Après refroidissement aux environs de 20°C, le mélange réactionnel est filtré sur célite puis le filtrat est concentré à sec sous pression réduite (2 kPa) à une température voisine de 40°C. Le résidu obtenu est repris par de l'eau et de l'éther diéthylique. La phase organique est séchée sur sulfate de magnésium, filtrée puis concentrée à sec comme ci-dessus. Le résidu obtenu est purifié par chromatographie sous pression atmosphérique, sur une colonne de gel de silice (granulométrie 70-200 µ ; diamètre 4 cm), en éluant par un mélange de dichlorométhane-méthanol (97,5/2,5 en volumes). Les fractions contenant le produit sont réunies puis concentrées à sec dans les conditions ci-dessus. On obtient 0,6 g de 3-(3-chloro-6-méthoxy-quinolin-4-yl)-9-[2-(2,5-difluoro-phénylthio)-éthyl]-2-oxa-9-aza-spiro[5.5]undecan-1-one.
Spectre de masse : EI m/z=532 M⁺ m/z=373 C₂₀H₂₂CIN₂O₃⁺ pic de base

Le 2-(2-bromo-éthylthio)-1,4-difluoro-benzène a été préparé dans l'exemple 14.

La 4-[3-(3-chloro-6-méthoxy-quinolin-4-yl)-3-(R,S)-hydroxy-propyl]-pipéridine-4-carboxylate de méthyle a été préparé dans l'exemple 49.

### Exemple 26

### Acide 4-[3-(3-chloro-6-méthoxy-quinolin-4-yl)-3-(R,S)-tluoro-propyl]-1-[2-(3,5-difluoro-phénoxy)-éthyl]-pipéridine-4-carboxylique

Un mélange de 0,4 g de 4-[3-(3-chloro-6-méthoxy-quinolin-4-yl)-3-(R,S)-fluoro-propyl]-1-[2-(3,5-difluoro-phénoxy)-éthyl]-pipéridine-4-carboxylate de méthyle dans 6,6 cm³ d'acide chlorhydrique aqueux 5 N est porté à une température voisine de 100°C sous agitation pendant 6 heures. Après refroidissement aux environs de 20°C, le mélange réactionnel est agité pendant 20 heures à cette même température puis concentré à sec sous pression réduite (2 kPa) à une température voisine de 40°C. Le résidu est repris par un mélange de dichlorométhane-méthanol (90/10 en volumes) puis est de nouveau concentré à sec dans les conditions ci-dessus. Le résidu d'évaporation est purifié par chromatographie sous une pression d'argon de 60 kPa, sur une colonne de gel de silice (granulométrie 40-60 µ ; diamètre 2,5 cm ; hauteur 41 cm), en éluant par un mélange de dichlorométhane-méthanol-ammoniaque à 28 % (83/15/2 en volumes) et en recueillant des fractions de 30 cm³. Les fractions 16 à 22 sont réunies puis concentrées à sec dans les conditions précédentes. Le résidu est trituré par 10 cm³ d'éther diisopropylique, filtré et séché. On obtient 0,21 g d'acide 4-[3-(3-chloro-6-méthoxy-quinolin-4-yl)-3-(R,S)-fluoro-propyl]-1-[2-(3,5-difluoro-phénoxy)-éthyl]-pipéridine-4-carboxylique, sous forme d'un solide blanc cassé fondant à 205°C.
Spectre de R.M.N. ¹H (300 MHz, (CD₃)₂SO d6, δ en ppm) : de 1,20 à 1,70 (mt : 3H) ; de 1,80 à 2,40 (mt : 7H); de 2,30 à 2,80 (mt : 2H) ; 2,61 (t, J = 6 Hz : 2H) ; 3,92 (s : 3H) ; 4,17 (t, J = 6 Hz : 2H) ; 6,36 (d mt, J_{HF} = 48 Hz : 1H) ; de 6,65 à 6,85 (mt : 3H) ; 7,51 (dd, J = 9 et 2,5 Hz : 1H) ; 7,55 (s large : 1H) ; 8,02 (d, J = 9 Hz : 1H) ; 8,74 (s : 1H).

### 4-[3-(3-Chloro-6-méthoxy-quinolin-4-yl)-3-(R,S)-tluoro-propyl]-1-[2-(3,5-difluoro-phénoxy)-éthyl]-pipéridine-4-carboxylate de méthyle

A une solution de 1,1 g de 4-[3-(3-chloro-6-méthoxy-quinolin-4-yl)-3-(R,S)-hydroxy-propyl]-1-[2-(3,5-difluoro-phénoxy)-éthyl]-pipéridine-4-carboxylate de méthyle dans 50 cm³ de dichlorométhane, on ajoute sous agitation et sous atmosphère inerte, à une température voisine de -10°C, 0,4 cm³ de trifluorure de diéthylaminosulfure. Après 4 heures d'agitation à une température voisine de 20°C, on ajoute au mélange réactionnel 0,4 cm³ de trifluorure de diéthylaminosulfure puis le mélange est agité pendant 20 heures à cette même température. On ajoute 20 cm³ d'une solution aqueuse d'hydrogénocarbonate de sodium saturée. La phase aqueuse est extraite avec 25 cm³ de dichlorométhane. La phase organique est lavée avec 2 fois 25 cm³ d'une solution aqueuse de chlorure de sodium à 10 % (en poids), séchée sur sulfate de magnésium, filtrée puis concentrée à sec sous pression réduite (2 kPa) à une température voisine de 40°C. L'huile obtenue est purifiée par chromatographie sous une pression d'argon de 100 kPa, sur une colonne de gel de silice (granulométrie 40-60 µ; diamètre 3,5 cm ; hauteur : 35 cm), en éluant par un mélange de dichlorométhane-méthanol (97/3) et en recueillant des fractions de 35 cm³. Les fractions 20 à 21 sont réunies puis concentrées à sec dans les conditions ci-dessus. On obtient 0,62 g de 4-[3-(3-chloro-6-méthoxy-quinolin-4-yl)-3-(R,S)-fluoro-propyl]-l-[2-(3,5-difluoro-phénoxy)-éthyl]-pipéridine-4-carboxylate de méthyle, sous forme d'une huile orangée.
Spectre infra rouge (CH₂Cl₂) : 2953 ; 1725 ; 1622 ; 1599 ; 1504 ; 1466 ; 1234 ; 1152 ; 1117 et 837 cm⁻¹ .

### 4-[3-(3-Chloro-6-méthoxy-quinolin-4-yl)-3-(R,S)-hydroxy-propyl]-1-[2-(3,5-difluoro-phénoxy)-éthyl]-pipéridine-4-carboxylate de méthyle

A un mélange de 1,1 g de 3-(3-chloro-6-méthoxy-quinolin-4-yl)-9-[2-(3,5-difluoro-phénoxy)-éthyl]-2-oxa-9-aza-spiro[5.5]undecan-1-one dans 20 cm³ de méthanol refroidi à -20°C, sous agitation et atmosphère inerte, on ajoute goutte à goutte 0,47 cm³ de chlorure de thionyle puis l'ensemble est agité pendant 24 heures à une température voisine de 20°C. Le mélange réactionnel est concentré à sec sous pression réduite (2 kPa) à une température voisine de 40°C. Le résidu est repris par 30 cm³ d'acétate d'éthyle et par 15 cm³ d'eau. Après addition de 5 g de carbonate de potassium et une agitation vive pendant 5 minutes, le mélange est décanté, la phase organique est séparée, lavée par 3 fois 15 cm³ d'une solution aqueuse de chlorure de sodium à 10 % (en poids). Après séchage sur sulfate de magnésium, puis filtration, la solution organique est concentrée à sec sous pression réduite (2 kPa) à une température voisine de 40°C. On obtient 1,12 g de 4-[3-(3-chloro-6-méthoxyquinolin-4- yl)-3-(R,S)-hydroxy-propyl]-1-[2-(3,5-difluoro-phénoxy)-éthyl]-pipéridine-4-carboxylate de méthyle, sous forme d'une laque brune.
Spectre infra rouge (CCl₄) : 3615 ; 2952 ; 1732 ; 1622 ; 1600 ; 1466 ; 1233 ; 1154 ; 1117 ; 841 et 671 cm⁻¹.

### 3-(3-Chloro-6-méthoxy-quinolin-4-yl)-9-[2-(3,5-ditluoro-phénoxy)-éthyl]-2-oxa-9-aza-spiro[5.5]undecan-1-one

Un mélange de 1 g de 4-[3-(3-chloro-6-méthoxy-quinolin-4-yl)-3-(R,S)-hydroxy-propyl]-pipéridine-4-carboxylate de méthyle, 0,8 g de 1-(2-bromo-éthoxy)-3,5-difluoro-benzène et de 0,42 g de carbonate de potassium dans 45 cm³ d'acétonitrile est porté à une température de 80°C, sous agitation et atmosphère d'argon pendant 20 heures. La suspension est filtrée, l'insoluble est lavé par de l'acétate d'éthyle puis le filtrat est concentré à sec sous pression réduite (2 kPa) à une température voisine de 40°C. Le résidu est purifié par chromatographie sous une pression d'argon de 100 kPa, sur une colonne de gel de silice (granulométrie 40-60 µ ; diamètre 3,5 cm ; hauteur : 38 cm), en éluant par un mélange de dichlorométhane-méthanol (97/4 en volumes) et en recueillant des fractions de 35 cm³. Les fractions 9 à 20 sont réunies puis concentrées à sec dans les conditions ci-dessus. On obtient 1,1 g de 3-(3-chloro-6-méthoxy-quinolin-4-yl)-9-[2-(3,5-difluoro-phénoxy)-éthyl]-2-oxa-9-aza-spiro[5.5]undecan-1-one.
Spectre infra rouge (CCl₄) : 2940 ; 1735 ; 1622 ; 1600 ; 1503 ; 1467 ; 1233 ; 1153 ; 1117 ; 841 et 671 cm⁻¹.

Le 4-[3-(3-chloro-6-méthoxy-quinolin-4-yl)-3-hydroxy-propyl]-pipéridine-4-carboxylate de méthyle a été préparé dans l'exemple 49.

Le 1-(2-bromoéthoxy)-3,5-difluorobenzène peut être obtenu par application de la méthode décrite dans l'exemple 16.

### Exemple 27

### 4-[3-(3-Chloro-6-méthoxy-quinolin-4-yl)-propyl]-1-[2-(3,5-difluoro-phénoxy)-éthyl]-pipéridine-4-carboxamide

A un mélange de 0,69 g d'acide 4-[3-(3-chloro-6-méthoxy-quinolin-4-yl)-propyl]-1-[2-(3,5-difluoro-phénoxy)-éthyl]-pipéridine-4-carboxylique dans 30 cm³ de dichlorométhane, on ajoute, sous agitation et atmosphère inerte, 13,4 cm³ d'une solution d'ammoniaque à 0,5 N dans le dioxanne, 0,229 g de (1-hydroxybenzotriazole hydrate), 0,64 g de (1-[3-(diméthylamino)propyl]-3-éthylcarbodiimide hydrochloride) et 0,46 cm³ de triéthylamine .La suspension obtenue est agitée pendant 17 heures à une température voisine de 20°C. Le mélange réactionnel est dilué par 50 cm³ d'eau, agité puis décanté. La phase aqueuse est extraite par 2 fois 25 cm³ de dichlorométhane et les extraits organiques sont réunis, lavés par 25 cm³ d'une solution aqueuse de chlorure de sodium saturée, séchés sur sulfate de magnésium puis concentrés à sec sous pression réduite (2 kPa) à une température voisine de 40°C. Le solide est repris par 20 cm³ d'éther diisopropylique, agité puis filtré. On obtient 0,52 g de 4-[3-(3-chloro-6-méthoxy-quinolin-4-yl)-propyl]-1-[2-(3,5-difluoro-phénoxy)-éthyl]-pipéridine-4-carboxamide.
Spectre de R.M.N. ¹H (300 MHz, (CD₃)₂SO d6, δ en ppm) : 1,34 (t large, J = 12,5 Hz : 2H) ; 1,54 (mt : 2H) ; 1,66 (mt : 2H) ; 2,02 (d large, J = 12,5 Hz : 2H) ; 2,12 (t large, J = 11 Hz : 2H) ; de 2,55 à 2,75 (mt : 4H) ; 3,16 (t large, J = 7,5 Hz : 2H) ; 3,97 (s : 3H) ; 4,08 (t, J = 6 Hz : 2H) ; de 6,65 à 6,85 (mt : 3H) ; 6,91 (s large : 1H) ; 7,15 (s large : 1H) ; 7,37 (d, J = 3 Hz : 1H) ; 7,45 (dd, J = 9 et 3 Hz : 1H) ; 7,97 (d, J = 9 Hz : 1H) ; 8,68 (s : 1H).

L'acide 4-[3-(3-chloro-6-méthoxy-quinolin-4-yl)-propyl]-1-[2-(3,5-difluoro-phénoxy)-éthyl]-pipéridine-4-carboxylique a été préparé dans l'exemple 2.

### Exemple 28

### Sel de sodium de l'acide 4-[3-(3-chloro-6-méthoxy-quinolin-4-yl)-propyl]-1-(cinnamyl)-pipéridine-4-carboxylique

Un mélange de 0,72 g de 4-[3-(3-chloro-6-méthoxy-quinolin-4-yl)-propyl]-1-(cinnamyl)-pipéridine-4-carboxylate d'éthyle dans 14 cm³ de dioxanne, 14 cm³ de méthanol et 14 cm³ d'une solution aqueuse de soude 5N est agité à une température voisine de 20°C. Au bout de 2 heures, on ajoute à nouveau 14 cm³ d'une solution aqueuse de soude 5N, puis une nouvelle fois 14 cm³ au bout de 18 heures supplémentaires. Le mélange réactionnel est porté à une température voisine de 70°C pendant 21 heures. Après refroidissement aux environs de 20°C, la masse réactionnelle est concentrée à sec sous pression réduite (2 kPa) à une température voisine de 40°C. Le résidu obtenu est repris par 15 cm³ d'eau distillée, filtré puis séché à l'air libre pendant 18 heures. On obtient 0,49 g de sel de sodium de l'acide 4-[3-(3-chloro-6-méthoxy-quinolin-4-yl)-propyl]-1-(cinnamyl)-pipéridine-4-carboxylique, sous forme d'un solide blanc fondant aux environs de 230°C.
Spectre de R.M.N. ¹H (300 MHz, (CD₃)₂SO d6, δ en ppm) : 1,16 (t très large, J = 12 Hz : 2H) ; de 1,45 à 1,70 (mt : 4H) ; de 1,95 à 2,20 (mt : 4H) ; de 2,45 à 2,60 (mt : 2H) ; 2,99 (d, J = 6,5 Hz : 2H) ; 3,14 (t large, J = 7 Hz : 2H) ; 3,99 (s : 3H) ; 6,28 (dt, J = 15,5 et 6,5 Hz : 1H) ; 6,48 (d, J = 15,5 Hz : 1H) ; 7,24 (t large, J = 7,5 Hz : 1H) ; 7,33 (t large, J = 7,5 Hz : 2H) ; de 7,40 à 7,50 (mt : 4H) ; 7,95 (d, J = 9 Hz : 1H) ; 8,66 (s : 1H).

### 4-[3-(3-Chtoro-6-méthoxy-quinolin-4-yl)-propyl]-1-(cinnamyl)-pipéridine-4-carboxylate d'éthyle

Un mélange de 1 g de 4-[3-(3-chloro-6-méthoxy-quinolin-4-yl)-propyl]-pipéridine-4-carboxylate d'éthyle, 0,551 g de bromure de cinnamyle, 0,42 g d'iodure de potassium et 1,05 g de carbonate de potassium dans 20 cm³ d'acétonitrile est agité pendant 18,5 heures à une température voisine de 20°C. Le mélange réactionnel est filtré et l'insoluble est rincé par 20 cm³ d'acétonitrile et par 2 fois 30 cm³ de dichlorométhane. Le filtrat est séché sur du sulfate de magnésium, filtré puis concentré à sec sous pression réduite (2 kPa) à une température voisine de 40°C. Le résidu obtenu est purifié par chromatographie sous pression d'argon de 50 kPa, sur une colonne de gel de silice (granulométrie 40-63 µ ; diamètre 2,5 cm ; masse 35 g), en éluant par un mélange de cyclohexane-acétate d'éthyle (90/10 en volumes) puis par un mélange cyclohexane-acétate d'éthyle (60/40 en volumes) et en recueillant des fractions de 20cm³. Les fractions contenant le produit sont réunies puis concentrées à sec dans les conditions décrites précédemment. On obtient 0,72 g de 4-[3-(3-chloro-6-méthoxy-quinohn-4-yl)-propyl]-l-(3-phényl-aMyl)-pipéridine-4-carboxytate d'éthyle, sous forme d'une huile visqueuse orange.
- Spectre de masse :: EI m/z=506 M⁺
m/z=415 C₂₃H₂₈ClN₂O₃⁺
m/z=300 C₁₉H₂₆NO₂⁺
m/z=117 C₉H₉⁺ pic de base

Le 4-[3-(3-chloro-6-méthoxy-quinolin-4-yl)-propyl]-pipéridine-4-carboxylate d'éthyle a été préparé sous forme de monochlorydrate dans l'exemple 5.

### Exemple 29

### Acide-4-[3-(3-chloro-6-méthoxy-quinolin-4-yl)-propyl]-1-[2-(3,5-difluoro-phénoxy)-éthyl]-pipéridine-4-acétique

Un mélange de 0,18 g de l'ester méthylique de l'acide 4-[3-(3-chloro-6-méthoxy-quinolin-4-yl)-propyl]-1-[2-(3,5-difluoro-phénoxy)-éthyl]-pipéridine-4-yl}-carboxylate dans 10 cm³ de dioxanne, 10 cm³ de méthanol et 0,99 cm³ d'une solution aqueuse de soude 5N est agité à une température voisine de 75°C pendant 17 heures. Après refroidissement aux environs de 45°C, le mélange réactionnel est concentré à sec sous pression réduite progressive jusqu'à 9 kPa à une température voisine de 40°C. Le résidu pâteux est repris par 3 cm³ de chloroforme-méthanol-ammoniaque (12/3/0,5 en volumes) puis la phase organique est purifiée par chromatographie sous pression atmosphérique, sur une colonne de gel de silice (bond élut ; granulométrie 70-200 µ ; diamètre 2 cm ; masse 5 g), en éluant par un mélange de chloroforme-méthanol-ammoniaque (12/3/0,5 en volumes). Les fractions contenant le produit sont réunies puis concentrées à sec sous pression réduite (5 kPa) à une température voisine de 40°C et le produit est séché à l'étuve sous pression réduite (50 Pa) à une température voisine de 50°C. On obtient 0,15 g d'acide 4-[3-(3-chloro-6-méthoxy-quinolin-4-yl)-propyl]-1-[2-(3,5-difluoro-phénoxy)-éthyl]-pipéridine-4-acétique.
Spectre de R.M.N. ¹H (500 MHz, (CD₃)₂SO d6, δ en ppm) : 1,39 (mt : 2H) ; 1,50 (mt : 2H) ; 1,61 (mt : 4H) ; 2,15 (s large : 2H) ; de 2,30 à 2,60 (mt : 4H) ; 2,63 (t large, J = 5,5 Hz : 2H) ; 3,12 (mf : 2H) ; 3,94 (s : 3H) ; 4,06 (t large, J = 5,5 Hz : 2H) ; de 6,65 à 6,75 (mt : 3H) ; 7,36 (s large: 1H) ; 7,41 (d large, J = 9 Hz : 1H) ; 7,93 (d, J = 9 Hz : 1H);8,64(s: 1H).

### {4-[3-(3-Chloro-6-méthoxy-quinolin-4-yl)-propyl]-1-[2-(3,5-difluoro-phénoxy)-éthyl]-pipéridine-4-yl}-acétate de méthyle

Un mélange de 0,35 g de {4-[3-(3-chloro-6-méthoxy-quinolin-4-yl)-propyl]-pipéridine-4-yl}-acétate de méthyle, 0,318 g de 1-(2-bromo-éthoxy)-3,5-difluorobenzène, 0,15 g d'iodure de potassium et 0,62 g de carbonate de potassium dans 20 cm³ d'acétonitrile est agité pendant 18 heures à une température voisine de 70°C. Après refroidissement aux environs de 20°C, le mélange réactionnel est filtré sur une cartouche de silice (bond élut) puis rincé par 2 fois 2 cm³ d'acétonitrile. La phase organique est concentrée à sec sous pression réduite (2,4 kPa) à une température voisine de 45°C. Le résidu d'évaporation obtenu est dissous dans 12 cm³ de diméthylformamide puis purifié par injection de 4 fois 3 cm³ par chromatographie, sur une cartouche SCX de gel de silice (masse 1 g), en éluant par un gradient de méthanol pur à un mélange de méthanol ammoniacal 4N. Les fractions contenant le produit sont réunies puis concentrées à sec sous air comprimé à une température voisine de 45°C. Le résidu d'évaporation obtenu est repris par du dichlorométhane et séché à l'air libre pendant 48 heures puis séché à l'étuve sous pression réduite (10 kPa) à une température voisine de 50°C. On obtient 0,195 g de {4-[3-(3-chloro-6-méthoxy-quinolin-4-yl)-propyl]-1-[2-(3,5-difluoro-phénoxy)-éthyl]-pipéridine-4-yl}-acétate de méthyle.
Spectre de R.M.N. ¹H (500 MHz, (CD₃)₂SO d6, à une température de 353 K, δ en ppm) : de 1,45 à 2,70 (mt : 16H) ; 3,17 (mt : 2H) ; 3,51 (s : 3H) ; 3,96 (s : 3H) ; 4,24 (mf: 2H) ; de 6,60 à 6,70 (mt : 3H) ; 7,38 (s large : 1H) ; 7,42 (d large, J = 9 Hz : 1H) ; 7,94 (d, J = 9 Hz : 1H) ; 8,62 (s large : 1H).

### {4-[3-(3-chloro-6-méthoxy-quinolin-4-yl)-propyl]-pipéridine-4-yl}-acétate de méthyle

Un mélange de 17 g de 4-[3-(3-chloro-6-méthoxy-quinolin-4-yl)-propyl]-4-cyanométhyl-pipéridine-1-carboxylate de *tert*-butyle dans 100 cm³ d'acide chlorhydrique aqueux 12N (concentré à sec) est porté progressivement à une température voisine de 100°C sous agitation pendant 18 heures. Le mélange réactionnel est concentré à sec sous pression réduite (2 kPa) à une température voisine de 60°C. L'huile obtenue est reprise par de l'acétone et le précipité est filtré, lavé avec de l'acétone. On obtient 20,4 g de dichlorhydrate d'acide {4-[3-(3-chloro-6-méthoxy-quinolin-4-yl)-propyl]-pipéridine-4-yl}-acétique, sous forme d'un solide grisâtre fondant à 181°C en collant. Le produit précédent est dissous dans 400 cm³ de méthanol et 10 cm³ d'acide sulfurique concentré à sec, agité et chauffé au voisinage de 100°C pendant 1,5 heures. Le mélange réactionnel est concentré à sec sous pression réduite (2 kPa) à une température voisine de 50°C. L'huile obtenue est reprise par 300 cm³ par de l'eau sous agitation, neutralisée par de l'hydrogénocarbonate de sodium, alcalinisée par du carbonate de potassium et extraite par de l'acétate d'éthyle. La phase organique est décantée, lavée par de l'eau, séchée sur du sulfate de magnésium, filtrée puis concentrée à sec sous pression réduite (2 kPa) à une température voisine de 40°C. La phase aqueuse est alcalinisée à pH 8 par une solution aqueuse de soude 5N et extraite par de l'acétate d'éthyle, décantée, lavée, séchée avec du sulfate de magnésium, et les extraits organiques sont concentrés à sec selon les conditions décrites précédemment. Tous les résidus d'évaporation organiques sont réunis et purifiés par chromatographie sous pression d'argon de 50 kPa sur une colonne de gel de silice (granulométrie 20-43 µ ; masse 200 g), en éluant par un mélange de dichlorométhane-méthanol-ammoniaque (40/5/0,5 en volumes). Les fractions contenant le produit sont réunies et concentrées à sec comme dans les conditions ci-dessus. On obtient 7,6 g de {4-[3-(3-chloro-6-méthoxy-quinolin-4-yl)-propyl]-pipéridine-4-yl}-acétlate de méthyle, sous forme d'une huile de couleur orangée.
- Spectre de masse :: EI m/z=390 M⁺ m/z=207 C₁₁H₁₀ClNO⁺ m/z=184 C₁₀H₁₈NO₂⁺

### 4-[3-(3-Chloro-6-méthoxy-quinolin-4-yl)-propyl]-4-cyanométhyl-pipéridine-1-carboxylate de tert-butyle

Un mélange de 30,58 g de 4-[3-(3-chloro-6-méthoxy-quinolin-4-yl)-propyl]-4-méthanesulfonyloxyméthyl-pipéridine-1-carboxylate de *tert*-butyle et 15,1 g de cyanure de potassium dans 436 cm³ de diméthylsulfoxyde est chauffé sous agitation à une température voisine de 100°C pendant 48 heures puis est agité au voisinage de 20°C pendant 24 heures. On verse 2000 cm³ d'eau glacée sur le mélange réactionnel, la suspension est agitée pendant 1 heure à une température voisine de 20°C puis filtrée et le gâteau est lavé par 3 fois 200 cm³ d'eau puis séché à l'air libre. Le résidu est purifié par chromatographie sous pression d'argon de 50 kPa sur une colonne de gel de silice (granulométrie 20-45 µ ; diamètre 9 cm ; hauteur 45 cm), en éluant par un mélange de cyclohexane-acétate d'éthyle (90/10 en volumes). Les fractions de 40 à 100 sont réunies et concentrées à sec sous pression réduite (2 kPa) à une température voisine de 45°C. On obtient 19,25 g de 4-[3-(3-chloro-6-méthoxy-quinolin-4-yl)-propyl]-4-cyanométhyl-pipéridine-1-carboxylate de *tert-*butyle sous forme d'un solide jaune fondant à 160°C.
Spectre infra rouge (KBr) : 2968; 2924; 2235; 1684; 1622; 1505; 1414; 1230; 1166; 1151; 826 et 738 cm⁻¹.

### 4-[3-(3-Chloro-6-méthoxy-quinolin-4-yl)-propyl]-4-méthanesulfonyloxyméthyl-pipéridine-1-carboxylate de tert-butyle

Un mélange de 24,8 g de 4-[3-(3-chloro-6-méthoxy-quinolin-4-yl)-propyl]-4-hydroxyméthyl-pipéridine-1-carboxylate de *tert*-butyle dans 400 cm³ de dichlorométhane et de 12,9 cm³ de triéthylamine est refroidi au voisinage de 0°C, sous agitation et atmosphère inerte. On verse goutte à goutte le chlorure de méthanesulfonyle préalablement dissous dans 125 cm³ de dichlorométhane puis le mélange réactionnel est agité pendant 18 heures à une température voisine de 20°C. On verse 200 cm³ d'eau sur la masse réactionnelle puis on ajoute une nouvelle fois 3000 cm³ d'eau. Les phases sont décantées et la phase aqueuse est extraite par 200 cm³ de dichlorométhane. Les extraits organiques sont lavés par 300 cm³ d'une solution aqueuse de chlorure de sodium, séchés sur du sulfate de sodium, filtrés puis concentrés à sec sous pression réduite (2 kPa) à une température voisine de 45°C. On obtient 30,6 g de 4-[3-(3-chloro-6-méthoxy-quinolin-4-yl)-propyl]-4-méthane-sulfonyloxyméthyl-pipéridine-1-carboxylate de *tert*-butyle, sous forme d'une huile de couleur orangée.
- Spectre de masse : DCI: m/z=507 MH⁺
m/z=431 M-CH₃SO₃
m/z=397 431-Cl
m/z=375 431-tBu
m/z=331 431-BOC

Le 1-(2-bromoéthoxy)-3,5-difluorobenzène peut être obtenu par application de la méthode décrite dans l'exemple a été préparé dans l'exemple 16.

Le 4-[3-(3-chloro-6-méthoxy-quinolin-4-yl)-propyl]-4-hydroxyméthyl-pipéridine-1-carboxylate de *tert-*butyle a été préparé dans l'exemple 19.

### Exemple 30

### Acide {4-[3-(3-chloro-6-méthoxy-quinolin-4-yl)-propyl]-1-[2-(2,5-difluoro-phénoxy)-éthyl]-pipéridine-4-yl}-acétique

Un mélange de 0,35 g de {4-[3-(3-chloro-6-méthoxy-quinolin-4-yl)-propyl]-1-[2-(2,5-difluoro-phénoxy)-éthyl]-pipéridine-4-yl}-acétate de méthyle dans 10 cm³ de dioxanne, 10 cm³ de méthanol et 1,91 cm³ d'une solution aqueuse de soude 5N est agité à une température voisine de 75°C pendant 17 heures. Après refroidissement aux environs de 45°C, le mélange réactionnel est concentré à sec sous pression réduite progressive jusqu'à 9 kPa à une température voisine de 40°C. Le résidu pâteux est repris par 3 cm³ de chloroforme-méthanol-ammoniaque (12/3/0,5 en volumes) puis la phase organique est purifiée par chromatographie sous pression atmosphérique, sur une colonne de gel de silice (granulométrie 70-200 µ ; diamètre 2 cm ; masse 5 g), en éluant par un mélange de chloroforme-méthanol-ammoniaque (12/3/0,5 en volumes). Les fractions contenant le produit sont réunies puis concentrées à sec sous pression réduite (5 kPa) à une température voisine de 40°C et le produit est séché à l'étuve sous pression réduite (50 Pa) à une température voisine de 50°C. On obtient 0,27 g d'acide {4-[3-(3-chloro-6-méthoxy-quinolin-4-yl)-propyl]-1-[2-(2,5-difluoro-phénoxy)-éthyl]-pipéridine-4-yl} -acétique.
Spectre de R.M.N. ¹H (500 MHz, (CD₃)₂SO d6, δ en ppm) : 1,39 (mt : 2H) ; 1,50 (mt : 2H) ; 1,60 (mt : 4H) ; 2,15 (s large : 2H) ; de 2,30 à 2,60 (mt : 4H) ; 2,67 (t, J = 6 Hz : 2H) ; 3,12 (mf : 2H) ; 3,93 (s : 3H) ; 4,12 (t, J = 6 Hz : 2H) ; 6,72 (mt : 1H) ; 7,11 (mt : 1H) ; 7,21 (mt : 1H) ; 7,37 (s large : 1H) ; 7,41 (d large, J = 9 Hz : 1H) ; 7,93 (d, J = 9 Hz : 1H) ; 8,64 (s : 1H).

### {4-[3-(3-Chloro-6-méthoxy-quinolin-4-yl)-propyl]-1-[2-(2,5-ditluoro-phénoxy)-éthyl]-pipéridine-4-yl}-acétate de méthyle

Un mélange de 0,35 g de {4-[3-(3-chloro-6-méthoxy-quinolin-4-yl)-propyl]-pipéridine-4-yl}-acétate de méthyle, 0,32 g de 2-(2-bromo-éthoxy)-1,4-difluorobenzène, 0,15 g d'iodure de potassium et 0,62 g de carbonate de potassium dans 20 cm³ d'acétonitrile est agité pendant 18 heures à une température voisine de 70°C. Après refroidissement aux environs de 20°C, le mélange réactionnel est filtré sur une cartouche de silice (bond élut) puis rincé par 2 fois 2 cm³ d'acétonitrile. La phase organique est concentrée à sec sous pression réduite (2,4 kPa) à une température voisine de 45°C. Le résidu d'évaporation obtenu est dissous dans 12 cm³ de diméthylformamide puis purifié par injection de 4 fois 3 cm³ par chromatographie sur une cartouche SCX de gel de silice (masse 1 g), en éluant par un gradient de méthanol pur à un mélange de méthanol ammoniacal 4N. Les fractions contenant le produit sont réunies puis concentrées à sec sous air comprimé à une température voisine de 45°C.

Le résidu d'évaporation obtenu est repris par du dichlorométhane et séché à l'air libre pendant 48 heures puis séché à l'étuve sous pression réduite (10 Pa) à une température voisine de 50°C. On obtient 0,38 g de {4-[3-(3-chloro-6-méthoxy-quinolin-4-yl)-propyl]-1-[2-(2,5-difluoro-phénoxy)-éthyl]-pipéridine-4-yl}-acétate de méthyle.
Spectre de R.M.N. ¹H (500 MHz, (CD₃)₂SO d6, δ en ppm) : de 1,30 à 1,65 (mt : 8H) ; 2,25 (s large : 2H) ; de 2,30 à 2,60 (mt : 4H) ; 2,69 (mt : 2H) ; 3,14 (mt : 2H) ; 3,47 (s large : 3H) ; 3,95 (s large : 3H) ; 4,12 (mt : 2H) ; 6,72 (mt : 1H) ; 7,11 (mt : 1H) ; 7,21 (mt : 1H) ; 7,38 (s large : 1H) ; 7,43 (d large, J = 9 Hz : 1H) ; 7,94 (d large, J = 9 Hz : 1H) ; 8,66 (s large: 1H).

Le 2-(2-bromo-éthoxy)-1,4-difluoro-benzène a été préparé dans l'exemple 16.

Le {4-[3-(3-chloro-6-méthoxy-quinolin-4-yl)-propyl]-pipéridine-4-yl}-acétate de méthyle a été préparé dans l'exemple 29.

### Exemple 31

### Acide 1-(2-cyclopentylthio-éthyl)-4-[3-(3-fluoro-6-méthoxy-quinolin-4-yl)-propyl]-pipéridine-4-carboxylique

Un mélange de 0,4 g de 1-(2-cyclopentylthio-éthyl)-4-[3-(3-fluoro-6-méthoxy-quinolin-4-yl)-propyl]-pipéridine-4-carboxylate d'éthyle dans 3 cm³ de dioxanne, 3 cm³ de méthanol et 2,4 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ : masse 10 g), en éluant par un mélange de dichlorométhane-méthanol-ammoniaque (40/5/0,5 en volumes) puis par un mélange chloroforme-méthanol-ammoniaque (13/3/0,5). Les fractions contenant un précipité sont réunies, filtrées puis lavées par du méthanol. Le solide obtenu est recristallisé par 15 cm³ de méthanol bouillant, refroidi, filtré, lavé par du méthanol, séché à l'étuve sous pression réduite (10 Pa) au voisinage de 20°C. Les jus de filtration sont réunis et concentrés à sec sous pression réduite (2 kPa) à une température voisine de 40°C. Les jus une fois concentrés à sec et le solide obtenu précédemment sont réunis et l'on obtient 0,179 g d'acide 1-(2-cyclopentylthio-éthyl)-4-[3-(3-fluoro-6-méthoxy-quinolin-4-yl)-propyl]-pipéridine-4-carboxylique sous la forme d'un solide blanc.
Spectre de R.M.N. ¹H (400 MHz, (CD₃)₂SO d6 avec ajout de quelques gouttes de CF₃COOD, δ en ppm): 1,41 (mt : 2H) ; de 1,50 à 1,75 (mt : 8H) ; de 1,90 à 2,05 (mt: 4H) ; 2,20 (d large, J = 14 Hz : 2H) ; de 2,75 à 2,90 (mt : 4H) ; de 3,10 à 3,25 (mt : 1H) ; 3,16 (t, J = 7 Hz : 2H) ; 3,29 (mt : 2H) ; 3,50 (d large, J = 14 Hz : 2H) ; 3,99 (s : 3H) ; 7,47 (d, J = 2,5 Hz : 1H) ; 7,55 (dd, J = 9 et 2,5 Hz : 1H) ; 8,06 (d, J = 9 Hz : 1H) ; 8,98 (s large : 1H).

### 4-[3-(3-Fluoro-6-méthoxy-quinolin-4-yl)-propyl]-pipéridine-4-carboxylate d'éthyle

Un mélange de 0,5 g de 4-[3-(3-fluoro-6-méthoxy-quinolin-4-yl)-propyl]-pipéridine-4-carboxylate d'éthyle, 0,253 g de (2-chloro-éthylthio)-cyclopentane, 0,25 g d'iodure de potassium et 0,92 g de carbonate de potassium dans 15 cm³ d'acétonitrile est agité pendant 18 heures à une température voisine de 70°C. Après refroidissement aux environs de 20°C, le mélange réactionnel est filtré puis rincé par 2 fois 3 cm³ d'acétonitrile. La phase organique est concentrée à sec sous pression réduite (2,4 kPa) à une température voisine de 45°C. Le résidu d'évaporation obtenu est dissous dans un mélange d'acétate d'éthyle-éther de pétrole 40-60°C (8/2 en volumes) puis est purifié par chromatographie sous pression atmosphérique, sur une cartouche de gel de silice (bond élut ; granulométrie 70-200 µ ; masse 10 g), en éluant par un mélange d'acétate d'éthyle-éther de pétrole 40-60°C (8/2 en volumes). Les fractions de 5 à 14 sont réunies puis concentrées à sec sous pression réduite (2 kPa) à une température voisine de 40°C. On obtient 0,43 g de 4-[3-(3-fluoro-6-méthoxy-quinolin-4-yl)-propyl]-pipéridine-4-carboxylate d'éthyle.
Spectre de R.M.N. ¹H (500 MHz, (CD₃)₂SO d6, δ en ppm) : 0,98 (t, J = 7 Hz : 3H) ; 1,36 (mt: 4H) ; de 1,40 à 1,70 (mt : 8H) ; de 1,80 à 2,00 (mt : 6H) ; de 2,30 à 2,65 (mt : 6H); 3,02 (mt : 2H) ; 3,09 (mt: 1H) ; 3,92 (s : 3H) ; 3,94 (q, J = 7 Hz : 2H) ; 7,31 (s large : 1H) ; 7,37 (d large, J = 9 Hz : 1H) ; 7,94 (d, J = 9 Hz : 1H) ; 8,66 (s large : 1H).

Le 4-[3-(3-fluoro-6-méthoxyquinolin-4-yl)-propyl]-pipéridine-4-carboxylate d'éthyle a été préparé dans l'exemple 11.

### Exemple 32

### Acide 1-(2-cyctohexyl-éthyl)-4-[3-(3-fluoro-6-méthoxy-quinolin-4-yl)-propyl]-pipéridine-4-carboxylique

Un mélange de 0,2 g de 1-(2-cyclohexyl-éthyl)-4-[3-(3-fluoro-6-méthoxy-quinolin-4-yl)-propyl]-pipéridine-4-carboxylate d'éthyle dans 3 cm³ de dioxanne, 3 cm³ de méthanol et 1,2 cm³ d'une solution aqueuse de soude 5N est agité à une température voisine de 70°C pendant 22 heures. Après refroidissement aux environs de 20°C, le mélange réactionnel est évaporé sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10g), en éluant par un mélange de dichlorométhane-méthanol-ammoniaque (40/5/0,5 en volumes) puis par un mélange chloroforme-méthanol-ammoniaque (13/3/0,5). Les fractions contenant le produit sont réunies, concentrées à sec sous pression réduite (2 kPa) à une température voisine de 40°C et séchées au dessicateur (10 kPa). On obtient 0,14 g d'acide 1-(2-cyclohexyléthyl)-4-[3-(3-fluoro-6-méthoxy-quinolin-4-yl)-propyl]-pipéridine-4-carboxylique sous la forme d'un solide blanc.
Spectre de R.M.N. ¹H (400 MHz, (CD₃)₂SO d6 avec ajout de quelques gouttes de CF₃COOD, δ en ppm) : de 0,80 à 1,00 (mt : 2H) ; de 1,05 à 1,35 (mt : 5H) ; de 1,40 à 1,75 (mt : 12H) ; 2,19 (d large, J = 14 Hz : 2H) ; 2,78 (t large, J = 12,5 Hz : 2H) ; 3,07 (mt : 2H) ; 3,20 (t large, J = 6,5 Hz : 2H) ; 3,45 (d large, J = 12,5 Hz : 2H) ; 4,00 (s : 3H) ; 7,52 (d, J = 2,5 Hz : 1H) ; 7,60 (dd, J = 9 et 2,5 Hz : 1H) ; 8,10 (d, J = 9 Hz : 1H) ; 9,10 (d, J = 2 Hz : 1H).

### 1-(2-Cyclohexyl-éthyl)-4-[3-(3-fluoro-6-méthoxy-quinolin-4-yl)-propyl]-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,245 g de 1-bromo-2-cyclohexyléthane, 0,18 g d'iodure de potassium et 0,737 g de carbonate de potassium dans 15 cm³ d'acétonitrile est agité pendant 18 heures à une température voisine de 70°C. Après refroidissement aux environs de 20°C, le mélange réactionnel concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,23 g de 1-(2-cyclohexyl-éthyl)-4-[3-(3-fluoro-6-méthoxy-quinolin-4-yl)-propyl]-pipéridine-4-carboxylate d'éthyle.
Spectre de R.M.N. ¹H (500 MHz, (CD₃)₂SO d6, δ en ppm) : 0,84 (mt : 2H) ; 0,98 (t, J = 7 Hz : 3H) ; de 1,00 à 2,65 (mt : 25H) ; 3,02 (mt : 2H) ; 3,93 (s : 3H) ; 3,95 (q, J = 7 Hz : 2H) ; 7,31 (s large : 1H) ; 7,35 (d large, J = 9 Hz : 1H) ; 7,94 (d, J = 9 Hz : 1H) ; 8,67 (s large : 1H).

Le 4-[3-(3-fluoro-6-méthoxyquinolin-4-yl)-propyl]-pipéridine-4-carboxylate d'éthyle a été préparé dans l'exemple 11.

### Exemple 33

### Acide-1-(2-cyclohexylthio-éthyl)-4-[3-(3-fluoro-6-méthoxy-quinolin-4-yl)-propyl]-pipéridine-4-carboxylique

Un mélange de 0,3 g de 1-(2-cyclohexylthio-éthyl)-4-[3-(3-fluoro-6-méthoxy-quinolin-4-yl)-propyl]-pipéridine-4-carboxylate d'éthyle dans 3 cm³ de dioxanne, 3 cm³ de méthanol et 1,7 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10g), en éluant par un mélange de dichlorométhane-méthanol-ammoniaque (40/5/0,5 en volumes) puis par un mélange chloroforme-méthanol-ammoniaque (13/3/0,5). Les fractions contenant le produit sont réunies, concentrées à sec sous pression réduite (2 kPa) à une température voisine de 40°C et séchées au dessicateur (10 kPa). On obtient 0,25 g d'acide 1-(2-cyclohexylthio-éthyl)-4-[3-(3-fluoro-6-méthoxy-quinolin-4-yl)-propyl]-pipéridine-4-carboxylique sous la forme d'un solide blanc.
Spectre de R.M.N. ¹H (400 MHz, (CD₃)₂SO d6 avec ajout de quelques gouttes de CF₃COOD, δ en ppm) : de 1,15 à 1,35 (mt : 5H) ; de 1,50 à 1,75 (mt : 8H) ; de 1,80 à 2,05 (mt : 3H) ; 2,20 (d large, J = 14 Hz : 2H) ; de 2,65 à 2,90 (mt : 5H) ; 3,20 (t large, J = 7 Hz : 2H) ; 3,25 (mt : 2H) ; 3,50 (d large, J = 12 Hz : 2H) ; 4,00 (s : 3H) ; 7,50 (d, J = 2,5 Hz : 1H) ; 7,59 (dd, J = 9 et 2,5 Hz : 1H) ; 8,09 (d, J = 9 Hz : 1H) ; 9,07 (d, J = 2,5 Hz : 1H).

### 1-(2-Cyclohexylthio-éthyl)-4-[3-(3-fluoro-6-méthoxy-quinolin-4-yl)-propyl]-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,23 g de 2-chloroéthylthiocyclohexane, 0,18 g d'iodure de potassium et 0,74 g de carbonate de potassium dans 15 cm³ d'acétonitrile est agité pendant 18 heures à une température voisine de 70°C. Après refroidissement aux environs de 20°C, le mélange réactionnel concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,32 g de 1-(2-cyclohexyl-éthyl)-4-[3-(3-fluoro-6-méthoxy-quinolin-4-yl)-propyl]-pipéridine-4-carboxylate d'éthyle.
Spectre de R.M.N. ¹H (500 MHz, (CD₃)₂SO d6, δ en ppm) : 0,97 (t, J = 7 Hz : 3H) ; de 1,05 à 1,70 (mt : 14H) ; de 1,75 à 2,00 (mt : 6H) ; de 2,25 à 2,60 (mt : 6H) ; 2,62 (mt : 1H) ; 3,01 (mt : 2H) ; 3,91 (s : 3H) ; 3,94 (q, J = 7 Hz : 2H) ; 7,30 (s large : 1H) 7,37 (d large, J = 9 Hz : 1H) ; 7,93 (d, J = 9 Hz : 1H) ; 8,66 (s large : 1H).

Le 4-[3-(3-fluoro-6-méthoxyquinolin-4-yl)-propyl]-pipéridine-4-carboxylate d'éthyle a été préparé dans l'exemple 11.

### Exemple 34

### Acide 4-[3-(3-fluoro-6-méthoxy-quinolin-4-yl)-propyl]-1-(3-phényl-propyl)-pipéridine-4-carboxylique

Un mélange de 0,1 g de 4-[3-(3-fluoro-6-méthoxy-quinolin-4-yl)-propyl]-1-(3-phényl-propyl)-pipéridine-4-carboxylate d'éthyle dans 3 cm³ de dioxanne, 3 cm³ de méthanol et 0,6 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10g), en éluant par un mélange de dichlorométhane-méthanol-ammoniaque (40/5/0,5 en volumes) puis par un mélange chloroforme-méthanol-ammoniaque (13/3/0,5). Les fractions contenant le produit sont réunies, concentrées à sec sous pression réduite (2 kPa) à une température voisine de 40°C et séchées au dessicateur (10 Pa). On obtient 0,25 g d'acide 4-[3-(3-fluoro-6-méthoxy-quinolin-4-yl)-propyl]-1-(3-phényl-propyl)-pipéridine-4-carboxylique sous la forme d'un solide blanc.
Spectre de R.M.N. ¹H (400 MHz, (CD₃)₂SO d6 avec ajout de quelques gouttes de CF₃COOD, δ en ppm) : de 1,50 à 1,75 (mt : 6H) ; de 1,85 à 2,05 (mt : 2H) ; 2,19 (d large, J = 14 Hz : 2H) ; 2,60 (t, J = 7,5 Hz : 2H) ; 2,81 (t large, J = 12,5 Hz : 2H) ; 3,07 (mt : 2H) ; 3,20 (t large, J = 7,5 Hz : 2H) ; 3,47 (d large, J = 12,5 Hz : 2H) ; 3,99 (s : 3H); de 7,15 à 7,35 (mt : 5H) ; 7,51 (d, J =2,5 Hz : 1H) ; 7,59 (dd, J = 9 et 2,5 Hz : 1H) ; 8,10 (d, J = 9 Hz : 1H) ; 9,08 (d, J = 2 Hz : 1H).

### 4-[3-(3-Fluoro-6-méthoxy-quinolin-4-yl)-propyl]-1-(3-phényl-propyl)-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,25 g de 1-bromo-3-phénylpropane, 0,181 g d'iodure de potassium et 0,74 g de carbonate de potassium dans 15 cm³ d'acétonitrile est agité pendant 18 heures à une température voisine de 70°C. Après refroidissement aux environs de 20°C, le mélange réactionnel concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,1 g de 4-[3-(3-fluoro-6-méthoxy-quinolin-4-yl)-propyl]-1-(3-phényl-propyl)-pipéridine-4-carboxylate d'éthyle.
Spectre de R.M.N. ¹H (500 MHz, (CD₃)₂SO d6, δ en ppm) : 0,97 (t, J = 7 Hz : 3H) ; de 1,25 à 2,00 (mt : 12H) ; de 2,25 à 2,70 (mt : 6H) ; 3,01 (mt : 2H) ; 3,91 (s : 3H) ; 3,94 (q large, J = 7 Hz : 2H) ; 7,15 (mt : 3H) ; 7,24 (t large, J = 7,5 Hz : 2H) ; 7,30 (s large : 1H) ; 7,36 (d large, J = 9 Hz : 1H) ; 7,93 (d, J = 9 Hz : 1H) ; 8,66 (s large : 1H).

Le 4-[3-(3-fluoro-6-méthoxyquinolin-4-yl)-propyl]-pipéridinc-4-carboxylatc d'éthyle a été préparé dans l'exemple 11.

### Exemple 35

### Acide 1-[2-(2,5-ditluoro-phénylthio)-éthyl]-4-[3-(3-fluoro-6-méthoxy-quinolin-4-yl)-propyl]-pipéridine-4-carboxylique

Un mélange de 0,26 g de 1-[2-(2,5-difluoro-phénylthio)-éthyl]-4-[3-(3-fluoro-6-méthoxy-quinolin-4-yl)-propyl]-pipéridine-4-carboxylate d'éthyle dans 3 cm³ de dioxanne, 3 cm³ de méthanol et 1,4 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10 g), en éluant par un mélange de dichlorométhane-méthanol-ammoniaque (90/9/0,9 en volumes) puis par un mélange chloroforme-méthanol-ammoniaque (77,5/19,5/3). Les fractions contenant le produit sont réunies, concentrées à sec sous pression réduite (2 kPa) à une température voisine de 40°C et séchées au dessicateur (10 kPa). On obtient 0,3 g d'acide 1-[2-(2,5-difluoro-phénylthio)-éthyl]-4-[3-(3-fluoro-6-méthoxy-quinolin-4-yl)-propyl]-pipéridine-4-carboxylique sous la forme d'un solide blanc.
Spectre de R.M.N. ¹H (400 MHz, (CD₃)₂SO d6 avec ajout de quelques gouttes de CF₃COOD, δ en ppm) : de 1,50 à 1,75 (mt : 6H) ; 2,21 (d large, J = 14 Hz : 2H) ; 2,89 (t large, J = 12,5 Hz : 2H) ; 3,16 (mt : 2H) ; de 3,25 à 3,50 (mt : 4H) ; 3,54 (d large, J = 12,5 Hz : 2H) ; 3,99 (s : 3H) ; 7,14 (mt : 1H) ; 7,30 (t dédoublé, J = 9 et 5 Hz : 1H) ; 7,39 (mt : 1H) ; 7,47 (d, J = 2,5 Hz : 1H) ; 7,54 (dd, J = 9 et 2,5 Hz : 1H) ; 8,07 (d, J = 9 Hz : 1H) ; 8,97 (d, J = 2 Hz : 1H).

### 1-[2-(2,5-Difluoro-phénylthio)-éthyl]-4-[3-(3-fluoro-6-méthoxy-quinolin-4-yl)-propyl]-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,324 g de 2-(2-bromo-éthylthio)-1,4-difluoro-benzène, 0,181 g d'iodure de potassium et 0,74 g de carbonate de potassium dans 15 cm³ d'acétonitrile est agité pendant 18 heures à une température voisine de 70°C. Après refroidissement aux environs de 20°C, le mélange réactionnel concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,27 g de 1-[2-(2,5-difluoro-phénylthio)-éthyl]-4-[3-(3-fluoro-6-méthoxy-quinolin-4-yl)-propyl]-pipéridine-4-carboxylate d'éthyle.
Spectre de R.M.N. ¹H (500 MHz, (CD₃)₂SO d6, δ en ppm) : 0,97 (t, J = 7 Hz : 3H); 1,31 (t large, J = 11 Hz : 2H) ; de 1,40 à 1,60 (mt: 4H) ; de 1,80 à 2,00 (mt : 4H) ; de 2,35 à 2,70 (mt : 4H) ; 3,01 (mt : 2H) ; 3,07 (t large, J = 7 Hz : 2H) ; 3,91 (s : 3H); 3,94 (q, J = 7 Hz : 2H) ; 7,00 (mt : 1H) ; 7,21 (mt : 1H) ; 7,26 (mt : 1H) ; 7,30 (s large : 1H) ; 7,37 (d large, J = 9 Hz : 1H) ; 7,93 (d, J = 9 Hz : 1H) ; 8,66 (s large : 1H).

Le 4-[3-(3-fluoro-6-méthoxyquinolin-4-yl)-propyl]-pipéridine-4-carboxylate d'éthyle a été préparé dans l'exemple 11.

1-(2-Bromo-éthylthio)-2,5-difluorobenzène a été préparé dans l'exemple 14.

### Exemple 36

### Acide 1-[2-(2,5-difluoro-phénoxy)-éthyl]-4-[3-(3-fluoro-6-méthoxy-quinolin-4-yl)-propyl]-pipéridine-4-carboxylique

Un mélange de 0,38 g de 1-[2-(2,5-difluoro-phénoxy)-éthyl]-4-[3-(3-fluoro-6-méthoxy-quinolin-4-yl)-propyl]-pipéridine-4-carboxylate d'éthyle dans 3 cm³ de dioxanne, 3 cm³ de méthanol et 2,1 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ; masse 10 g), en éluant par un mélange de dichlorométhane-méthanol-ammoniaque (90/9/0,9 en volumes) puis par un mélange chloroforme-méthanol-ammoniaque (77,5/19,5/3). Les fractions contenant le produit sont réunies, concentrées à sec sous pression réduite (2 kPa) à une température voisine de 40°C et séchées au dessicateur (10 kPa). On obtient 0,36 g d'acide 1-[2-(2,5-difluoro-phénoxy)-éthyl]-4-[3-(3-fluoro-6-méthoxy-quinolin-4-yl)-propyl]-pipéridine-4-carboxylique sous la forme d'un solide blanc.
Spectre de R.M.N. ¹H (300 MHz, (CD₃)₂SO d6, δ en ppm) : 1,32 (t large, J = 13 Hz : 2H); de 1,50 à 1,70 (mt : 4H) ; 1,96 (d large, J = 13 Hz : 2H) ; 2,09 (t large, J = 11,5 Hz : 2H) ; 2,64 (t, J = 6 Hz : 2H) ; 2,70 (d large, J = 11,5 Hz : 2H) ; 3,05 (t très large, J = 6,5 Hz : 2H) ; 3,96 (s : 3H) ; 4,13 (t, J = 6 Hz : 2H) ; 6,75 (mt : 1H) ; 7,14 (mt : 1H) ; 7,24 (mt : 1H) ; 7,34 (d, J = 2,5 Hz : 1H) ; 7,41 (dd, J = 9 et 2,5 Hz : 1H) ; 7,97 (d, J = 9 Hz : 1H) ; 8,69 (s large: 1H).

### 1-[2-(2,5-Difluoro-phénoxy)-éthyl]-4-[3-(3-fluoro-6₋méthoxy-quinolin-4-yl)-propyl]-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,30 g de 2-(2-bromo-éthoxy)-1,4-difluoro-benzène, 0,181 g d'iodure de potassium et 0,74 g de carbonate de potassium dans 15 cm³ d'acétonitrile est agité pendant 18 heures à une température voisine de 70°C. Après refroidissement aux environs de 20°C, le mélange réactionnel est concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,4 g de 1-[2-(2,5-ditluoro-phénoxy)-éthyl]-4-[3-(3-fluoro-6-méthoxy-quinolin-4-yl)-propyl]-pipéridine-4-carboxylate d'éthyle.
Spectre de R.M.N. ¹H (500 MHz, (CD₃)₂SO d6, δ en ppm) : 0,97 (t, J = 7 Hz : 3H) ; 1,36 (t large, J = 11,5 Hz : 2H) ; de 1,40 à 1,60 (mt : 4H) ; de 1,80 à 2,10 (mt : 4H) ; 2,61 (t large, J = 5,5 Hz : 2H) ; 2,69 (d large, J = 11 Hz : 2H) ; 3,02 (mt : 2H) ; 3,92 (s : 3H) ; 3,94 (q, J = 7 Hz : 2H) ; 4,09 (mt : 2H) ; 6,71 (mt : 1H) ; 7,10 (mt : 1H) ; 7,19 (mt : 1H) ; 7,29 (s large: 1H) ; 7,37 (d large, J = 9 Hz : 1H) ; 7,93 (d, J = 9 Hz : 1H) ; 8,66 (s large: 1H).

Le 4-[3-(3-fluoro-6-méthoxyquinolin-4-yl)-propyl]-pipéridine-4-carboxylate d'éthyle a été préparé dans l'exemple 11.

Le 2-(2-bromo-éthoxy)-1,4-difluoro-benzène a été préparé dans l'exemple 16.

### Exemple 37

### Acide 4-[3-(3-fluoro-6-méthoxy-quinolin-4-yl)-propyl]-1-[2-(2,3,5-trifluoro-phénoxy)-éthyl]-pipéridine-4-carboxylique

Un mélange de 0,42 g de 4-[3-(3-fluoro-6-méthoxy-quinolin-4-yl)-propyl]-1-[2-(2,3,5-trifluoro-phénoxy)-éthyl]-pipéridine-4-carboxylate d'éthyle dans 3 cm³ de dioxanne, 3 cm³ de méthanol et 2,3 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10 g), en éluant par un mélange de dichlorométhane-méthanol-ammoniaque (90/9/0,9 en volumes) puis par un mélange chloroforme-méthanol-ammoniaque (77,5/19,5/3). Les fractions contenant le produit sont réunies, concentrées à sec sous pression réduite (2 kPa) à une température voisine de 40°C et séchées au dessicateur (10 kPa). On obtient 0,39 g d'acide 4-[3-(3-fluoro-6-méthoxy-quinolin-4-yl)-propyl]-1-[2-(2,3,5-trifluoro-phénoxy)-éthyl]-pipéridine-4-carboxylique sous la forme d'un solide blanc.
Spectre de R.M.N. ¹H (300 MHz, (CD₃)₂SO d6, δ en ppm) : 1,32 (t très large, J = 12,5 Hz : 2H) ; 1,60 (mt : 4H) ; 1,95 (d large, J = 12,5 Hz : 2H) ; 2,09 (t large, J = 11 Hz : 2H) ; 2,64 (t, J = 5,5 Hz : 2H) ; 2,70 (d large, J = 11 Hz : 2H) ; 3,04 (t très large, J = 6 Hz : 2H) ; 3,96 (s : 3H) ; 4,16 (t, J = 5,5 Hz : 2H) ; de 6,95 à 7,15 (mt : 2H) ; 7,34 (d, J = 2,5 Hz : 1H) ; 7,40 (dd, J = 9 et 2,5 Hz : 1H) ; 7,96 (d, J = 9 Hz : 1H) ; 8,69 (s large: 1H).

### 4-[3-(3-Fluoro-6-méthoxy-quinolin-4-yl)-propyl]-1-[2-(2,3,5-tritluoro-phénoxy)-éthyl]-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,33 g de 1-(2-bromo-éthoxy)-2,3,5-trifluoro-benzène, 0,181 g d'iodure de potassium et 0,74 g de carbonate de potassium dans 15 cm³ d'acétonitrile est agité pendant 18 heures à une température voisine de 70°C. Après refroidissement aux environs de 20°C, le mélange réactionnel est concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ ; masse 12 g ; volume 25 cm³), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,43 g de 4-[3-(3-fluoro-6-méthoxy-quinolin-4-yl)-propyl]-1-[2-(2,3,5-trifluoro-phénoxy)-éthyl]-pipéridine-4-carboxylate d'éthyle.
Spectre de R.M.N. ¹H (500 MHz, (CD₃)₂SO d6, δ en ppm) : 0,98 (t, J = 7 Hz : 3H) ; 1,34 (t large, J = 11,5 Hz : 2H) ; de 1,45 à 1,60 (mt : 4H) ; de 1,85 à 2,10 (mt : 4H) ; de 2,50 à 2,70 (mt : 4H) ; 3,02 (mt : 2H) ; 3,92 (s : 3H) ; 3,94 (q, J = 7 Hz : 2H) ; 4,14 (mt : 2H) ; 7,02 (mt : 2H) ; 7,30 (s large : 1H) ; 7,37 (d large, J = 9 Hz : 1H) ; 7,94 (d, J = 9 Hz : 1H) ; 8,66 (s large : 1H).

Le 4-[3-(3-fluoro-6-méthoxyquinolin-4-yl)-propyl]-pipéridine-4-carboxylate d'éthyle a été préparé dansl'exemple 11.

Le 1-(2-bromo-éthoxy)-2,3,5-trifluoro-benzène a été préparé comme décrit dans l'exemple 13.

### Exemple 38

### Acide 4-[3-(3-fluoro-6-méthoxy-quinolin-4-yl)-propyl]-1-heptyl-pipéridine-4-carboxylique

Un mélange de 0,16 g de 4-[3-(3-fluoro-6-méthoxy-quinolin-4-yl)-propyl]-1-heptyl-pipéridine-4-carboxylate d'éthyle dans 3 cm³ de dioxanne, 3 cm³ de méthanol et 1 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ; masse 10g), en éluant par un mélange de dichlorométhane-méthanol-ammoniaque (40/5/0,5 en volumes) puis par un mélange chloroforme-méthanol-ammoniaque (13/3/0,5). Les fractions contenant le produit sont réunies, concentrées à sec sous pression réduite (2 kPa) à une température voisine de 40°C et séchées au dessicateur (10 kPa). On obtient 0,07 g d'acide 4-[3-(3-fluoro-6-méthoxy-quinolin-4-yl)-propyl]-1-heptyl-pipéridine-4-carboxylique sous la forme d'un solide blanc.
Spectre de R.M.N. ¹H (400 MHz, (CD₃)₂SO d6 avec ajout de quelques gouttes de CF₃COOD, δ en ppm) : 0,85 (t, J = 7 Hz : 3H) ; de 1,15 à 1,35 (mt : 8H) ; de 1,50 à 1,75 (mt : 8H) ; 2,19 (d large, J = 14 Hz : 2H) ; 2,78 (t large, J = 12,5 Hz : 2H) ; 3,02 (mt : 2H) ; 3,19 (t large, J = 7 Hz : 2H) ; 3,45 (d large, J = 12,5 Hz : 2H) ; 4,00 (s : 3H) ;7,50 (d, J = 2,5 Hz : 1H) ; 7,58 (dd, J = 9 et 2,5 Hz : 1H) ; 8,09 (d, J = 9 Hz : 1H);9,04 (d, J = 2 Hz : 1H).

### 4-[3-(3-Fluoro-6-méthoxy-quinolin-4-yl)-propyl]-1-heptyl-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,23 g de 1-bromoheptane, 0,18 g d'iodure de potassium et 0,737 g de carbonate de potassium dans 15 cm³ d'acétonitrile est agité pendant 18 heures à une température voisine de 70°C. Après refroidissement aux environs de 20°C, le mélange réactionnel est concentré à sec sous pression réduite (5 kPa) à une température voisine de 40°C. Le résidu d'évaporation est purifié par chromatographie sous pression atmosphérique sur une cartouche de gel de silice (granulométrie 70-200 µ ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,2 g de 4-[3-(3-fluoro-6-méthoxy-quinolin-4-yl)-propyl]-1-heptyl-pipéridine-4-carboxylate d'éthyle.
Spectre de R.M.N. ¹H (500 MHz, (CD₃)₂SO d6, δ en ppm) : 0,84 (t, J = 7 Hz : 3H) ; 0,98 (t, J = 7 Hz : 3H) ; de 1,10 à 1,30 (mt : 8H) ; 1,33 (mt :4H) : de 1,40 à 1,60 (mt : 4H) ; 1,83 (t large, J = 11 Hz : 2H) ; 1,93 (d large, J = 13 Hz : 2H) ; 2,13 (mt : 2H) ; de 2,35 à 2,55 (mt : 2H); 3,01 (mt : 2H); 3,89 (s : 3H) ; 3,94 (mt : 2H) ; 7,31 (s large : 1H) ; 7,37 (d large, J = 9 Hz : 1H) ; 7,94 (d, J = 9 Hz : 1H) ; 8,67 (s large : 1H).

Le 4-[3-(3-fluoro-6-méthoxyquinolin-4-yl)-propyl]-pipéridinc-4-carboxylate d'éthyle a éte préparé dans l'exemple 11.

### Exemple 39

### Acide 4-[3-(3-fluoro-6-méthoxy-quinolin-4-yl)-propyl]-1-(2-phénylthio-éthyl)-pipéridine-4-carboxylique

Un mélange de 0,39 g de 4-[3-(3-fluoro-6-méthoxy-quinolin-4-yl)-propyl]-1-(2-phénylthio-éthyl)-pipéridine-4-carboxylate d'éthyle dans 3 cm³ de dioxanne, 3 cm³ de méthanol et 2,3 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie atmosphérique, sur une cartouche de gel de silice (bond élut ; granulométrie 70-200 µ ; masse 10 g), en éluant par un mélange de dichlorométhane-méthanol-ammoniaque (90/9/0,9 en volumes) puis par un mélange chloroforme-méthanol-ammoniaque (77,5/19,5/3). Les fractions contenant le produit sont réunies, concentrées à sec sous pression réduite (2 kPa) à une température voisine de 40°C et séchées au dessicateur (10 kPa). On obtient 0,3 g d'acide 4-[3-(3-fluoro-6-méthoxy-quinolin-4-yl)-propyl)-1-(2-phénylthio-éthyl)-pipéridine-4-carboxylique sous la forme d'un solide blanc.
Spectre de R.M.N. ¹H (400 MHz, (CD₃)₂SO d6 avec ajout de quelques gouttes de CF₃COOD, δ en ppm) : de 1,50 à 1,75 (mt : 6H) ; 2,20 (d large, J = 14 Hz : 2H) ; 2,87 (t large, J = 13 Hz : 2H) ; 3,16 (mt : 2H) ; 3,41 (s : 4H) ; 3,53 (d large, J = 13 Hz : 2H) ; 4,00 (s : 3H) ; 7,25 (t large, J = 7,5 Hz : 1H) ; de 7,30 à 7,45 (mt : 4H) ; 7,48 (d, J = 2,5 Hz : 1H) ; 7,54 (dd, J = 9 et 2,5 Hz : 1H) ; 8,07 (d, J = 9 Hz : 1H) ; 8,97 (d, J = 2 Hz : 1H).

### 4-[3-(3-Fluoro-6-méthoxy-quinolin-4-yl)-propyl]-1-(2-phénylthio-éthyl)-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,28 g de 2-bromoéthylphénylsulfide, 0,181 g d'iodure de potassium et 0,74 g de carbonate de potassium dans 15 cm³ d'acétonitrile est agité pendant 18 heures à une température voisine de 70°C. Après refroidissement aux environs de 20°C, le mélange réactionnel est concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ ; masse 12 g), en éluant par un mélange.d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,4 g de 4-[3-(3-fluoro-6-méthoxy-quinolin-4-yl)-propyl]-1-(2-phénylthio-éthyl)-pipéridine-4-carboxylate d'éthyle.
Spectre de R.M.N ¹H (500 MHz, (CD₃)₂SO d6, δ en ppm) : 0,97 (t, J = 7 Hz : 3H) ; 1,33 (t large, J = 12,5 Hz : 2H) ; de 1,45 à 1,65 (mt : 4H) ; de 1,85 à 2,00 (mt : 4H) ; de 2,30 à 2,70 (mt : 4H) ; 3,01 (mt : 4H) ; 3,92 (s : 3H) ; 3,94 (mt : 2H) ; 7,15 (mt : 1H) ; de 7,20 à 7,35 (mt : 5H) ; 7,37 (d large, J = 9 Hz : 1H) ; 7,93 (d, J = 9 Hz : 1H) ; 8,66 (s large : 1H).

Le 4-[3-(3-fluoro-6-méthoxyquinolin-4-yl)-propyl]-pipéridine-4-carboxylate d'éthyle a éte préparé dans l'exemple 11.

### Exemple 40

### Acide 4-[3-(3-fluoro-6-méthoxy-quinolin-4-yl)-propyl]-1-[2-(3-fluoro-phénylthio)-éthyl]-pipéridine-4-carboxylique

Un mélange de 0,2 g de 4-[3-(3-fluoro-6-méthoxy-quinolin-4-yl)-propyl]-1-(2-phénylthio-éthyl)-pipéridine-4-carboxylate d'éthyle dans 3 cm³ de dioxanne, 3 cm³ de méthanol et 1,1 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10 g), en éluant par un mélange de dichlorométhane-méthanol-ammoniaque (90/9/0,9 en volumes) puis par un mélange chloroforme-méthanol-ammoniaque (77,5/19,5/3). Les fractions contenant le produit sont réunies, concentrées à sec sous pression réduite (2 kPa) à une température voisine de 40°C et séchées au dessicateur (10 kPa). On obtient 0,18 g d'acide 4-[3-(3-fluoro-6-méthoxy-quinolin-4-yl)-propyl]- 1-[2-(3-fluoro-phénylthio)-éthyl]-pipéridine-4-carboxylique.
Spectre de R.M.N. ¹H (400 MHz, (CD₃)₂SO d6 avec ajout de quelques gouttes de CF₃COOD, δ en ppm) : de 1,50 à 1,75 (mt : 6H) ; 2,20 (d large, J = 14 Hz : 2H) ; 2,89 (t large, J = 12,5 Hz : 2H) ; 3,18 (mt : 2H) ; de 3,25 à 3,45 (mt : 4H) ; 3,54 (d large, J = 12,5 Hz : 2H) ; 3,99 (s : 3H) ; 7,05 (t dédoublé, J = 9 et 2,5 Hz : 1H) ; de 7,15 à 7,30 (mt : 2H) ; 7,38 (mt : 1H) ; 7,48 (d, J = 2,5 Hz : 1H) ; 7,55 (dd, J = 9 et 2,5 Hz : 1H) ; 8,08 (d, J = 9 Hz : 1H) ; 9,00 (d, J = 2 Hz : 1H).

### 4-[3-(3-Fluoro-6-méthoxy-quinolin-4-yl)-propyl]-1-(2-phénylthio-éthyl)-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,30 g de 3-fluoro-2-bromoéthylphénylsulfure, 0,18 g d'iodure de potassium et 0,74 g de carbonate de potassium dans 15 cm³ d'acétonitrile est agité pendant 18 heures à une température voisine de 70°C. Après refroidissement aux environs de 20°C, le mélange réactionnel est concentré à sec sous pression réduite (5 kPa) à une température voisine de 40°C. Le résidu d'évaporation est purifié par chromatographie sous pression d'argon atmosphérique sur une cartouche de gel de silice (bond élut ; granulométrie 70-200 µ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,22 g de 4-[3-(3-fluoro-6-méthoxy-quinolin-4-yl)-propyl]-1-(2-phénylthio-éthyl)-pipéridine-4-carboxylate d'éthyle.
Spectre de R.M.N. ¹H (500 MHz, (CD₃)₂SO d6, δ en ppm) : 0,98 (t, J = 7 Hz : 3H) ; 1,33 (t large, J = 12 Hz : 2H) ; de 1,45 à 1,65 (mt : 4H) ; de 1,85 à 2,00 (mt : 4H) ; de 2,30 à 2,70 (mt : 4H) ; 3,02 (mt : 2H) ; 3,07 (t, J = 6,5 Hz : 2H) ; 3,92 (s : 3H) ; 3,94 (q, J = 7 Hz : 2H) ; 6,94 (t large, J = 8,5 Hz :1H) ; de 7,05 à 7,20 (mt : 2H) ; de 7,25 à 7,35 (mt : 2H) ; 7,37 (d large, J = 9 Hz : 1H) ; 7,94 (d, J = 9 Hz : 1H) ; 8,66 (s large : 1H).

Le 4-[3-(3-fluoro-6-méthoxyquinolin-4-yl)-propyl]-pipéridine-4-carboxylate d'éthyle a éte préparé dans l'exemple 11.

Le 3-fluoro-2-bromoéthylphénylsulfure a éte préparé selon la méthode décrite dans l'exemple 14.

### Exemple 41

### Acide 1-[2-(3,4-difluoro-phénoxy)-éthyl]-4-[3-(3-fluoro-6-méthoxy-quinolin-4-yl)-propyl]-pipéridine-4-carboxylique

Un mélange de 0,38 g de 1-[2-(3,4-difluoro-phénoxy)-éthyl]-4-[3-(3-fluoro-6-méthoxy-quinolin-4-yl)-propyl]-pipéridine-4-carboxylate d'éthyle dans 3 cm³ de dioxanne, 3 cm³ de méthanol et 2,1 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10 g), en éluant par un mélange de dichlorométhane-méthanol-ammoniaque (90/9/0,9 en volumes) puis par un mélange chloroforme-méthanol-ammoniaque (77,5/19,5/3). Les fractions contenant le produit sont réunies, concentrées à sec sous pression réduite (2 kPa) à une température voisine de 40°C et séchées au dessicateur (10 kPa). On obtient 0,3 g d'acide 1-[2-(3,4-difluoro-phénoxy)-éthyl]-4-[3-(3-fluoro-6-méthoxy-quinolin-4-yl)-propyl]-pipéridine-4-carboxylique sous la forme d'un solide blanc.
Spectre de R.M.N ¹H (400 MHz, (CD₃)₂SO d6 avec ajout de quelques gouttes de CF₃COOD, δ en ppm) : de 1,50 à 1,75 (mt : 6H) ; 2,21 (d large, J = 14 Hz : 2H) ; 2,96 (t large, J = 13 Hz : 2H) ; 3,19 (t large, J = 6,5 Hz : 2H) ; de 3,50 à 3,65 (mt : 4H) ; 4,00 (s : 3H) ; 4,31 (t, J = 5,5 Hz : 2H) ; 6,82 (mt : 1H) ; 7,11 (mt : 1H) ; 7,35 (mt : 1H) ; 7,50 (d, J = 2,5 Hz : 1H) ; 7,57 (dd, J = 9 et 2,5 Hz : 1H) ; 8,09 (d, J = 9 Hz : 1 H) ; 9,03 (d, J = 2 Hz : 1H).

### 1-[2-(3,4-Ditluoro-phénoxy)-éthyl]-4-[3-(3-tluoro-6-méthoxy-quinolin-4-yl)-propyl]-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,3 g de 4-(2-bromo-éthoxy)-1,2-difluoro-benzène, 0,18 g d'iodure de potassium et 0,74 g de carbonate de potassium dans 15 cm³ d'acétonitrile est agité pendant 18 heures à une température voisine de 70°C. Après refroidissement aux environs de 20°C, le mélange réactionnel est concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,4 g de 1-[2-(3,4-difluoro-phénoxy)-éthyl]-4-[3-(3-fluoro-6-méthoxy-quinolin-4-yl)-propyl]-pipéridine-4-carboxylate d'éthyle.
Spectre de R.M.N. ¹H (500 MHz, (CD₃)₂SO d6, δ en ppm) : 0,98 (t, J = 7 Hz : 3H) ; 1,36 (t large, J = 12 Hz : 2H) ; de 1,45 à 1,65 (mt : 4H) ; 1,93 (d large, J = 12 Hz : 2H) ; 2,00 (t large, J = 11,5 Hz : 2H) ; de 2,40 à 2,70 (mt : 4H) ; 3,02 (mt : 2H) ; 3,92 (s : 3H) ; 3,94 (q, J = 7 Hz : 2H) ; 3,99 (mt : 2H) ; 6,73 (mt : 1H) ; 7,03 (mt : 1H) ; de 7,25 à 7,35 (mt : 2H) ; 7,37 (d large, J = 9 Hz : 1H) ; 7,93 (d, J = 9 Hz : 1H) ; 8,66 (s large : 1H).

### 4-(2-Bromo-éthoxy)-1,2-difluoro-benzène

Un mélange de 15 g de 3,4-difluorophénol, 23,5 g de carbonate de potassium et 60 cm³ de 1,2-dibromoéthane dans 250 cm³ d'acétonitrile est agité à une température voisine de 70°C pendant 18 heures. Après refroidissement aux environs de 20°C, le mélange réactionnel est filtré sur de la célite puis concentré à sec sous pression réduite (2 kPa) à une température voisine de 40°C. Le résidu obtenu est purifié par chromatographie sous pression atmosphérique sur une colonne de gel de silice (granulométrie 70-200 µ ; diamètre 8 cm ; masse 400 g), en éluant par de l'éther de pétrole 40-60°C. Les fractions contenant le produit sont réunies puis concentrées à sec sous pression réduite (2 kPa) au voisinage de 40°C. On obtient 15,7 g de 4-(2-bromo-éthoxy)-1,2-difluoro-benzène, sous forme d'une huile.
Spectre infra-rouge (CCl₄):1609; 1516; 1264; 1253; 1215; 1206; 1162; 1019; 854 et 834 cm⁻¹ .

Le 4-[3-(3-fluoro-6-méthoxyquinolin-4-yl)-propyl]-pipéridine-4-carboxylate d'éthyle a été préparé dans l'exemple 11.

### Exemple 42

### Acide 4-[3-(3-fluoro-6-méthoxy-quinolin-4-yl)-propyl]-1-(2-phénoxy-éthyl)-pipéridine-4-carboxylique

Un mélange de 0,39 g de 4-[3-(3-fluoro-6-méthoxy-quinolin-4-yl)-propyl]-1-(2-phénoxy-éthyl)-pipéridine-4-carboxylate d'éthyle dans 3 cm³ de dioxanne, 3 cm³ de méthanol et 2,4 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10g), en éluant par un mélange de dichlorométhane-méthanol-ammoniaque (90/9/0,9 en volumes) puis par un mélange chloroforme-méthanol-ammoniaque (77,5/19,5/3). Les fractions contenant le produit sont réunies, concentrées à sec sous pression réduite (2 kPa) à une température voisine de 40°C et séchées au dessicateur (10 kPa). On obtient 0,33 g d'acide 4-[3-(3-fluoro-6-méthoxy-quinolin-4-yl)-propyl]-1 -(2-phénoxy-éthyl)-pipéridine-4-carboxylique sous la forme d'un solide blanc.
Spectre de R.M.N. ¹H (300 MHz, (CD₃)₂SO d6 avec ajout de quelques gouttes de CF₃COOD, δ en ppm) : 1,36 (t large, J = 12,5 Hz : 2H) ; de 1,50 à 1,70 (mt : 4H) ; 1,96 (d large, J = 12,5 Hz : 2H) ; 2,08 (t large, J = 11,5 Hz : 2H) ; 2,62 (t, J = 6 Hz : 2H) ; 2,71 (d large, J = 11,5 Hz : 2H) ; 3,05 (t large, J = 6,5 Hz : 2H) ; 3,97 (s : 3H) ; 4,03 (t, J = 6 Hz : 2H) ; de 6,85 à 7,00 (mt : 3H) ; 7,29 (t large, J = 8 Hz : 2H) ; 7,35 (d, J = 2,5 Hz : 1H) ; 7,40 (dd, J = 9 et 2,5 Hz : 1H) ; 7,97 (d, J = 9 Hz : 1H) ; 8,70 (s large : 1 H).

### 4-[3-(3-Fluoro-6-méthoxy-quinolin-4-yl)-propyl]-1-(2-phénoxy-éthyl)-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,26 g de 2-bromoéthylphényléther, 0,181 g d'iodure de potassium et 0,737 g de carbonate de potassium dans 15 cm³ d'acétonitrile est agité pendant 18 heures à une température voisine de 70°C. Après refroidissement aux environs de 20°C, le mélange réactionnel concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,4 g de 4-[3-(3-fluoro-6-méthoxy-quinolin-4-yl)-propyll]-1-(2-phénoxy-éthyl)-pipéridine-4-carboxylate d'éthyle.
Spectre de R.M.N. ¹H (500 MHz, (CD₃)₂SO d6, δ en ppm) : 0,99 (t, J = 7 Hz : 3H) ; 1,37 (mt : 2H) ; de 1,45 à 1,65 (mt : 4H) ; de 1,85 à 2,10 (mt : 4H) ; de 2,35 à 2,75 (mt : 4H) ; 3,03 (mt : 2H) ; 3,93 (s : 3H) ; 3,96 (q, J = 7 Hz : 2H) ; 4,02 (mt : 2H) ; 6,90 (mt : 3H) ; 7,26 (t large, J = 7,5 Hz : 2H) ; 7,32 (s large : 1H) ; 7,38 (d large, J = 9 Hz : 1H) ; 7,95 (d, J = 9 Hz : 1H) ; 8,68 (s large : 1H).

Le 4-[3-(3-fluoro-6-méthoxyquinolin-4-yl)-propyl]-pipéridine-4-carboxylate d'éthyle a été préparé dans l'exemple 11.

### Exemple 43

### Acide 4-[3-(3-fluoro-6-méthoxy-quinolin-4-yl)-propyl]-1-[2-(3-fluoro-phénoxy)-éthyl]-pipéridine-4-carboxylique

Un mélange de 0,415 g de 4-[3-(3-fluoro-6-méthoxy-quinolin-4-yl)-propyl]-1-[2-(3-fluoro-phénoxy)-éthyl]-pipéridine-4-carboxylate d'éthyle dans 3 cm³ de dioxanne, 3 cm³ de méthanol et 2,4 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10 g), en éluant par un mélange de dichlorométhane-méthanol-ammoniaque (90/9/0,9 en volumes) puis par un mélange chloroforme-méthanol-ammoniaque (77,5/19,5/3). Les fractions contenant le produit sont réunies, concentrées à sec sous pression réduite (2 kPa) à une température voisine de 40°C et séchées au dessicateur (10 kPa). On obtient 0,34 g d'acide 4-[3-(3-fluoro-6-méthoxy-quinolin-4-yl)-propyl]-1-[2-(3-fluoro-phénoxy)-éthyl]-pipéridine-4-carboxylique sous la forme d'un solide blanc.
Spectre de R.M.N. ¹H (300 MHz, (CD₃)₂SO d6 avec ajout de quelques gouttes de CF₃COOD, δ en ppm) : 1,34 (t large, J = 12,5 Hz : 2H) ; de 1,50 à 1,70 (mt : 4H) ; 1,96 (d large, J = 12,5 Hz : 2H) ; 2,08 (t large, J = 11 Hz : 2H) ; 2,63 (t, J = 6 Hz : 2H) ; 2,70 (d large, J = 11 Hz : 2H) ; 3,05 (t large, J = 6,5 Hz : 2H) ; 3,97 (s : 3H) ; 4,06 (t, J = 6 Hz : 2H) ; de 6,70 à 6,90 (mt : 3H) ; de 7,25 à 7,40 (mt : 1H) ; 7,35 (d, J = 2,5 Hz : 1H) ; 7,40 (dd, J = 9 et 2,5 Hz : 1H) ; 7,97 (d, J = 9 Hz : 1H) ; 8,70 (s large : 1H).

### 4-[3-(3-Fluoro-6-méthoxy-quinolin-4-yl)-propyl]-1-[2-(3-fluoro-phénoxy)-éthyl]-pipéridine-4-carboxylate d'éthyle

Un mélange de 0,4 g de 4-[3-(3-fluoro-6-méthoxy-quinolin-4-yl)-propyl]-pipéridine-4-carboxylate d'éthyle, 0,281 g de 1-(2-bromo-éthoxy)-3-fluoro-benzène, 0,181 g d'iodure de potassium et 0,737 g de carbonate de potassium dans 15 cm³ d'acétonitrile est agité pendant 18 heures à une température voisine de 70°C. Après refroidissement aux environs de 20°C, le mélange réactionnel est concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,43 g de 4-[3-(3-fluoro-6-méthoxy-quinolin-4-yl)-propyl]-1-[2-(3-fluoro-phénoxy)-éthyl]-pipéridine-4-carboxylate d'éthyle.
Spectre de R.M.N. ¹H (500 MHz, (CD₃)₂SO d6, δ en ppm) : 0,98 (t, J = 7 Hz : 3H) ; 1,35 (t large, J = 12 Hz : 2H) ; de 1,45 à 1,65 (mt : 4H) ; 1,92 (d large, J = 12 Hz : 2H) ; 2,00 (t large, J = 11,5 Hz : 2H) ; de 2,30 à 2,70 (mt : 4H) ; 3,02 (mt : 2H) ; 3,91 (s : 3H) ; 3,94 (q, J = 7 Hz : 2H) ; 4,01 (mt : 2H) ; de 6,65 à 6,80 (mt : 3H) ; de 7,20 à 7,30 (mt : 1H) ; 7,28 (s large : 1H) ; 7,36 (d large, J = 9 Hz : 1H) ; 7,93 (d, J = 9 Hz : 1H) ; 8,65 (s large : 1H).

### 1-(2-Bromo-éthoxy)-3-fluoro-benzène

Un mélange de 8,6 g de 3-fluorophénol, 15,3 g de carbonate de potassium et 40,5 cm³ de 1,2-dibromoéthane dans 200 cm³ d'acétonitrile est agité, sous atmosphère inerte, à une température voisine de 70°C pendant 25 heures. Après refroidissement aux environs de 20°C, la suspension est filtrée, l'insoluble est rincé par 3 fois 50 cm³ d'acétonitrile puis le filtrat est concentré à sec sous pression réduite (2 kPa) à une température voisine de 40°C. Le résidu obtenu est repris par 50 cm³ d'éther diéthylique puis filtré et les jus de filtration sont concentrés à sec comme dans les conditions précédentes. L'huile obtenue par évaporation est purifiée par chromatographie sous pression d'argon de 50 kPa sur une colonne de gel de silice (granulométrie 20-45 µ ; diamètre 4,5 cm ; hauteur 22 cm), en éluant par de l'éther de pétrole 40-60°C. Les fractions 8 à 60 sont réunies puis concentrées à sec sous pression réduite (2 kPa) au voisinage de 40°C. On obtient 7,67 g de 1-(2-bromo-éthoxy)-3-fluoro-benzène.
Spectre infra-rouge (CCl₄) : 1616; 1596; 1492; 1279; 1265; 1168; 1140;1025; 853; 834 et 679 cm⁻¹.

Le 4-[3-(3-fluoro-6-méthoxyquinolin-4-yl)-propyl]-pipéridine-4-carboxylate d'éthyle a éte préparé dans l'exemple 11.

### Exemple 44

### Acide 1-[2-(2,6-difluoro-phénoxy)-éthyl]-4-[3-(3-fluoro-6-méthoxy-quinolin-4-yl)-propyl]-pipéridine-4-carboxylique

Un mélange de 0,39 g de 1-[2-(2,6-difluoro-phénoxy)-éthyl]-4-[3-(3-fluoro-6-méthoxy-quinolin-4-yl)-propyl]-pipéridine-4-carboxylate d'éthyle dans 3 cm³ de dioxanne, 3 cm³ de méthanol et 2,2 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10 g), en éluant par un mélange de dichlorométhane-méthanol-ammoniaque (90/9/0,9 en volumes) puis par un mélange chloroforme-méthanol-ammoniaque (77,5/19,5/3). Les fractions contenant le produit sont réunies, concentrées à sec sous pression réduite (2 kPa) à une température voisine de 40°C et séchées au dessicateur (10 kPa). On obtient 0,3 g d'acide 1-[2-(2,6-difluoro-phénoxy)-éthyl]-4-[3-(3-fluoro-6-méthoxy-quinolin-4-yl)-propyl]-pipéridine-4-carboxylique sous la forme d'un solide blanc.
Spectre de R.M.N. ¹H (300 MHz, (CD₃)₂SO d6, δ en ppm) : 1,33 (t large, J = 12,5 Hz : 2H) ; de 1,50 à 1,65 (mt : 4H) ; 1,91 (d large, J = 12,5 Hz : 2H) ; 2,05 (t large, J = 11 Hz : 2H) ; 2,60 (t, J = 6 Hz : 2H) ; 2,63 (mt : 2H) ; 3,05 (mt : 2H) ; 3,97 (s : 3H) ; 4,16 (t, J = 6 Hz : 2H) ; de 7,05 à 7,20 (mt : 3H) ; 7,35 (d, J = 2,5 Hz : 1H) ; 7,40 (dd, J = 9 et 2,5 Hz : 1H) ; 7,97 (d, J = 9 Hz : 1H) ; 8,70 (s large : 1H).

### 1-[2-(2,6-Difluoro-phénoxy)-éthyl]-4-[3-(3-fluoro-6-méthoxy-quinolin-4-yl)-propyl]-pipéridine-4-carboxylate d'éthyle

Un mélange de 0,4 g 4-[3-(3-fluoro-6-méthoxy-quinolin-4-yl)-propyl]-pipéridine-4-carboxylate d'éthyle, 0,304 g de 1-(2-bromo-éthoxy)-3-fluoro-benzène, 0,181 g d'iodure de potassium et 0,74 g de carbonate de potassium dans 15 cm³ d'acétonitrile est agité pendant 18 heures à une température voisine de 70°C. Après refroidissement aux environs de 20°C, le mélange réactionnel est concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,4 g de 1-[2-(2,6-difluoro-phénoxy)-éthyl]-4-[3-(3-fluoro-6-méthoxy-quinolin-4-yl)-propyl]-pipéridine-4-carboxylate d'éthyle.
Spectre de R.M.N. ¹H (500 MHz, (CD₃)₂SO d6, δ en ppm) : 0,97 (t, J = 7 Hz : 3H) ; 1,26 (t large, J = 125 Hz : 2H) ; de 1,40 à 1,60 (mt : 4H) ; 1,88 (d large, J = 12 Hz : 2H) ; 1,98 (t large, J = 11 Hz : 2H) ; de 2,30 à 2,65 (mt : 4H) ; 3,00 (mt : 2H) ; 3,92 (s : 3H) ; 3,94 (mt : 2H) ; 4,11 (mt : 2H) ; de 6,95 à 7,10 (mt : 3H) ; 7,30 (s large : 1H) ; 7,37 (d large, J = 9 Hz : 1H) ; 7,93 (d, J = 9 Hz : 1H) ; 8,66 (s large : 1H).

Le 4-[3-(3-fluoro-6-méthoxyquinolin-4-yl)-propyl]-pipéridine-4-carboxylate d'éthyle a éte préparé dans l'exemple 11.

Le 1-(2-bromo-éthoxy)-3-fluoro-benzène a éte préparé selon la méthode décrite dans l'exemple 15

### Exemple 45

### Acide 1-[2-(2,3-difluoro-phénoxy)-éthyl]-4-[3-(3-fluoro-6-méthoxy-quinolin-4-yl)-propyl]-pipéridine-4-carboxylique

Un mélange de 0,42 g de 1-[2-(2,3-difluoro-phénoxy)-éthyl]-4-[3-(3-fluoro-6-méthoxy-quinolin-4-yl)-propyl]-pipéridine-4-carboxylate d'éthyle dans 3 cm³ de dioxanne, 3 cm³ de méthanol et 2,4 cm³ d'une solution aqueuse de soude 5N est agité à une température voisine de 70°C pendant 17 heures. Après refroidissement aux environs de 20°C, le mélange réactionnel est concentré à sec sous pression réduite successive de 30 kPa à 2,5 kPa et à une température voisine de 45°C. Le résidu est repris par 5 cm³ d'un mélange dichlorométhane-métanol-ammoniaque (40/5/0,5 en volumes) puis est purifié par chromatographie sous pression atmosphérique, sur une cartouche de gel de silice (bond élut ; granulométrie 70-200 µ ; masse 10 g), en éluant par un mélange de dichlorométhane-méthanol-ammoniaque (90/9/0,9 en volumes) puis par un mélange chloroforme-méthanol-ammoniaque (77,5/19,5/3). Les fractions contenant le produit sont réunies, concentrées à sec sous pression réduite (2 kPa) à une température voisine de 40°C et séchées au dessicateur (10 kPa). On obtient 0,32 g d'acide 1-[2-(2,3-difluoro-phénoxy)-éthyl]-4-[3-(3-fluoro-6-méthoxy-quinolin-4-yl)-propyl]-pipéridine-4-carboxylique sous la forme d'un solide blanc.
Spectre de R.M.N. ¹H (300 MHz, (CD₃)₂SO d6, δ en ppm) : 1,34 (t large, J = 12,5 Hz : 2H) ; de 1,50 à 1,70 (mt : 4H) ; 1,96 (d large, J = 12,5 Hz : 2H) ; 2,09 (t large, J = 11,5 Hz : 2H) ; 2,65 (t, J = 6 Hz : 2H) ; 2,71 (d large, J = 11,5 Hz : 2H) ; 3,05 (mt : 2H) ; 3,97 (s : 3H) ; 4,16 (t, J = 6 Hz : 2H) ; de 6,90 à 7,20 (mt : 3H) ; 7,34 (d, J = 2,5 Hz : 1H) ; 7,40 (dd, J = 9 et 2,5 Hz : 1H) ; 7,97 (d, J = 9 Hz : 1H) ; 8,70 (s large : 1H).

### 1-[2-(2,3-Difluoro-phénoxy)-éthyl]-4-[3-(3-fluoro-6-méthoxy-quinolin-4-yl)-propyl]-pipéridine-4-carboxylate d'éthyle

Un mélange de 0,4 g 4-[3-(3-fluoro-6-méthoxy-quinolin-4-yl)-propyl]-pipéridine-4-carboxylate d'éthyle, 0,3 g de 1-(2-bromo-éthoxy)-2,3-difluoro-benzène, 0,181 g d'iodure de potassium et 0,74 g de carbonate de potassium dans 15 cm³ d'acétonitrile est agité pendant 18 heures à une température voisine de 70°C. Après refroidissement aux environs de 20°C, le mélange réactionnel est concentré à sec sous pression réduite (5 kPa) à une température voisine de 40°C. Le résidu d'évaporation est purifié par chromatographie sous pression atmosphérique sur une cartouche de gel de silice (bond élut ; granulométrie 70-200 µ ; masse 12 g), en éluant par un mélange d'acétate d'éthyle-éther de pétrole 40-60°C (8/2 en volumes). Les fractions contenant le produit sont réunies puis concentrées à sec sous pression réduite (2 kPa) à une température voisine de 40°C. On obtient 0,43 g de 1-[2-(2,3-difluoro-phénoxy)-éthyl]-4-[3-(3-fluoro-6-méthoxy-quinolin-4-yl)-propyl]-pipéridine-4-carboxylate d'éthyle.
Spectre de R.M.N. ¹H (500 MHz, (CD₃)₂SO d6, δ en ppm) : 0,99 (mt : 3H) ; 1,34 (mt : 2H) ; de 1,40 à 1,60 (mt : 4H) ; 1,93 (d large, J = 12,5 Hz : 2H) ; de 1,95 à 2,10 (mt : 2H) ; de 2,45 à 2,70 (mt : 4H) ; 3,02 (mt : 2H) ; 3,93 (s : 3H) ; 3,95 (mt : 2H) ; 4,12 (mt : 2H) ; de 6,85 à 7,05 (mt : 2H) ; 7,09 (mt : 1 H) ; 7,31 (s large : 1H) ; 7,37 (d large, J = 9 Hz : 1H) ; 7,94 (d, J = 9 Hz : 1H) ; 8,66 (s large : 1H).

Le 4-[3-(3-fluoro-6-méthoxyquinolin-4-yl)-propyl]-pipéridine-4-carboxylate d'éthyle a éte préparé dans l'exemple 11.

Le 1-(2-bromo-éthoxy)-2,3-difluoro-benzène a éte préparé dans l'exemple 17.

### Exemple 46

### Acide 4-[3-(3-fluoro-quinolin-4-yl)-propyl]-1-[2-(thien-2-yl)-thioéthyl]-pipéridine-4-carboxylique

Un mélange de 1,29 g de 4-[3-(3-fluoro-quinolin-4-yl)-propyl]-1-[2-(thien-2-yl)-thioéthyl]-pipéridine-4-carboxylate d'éthyle dans 65 cm³ de dioxanne, 65 cm³ de méthanol et 8 cm³ d'une solution aqueuse de soude 5N est agité à une température voisine de 70°C pendant 20 heures. Il est ajouté 8 cm³ d'une solution aqueuse de soude 5N au mélange réactionnel qui est agité au voisinage de 70°C pendant 6 heures supplémentaires. Après refroidissement aux environs de 20°C, la masse réactionnelle est concentrée à sec sous pression réduite (2 kPa) à une température voisine de 50°C. Le résidu est purifié par chromatographie sous pression atmosphérique, sur une colonne de gel de silice (granulométrie 20-45 µ ; diamètre 6 cm ; hauteur 30 cm), en éluant par un mélange de chloroforme-méthanol-ammoniaque (12/3/0,5 en volumes) et en recueillant des fractions de 100 cm³. Les fractions 8 à 24 sont réunies, concentrées à sec sous pression réduite (2 kPa) à une température voisine de 40°C. Le solide blanc est repris par 60 cm³ d'éther diisopropylique et agité pendant 18 heures à une température voisine de 20°C. La suspension est filtrée, lavée par 3 fois 15 cm³ d'éther diisopropylique, essorée puis séchée sous pression réduite (10 kPa) aux environs de 50°e. On obtient 0,8 g d'acide 4-[3-(3-fluoro-quinolin-4-yl)-propyl]-1-[2-(thien-2-yl)-thioéthyl]-pipéridine-4-carboxylique, sous forme d'un solide blanc fondant à 180°C.
Spectre de R.M.N. ¹H (300 MHz, (CD₃)₂SO d6, δ en ppm) : 1,27 (mt : 2H) ; 1,56 (mt : 4H) ; de 1,85 à 2,05 (mt : 4H) ; 2,44 (t large, J = 7 Hz : 2H) ; 2,57 (mt : 2H) ; 2,90 (t large, J = 7 Hz : 2H) ; 3,06 (mf : 2H) ; 7,04 (dd, J = 5,5 et 3,5 Hz : 1H) ; 7,17 (dd, J = 3,5 et 1,5 Hz : 1H) ; 7,60 (dd, J = 5,5 et 1,5 Hz : 1H) ; 7,71 (t large, J = 7,5 Hz : 1H) ; 7,77 (t large, J = 7,5 Hz : 1H) ; 8,08 (d large, J = 7,5 Hz : 1H) ; 8,15 (d large, J = 7,5 Hz : 1H) ; 8,89 (d, J = 1,5 Hz : 1H).

### 4-[3-(3-Fluoro-quinolin-4-yl)-propyl]-1-[2-(thien-2-yl)-thioéthyl]-pipéridine-4-carboxylate d'éthyle

A un mélange de 1,8 g de monochlorhydrate 1-(2-chloro-éthyl)-4-[3-(3-fluoro-quinolin-4-yl)-propyl]-pipéridine-4-carboxylate d'éthyle, 0,48 cm³ de thiophène-2-thiol, 2,8 g de carbonate de potassium et 0,75 g d'iodure de potassium dans 200 cm³ d'acétonitrile anhydre est agité sous atmosphère inerte pendant 18 heures à une température voisine de 70°C. Après refroidissement aux environs de 20°C, la suspension est filtrée, lavée par 3 fois 30 cm³ d'acétonitrile puis le filtrat est concentré à sec sous pression réduite (2 kPa) à une température voisine de 50°C. Le résidu d'évaporation est purifié par chromatographie sous pression atmosphérique sur une colonne de gel de silice (granulométrie 20-45 µ ; diamètre 6 cm ; hauteur 30 cm) en éluant par de l'acétate d'éthyle et en recueillant des fractions de 50 cm³. Les fractions 25 à 52 sont réunies puis concentrées à sec sous pression réduite (2 kPa) à une température voisine de 40°C. On obtient 1,3 g de 4-[3-(3-fluoro-quinolin-4-yl)-propyl]-1-[2-(thiophèn-2-ylthio)-éthyl]-pipéridine-4-carboxylate d'éthyle, sous forme d'une huile visqueuse de couleur orangée.
Spectre de R.M.N. ¹H (300 MHz, (CD₃)₂SO d6, δ en ppm) : 0,99 (t, J = 7 Hz : 3H) ; 1,34 (t très large, J = 13 Hz : 2H) ; 1,56 (mt : 4H) ; de 1,85 à 2,00 (mt : 4H) ; 2,45 (t large, J = 7 Hz : 2H) ; 2,59 (d large, J = 11,5 Hz : 2H) ; 2,90 (t large, J = 7 Hz : 2H) ; 3,07 (t large, J = 6,5 Hz : 2H) ; 3,95 (q, J = 7 Hz : 2H) ; 7,04 (dd, J = 5,5 et 3,5 Hz : 1H) ; 7,17 (dd, J = 3,5 et 1,5 Hz : 1H) ; 7,60 (dd, J = 5,5 et 1,5 Hz : 1H) ; 7,70 (t large, J = 7,5 Hz : 1H) ; 7,77 (t dédoublé, J = 7,5 et 1,5 Hz : 1H) ; 8,08 (d large, J = 7,5 Hz : 1H) ; 8,13 (d large, J = 7,5 Hz : 1H) ; 8,89 (d, J = 1 Hz : 1H).

### Chlorhydrate de 1-(2-chloro-éthyl)-4-[3-(3-fluoro-quinolin-4-yl)-propyl]-pipéridine-4-carboxylate d'éthyle

A une solution de 1,55 g de 4-[3-(3-fluoro-quinolin-4-yl)-propyl]-1-(2-hydroxy-éthyl)-pipéridine-4-carboxylate d'éthyle dans 35 cm³ de dichlorométhane, on ajoute sous agitation à une température voisine de 20°C, 1,2 cm³ de chlorure de thionyle dans 5 cm³ de dichlorométhane. Après 42 heures d'agitation à une température voisine de 20°C, le mélange réactionnel est concentré à sec sous pression réduite (1,2 kPa) aux environs de 50°C. La meringue obtenue est reprise par 3 fois 100 cm³ de cyclohexane, concentrée à sec sous pression réduite (2 kPa) à une température voisine de 40°C, séchée à l'étuve sous pression réduite (10 kPa) à une température voisine de 20°C. On obtient 1,8 g de monochlorhydrate de 1-(2-chloro-éthyl)-4-[3-(3-fluoro-quinolin-4-yl)-propyl]-pipéridine-4-carboxylate d'éthyle, sous forme d'un solide de couleur crème.
Spectre de R.M.N. ¹H (300 MHz, (CD₃)₂SO d6, δ en ppm) : 1,03 (t, J = 7 Hz : 3H) ; de 1,45 à 2,10 (mt : 6H) ; 2,16 (d large, J = 14 Hz : 2H) ; de 2,70 à 3,00 (mt : 2H) ; 3,12 (mt : 2H) ; de 3,40 à 3,55 (mt : 4H) ; de 3,95 à 4,10 (mt : 4H) ; 7,72 (t large, J = 7,5 Hz : 1H) ; 7,79 (t large, J = 7,5 Hz : 1H) ; 8,09 (d large, J = 7,5 Hz : 1H) ; 8,18 (d large, J = 7,5 Hz : 1H) ; 8,92 (s large : 1H) ; de 10,10 à 10,35 (mf: 1H).

### 4-[3-(3-Fluoro-quinolin-4-yl)-propyl]-1-(2-hydroxy-éthyl)-pipéridine-4-carboxylate d'éthyle

A une solution de 4,4 g de 4-[3-(3-fluoro-quinolin-4-yl)-propyl]-pipéridine-4-carboxylate d'éthyle dans 100 cm³ d'acétonitrile anhydre on ajoute à une température voisine de 20°C, sous une vive agitation et atmosphère inerte, 1,14 cm³ de 2-iodoéthanol et 1,9 g de carbonate de potassium. Le mélange réactionnel est agité pendant 18 heures au voisinage de 20°C, filtré, lavé par 3 fois 30 cm³ d'acétonitrile. Le filtrat est concentré à sec sous pression réduite (2 kPa) à une température voisine de 50°C. Le résidu d'évaporation est purifié par chromatographie sous pression atmosphérique sur une colonne de gel de silice (granulométrie 20-45 µ ; diamètre 5 cm ; hauteur 32 cm) en éluant par du dichlorométhane-méthanol-ammoniaque (40/5/0,5 en volumes) et en recueillant des fractions de 100 cm³. Les fractions 8 à 12 sont réunies puis concentrées à sec sous pression réduite (2 kPa) à une température voisine de 40°C. On obtient 3,5 g de 4-[3-(3-fluoro-quinolin-4-yl)-propyl]-1-(2-hydroxy-éthyl)-pipéridine-4-carboxylate d'éthyle, sous forme d'une huile visqueuse de couleur orangée.
Spectre de R.M.N. ¹H (300 MHz, (CD₃)₂SO d6, δ en ppm) : 0,99 (t, J = 7 Hz : 3H) ; 1,36 (mt : 2H) ; 1,57 (mt : 4H) ; de 1,85 à 2,05 (mt : 4H) ; 2,29 (t, J = 6,5 Hz : 2H) ; 2,61 (d mt, J = 12 Hz : 2H) ; 3,08 (t large, J = 6 Hz : 2H) ; 3,45 (mt : 2H) ; 3,96 (q, J = 7 Hz : 2H) ; 4,3.1 (t, J = 5,5 Hz : 1H) ; 7,70 (t large, J = 7,5 Hz : 1H) ; 7,77 (t dédoublé, J = 7,5 et 1,5 Hz : 1H); 8,08 (dd, J = 7,5 et 1,5 Hz : 1H) ; 8,14 (dd, J = 7,5 et 1,5 Hz : 1H) ; 8,89 (d, J = 1 Hz : 1H).

### 4-[3-(3-Fluoro-quinolin-4-yl)-propyl]-pipéridine-4-carboxylate d'éthyle

A une solution de 8,7 g de 4-[3-(3-fluoro-quinolin-4-yl)-propyl]-1-(*tert-*butyloxycarbonyl)-pipéridine-4-carboxylate d'éthyle dans 150 cm³ de dioxanne anhydre, on ajoute précautionneusement 50 cm³ d'une solution de d'acide chlorhydrique dans le dioxanne à une concentration de 4 M et la température est maintenue en-dessous de 30°C lors de l'addition. Après 18 heures d'agitation au voisinage de 20°C, la suspension est diluée par 250 cm³ d'éther diéthylique, filtrée, lavée par 5 fois 50 cm³ d'éther diéthylique et le solide est séché au dessicateur sous pression réduite (2 kPa) et à une température voisine de 20°C. Le solide est repris par 50 cm³ d'eau et l'on ajoute une solution de soude aqueuse 5N pour que le pH soit aux environs de 10 puis l'on extrait par 5 fois 100 cm³ d'éther diéthylique. Les phases organiques sont réunies, séchées sur sulfate de magnésium, reprises avec du noir végétal (3S), filtrées et concentrées à sec sous pression réduite (2 kPa) à une température voisine de 50°C. On obtient 4,7 g de 4-[3-(3-fluoro-quinolin-4-yl)-propyl]-pipéridine-4-carboxylate d'éthyle, sous forme d'une huile visqueuse de couleur orangée.
- Spectre de masse :: EI m/z=344 M⁺ m/z=288 C₁₇H₁₉FNO₂⁺ m/z=184 C₁₀H₁₈NO₂⁺
pic de base m/z=161 C₁₀H₈FN⁺

### 4-[3-(3-Fluoro-quinolin-4-yl)-propyl]-1-(tert-butyloxycarbonyl)-pipéridine-4-carboxylate d'éthyle

On refroidit, à une température voisine de -30°C, 17,7 g de 4-allyl-1-(*tert-*butyloxycarbonyl)-pipéridin-4-carboxylate d'éthyle dans 200 cm³ de tétrahydrofuranne et l'on ajoute sous agitation et sous atmosphère inerte 135 cm³ d'une solution 0,5 M de 9-borabicyclo-[3,3,1]-nonane dans le tétrahydrofurane. Après l'addition, la température du mélange est ramenée aux environs de 20°C et l'on agite pendant 2 heures. On ajoute 14,8 g de 3-fluoro-4-iodo-quinoline dans 430 cm³ de tétrahydrofurane, 1,3 g de chlorure de palladium diphénylphosphino ferrocène, 29,8 g de phosphate de potassium tribasique. Le mélange réactionnel est ensuite chauffé au voisinage de 70°C pendant 20 heures. Après refroidissement à une température voisine de 20°C, la masse réactionnelle est filtrée, lavée par 3 fois 100 cm³ de tétrahydrofurane. Le filtrat est concentré à sec sous pression réduite (2 kPa) à une température voisine de 40°C. Le résidu d'évaporation est repris par 500 cm³ d'éther diéthylique, l'insoluble est lavé par 3 fois 100 cm³ d'éther diéthylique et le filtrat est concentré à sec sous pression réduite (2 kPa) à une température voisine de 40°C. L'huile obtenue est purifiée par chromatographie, sous pression atmosphérique, sur une colonne de gel de silice (granulométrie 20-45 µ; diamètre 6 cm ; hauteur 45 cm), en éluant par un mélange de dichlorométhane-acétate d'éthyle (90/10 en volumes), et en recueillant des fractions de 120 cm³. Les fractions 30 à 76 sont réunies puis concentrées à sec selon les mêmes conditions que ci-dessus. On obtient 13,7 g de 4-[3-(3-fluoro-quinolin-4-yl)-propyl]-1-(tert-butyloxycarbonyl)-pipéridine-4-carboxylate d'éthyle, sous forme d'une huile visqueuse de couleur orangée.
- Spectre de masse :: EI m/z=444 M⁺ m/z=388 [M - tBu]⁺ m/z=343 [M - BOC]⁺
m/z=288 C₁₇H₁₉FNO₂⁺ m/z=184 C₁₀H₁₈NO₂⁺
m/z=161 C₁₀H₈FN⁺ m/z=57 C₄H₉⁺

### 3-Fluoro-4-iodo-quinoline

On refroidit, à une température voisine de -75°C, 17,3 cm³ de diisopropylamine dans 650 cm³ de tétrahydrofurane et l'on ajoute sous agitation et sous atmosphère inerte 76 cm³ d'une solution 1,6 M de butyllithium dans l'hexane en maintenant la température aux environs de -70°C. Après une agitation de 20 minutes à une température voisine de -75°C on ajoute une solution de 11,9 g de 3-fluoro-quinoline dans 200 cm³ de tétrahydrofurane. La solution obtenue est agitée pendant encore 4 heures à -75°C, puis l'on ajoute une solution de 32,2 g d'iode bisublimé dans 150 cm³ de tétrahydrofurane. Après 2 heures d'agitation à une température voisine de -40°C, le mélange réactionnel est hydrolysé par 200 cm³ d'un mélange tétrahydrofurane-eau (90/10 en volumes) puis 200 cm³ d'une solution saturée de chlorure de sodium. Au voisinage de 20°C, le mélange est dilué par 300 cm³ d'acétate d'éthyle et lavé par 2 fois 250 cm³ d'une solution saturée en chlorure de sodium. La phase organique est séchée sur du sulfate de magnésium, filtrée, concentrée à sec sous pression réduite (2 kPa) à une température voisine de 50°e. Le résidu obtenu est purifié par chromatographie sous pression atmosphérique, sur une colonne de gel de silice (granulométrie 20-45 µ ; diamètre 10 cm ; hauteur 30 cm), en éluant avec du dichlorométhane et en recueillant des fractions de 100 cm³. Les fractions 45 à 80 sont réunies puis concentrées à sec sous pression réduite (2 kPa) à une température voisine de 40°C. On obtient 15,1 g de 3-fluoro-4-iodo-quinoline, sous forme d'un solide de couleur crème fondant à 110°C.
Spectre de masse : El m/z=273 M⁺- pic de base m/z=146 [M-I]⁺

### 3-Fluoro-quinoline

A 100 cm³ d'acide tétrafluoroborique refroidi aux alentours de 0°C, il est ajouté précautionneusement sous une vive agitation, 23,5 g d'amino-3-quinoline, 12,1 g de nitrite de sodium dans 20 cm³ d'eau distillée et le mélange réactionnel est ainsi agité pendant 30 minutes. La suspension est filtrée, essorée, lavée par 3 fois 30 cm³ d'acide tétrafluoroborique glacée, 50 cm³ d'éthanol glacée et 4 fois 30 cm³ d'éther diéthylique. Le solide est séché au dessicateur (2 kPa) au voisinage de 20°C puis repris dans 200 cm³ de toluène et chauffé à une température voisine de 90°C pendant 1 heure et sous agitation. Après refroidissement aux environs de 20°C, la masse réactionnelle est décantée et l'huile insoluble est lavée par 3 fois 100 cm³ de toluène et reprise dans 110 cm³ d'eau et l'on alcalinise par addition lente d'hydrogénocarbonate de sodium pour que le pH soit aux alentours de 8. La phase aqueuse est extraite par 5 fois 100 cm³ d'éther diéthylique et les phases organiques sont réunies, lavées par 2 fois 50 cm³ d'eau, séchées sur du sulfate de magnésium et reprises avec du noir végétal (3S), filtrées, concentrées à sec sous pression réduite (2 kPa) à une température voisine de 45°C. L'huile est reprise par 50 cm³ d'un mélange éther de pétrole 40-60°C-acétate d'éthyle (90/10 en volumes) et l'insoluble est filtré, rincé par 2 fois 25 cm³ d'un mélange éther de pétrole 40-60°C-acétate d'éthyle (90/10 en volumes), séché au dessicateur sous pression réduite (2 IcPa) à une température voisine de 20°C. Le filtrat est concentré à sec sous pression réduite (2 kPa) à une température voisine de 40°C. Le résidu obtenu est purifié par chromatographie sous pression atmosphérique, sur une colonne de gel de silice (granulométrie 20-45 µ ; diamètre 5 cm ; hauteur 45 cm), en éluant par un mélange éther de pétrole 40-60°C-acétate d'éthyle (90/10 en volumes) et en recueillant des fractions de 100 cm³. Les fractions 20 à 31 sont réunies puis concentrées à sec sous pression réduite (2 kPa) à une température voisine de 40°C. On obtient 13 g de 3-fluoro-quinoline, sous forme d'un liquide incolore.
- Spectre de masse :: EI m/z=147 M⁺ pic de base m/z=127 [M-HF]⁺
m/z=120 [M - HCN]⁺

Le 4-allyl-1-(tert-butoxycarbonyl)-pipéridin-4-carboxylate d'éthyle a été préparé dans l'exemple 1.

### Exemple 47

On prépare le 4-[3-(3-chloro-6-méthoxy-quinolin-4-yl)-propyl]-1-[2-(pyridin-2-yloxy)-éthyl]-pipéridine-4-carboxylate d'éthyle, sous forme d'une huile visqueuse incolore.
Spectre de R.M.N. ¹H (300 MHz, (CD₃)₂SO d6, δ en ppm) : 1,04 (t, J = 7 Hz : 3H); 1,39 (t très large, J = 12 Hz: 2H); 1,51 (mt : 2H); 1,68 (mt : 2H) ; de 1,90 à 2,15 (mt : 4H) ; 2,60 (t, J = 6 Hz : 2H) ; 2,70 (d large, J = 12 Hz : 2H) ; 3,16 (t large, J = 7,5 Hz : 2H) ; 3,95 (s : 3H) ; 3,99 (q, J = 7 Hz : 2H) ; 4,31 (t, J = 6 Hz : 2H) ; 6,78 (d, J = 8 Hz : 1H) ; 6,96 (dd large, J = 7,5 et 5 Hz : 1H) ; 7,37 (d, J = 2,5 Hz : 1H) ; 7,45 (dd, J = 9 et 2,5 Hz : 1H) ; 7,69 (ddd, J = 8 - 7,5 et 2 Hz : 1H) ; 7,96 (d, J = 9 Hz : 1H); 8,14 (dd large, J = 5 et 2 Hz: 1H); 8,67 (s: 1H).

### Acide 4-[3-(3-chloro-6-méthoxy-quinolin-4-yl)-propyl]-1-[2-(pyridin-2-yloxy)-éthyl]-pipéridine-4-carboxylique

Un mélange de 0,12 g de 4-[3-(3-chloro-6-méthoxy-quinolin-4-yl)-propyl]-1-[2-(pyridin-2-yloxy)-éthyl]-pipéridine-4-carboxylate d'éthyle dans 5 cm³ de dioxanne, 5 cm³ de méthanol et 1 cm³ d'une solution aqueuse de soude 5N est agité à une température voisine de 70°C pendant 21 heures. Après refroidissement aux environs de 20°C, la masse réactionnelle est concentrée à sec sous pression réduite (5 kPa) à une température voisine de 60°C. Le résidu est repris par 5 cm³ d'un mélange de dichlorométhane-méthanol-ammoniaque (40/5/0,5 en volumes) puis purifié par chromatographie sous pression atmosphérique, sur une colonne de gel de silice (granulométrie 70-200 µ ; diamètre 1,5 cm ; masse 20 g), en éluant par un mélange de dichlorométhane-méthanol-ammoniaque (40/5/0,5 en volumes). Les fractions contenant le produit sont réunies, concentrées à sec sous pression réduite (2 kPa) à une température voisine de 40°C. La meringue est reprise par 2 cm³ de méthanol, filtrée, lavée par 1 cm³ de méthanol et 2 cm³ d'éther diéthylique puis séchée à l'étuve sous pression réduite (10 kPa) aux environs de 50°C. On obtient 0,115 g d'acide 4-[3-(3-chloro-6-méthoxy-quinolin-4-yl)-propyl]-1-[2-(pyridin-2-yloxy)-éthyl]-pipéridine-4-carboxylique, sous forme d'un solide blanc fondant aux environs de 70°C en collant.

Spectre de R.M.N. ¹H (300 MHz, (CD₃)₂SO d6, δ en ppm) : de 1,20 à 1,80 (mt : 6H) ; 1,98 (d très large, J = 13,5 Hz : 2H) ; 2,09 (t très large, J = 11 Hz : 2H) ; 2,60 (t, J = 6 Hz : 2H) ; 2,67 (mt : 2H) ; 3,16 (mt : 2H) ; 3,98 (s : 3H) ; 4,32 (t, J = 6 Hz : 2H) ; 6,80 (d, J = 8 Hz : 1H) ; 6,96 (ddd, J = 7,5 - 5 et 1 Hz : 1H) ; 7,39 (s large : 1H) ; 7,45 (dd, J = 9 et 2,5 Hz : 1H) ; 7,70 (ddd, J = 8 - 7,5 et 2 Hz : 1H) ; 7,97 (d, J = 9 Hz : 1H) ; 8,16 (dd large, J = 5 et 2 Hz : 1H) ; 8,67 (s : 1H).

### Exemple 48

### Acide 4-[3-(3-chloro-6-méthoxy-quinolin-4-yl)-3-(R,S)-fluoro-propyl]-1-[2-(2,5-difluoro-phénylthio)-éthyl]-pipéridine-4-carboxylique

Un mélange de 0,07 g de 4-[3-(3-chloro-6-méthoxy-quinolin-4-yl)-3-(R,S)-fluoro-propyl]-1-[2-(2,5-difluoro-phénylthio)-éthyl]-pipéridine-4-carboxylate de méthyle dans 3 cm³ de dioxanne, 3 cm³ de méthanol et 0,9 cm³ d'une solution aqueuse de soude 5N est porté à une température voisine de 70°C pendant 4 heures. Après refroidissement aux environs de 20°C, le mélange réactionnel est concentré à sec sous pression réduite (2 kPa) à une température voisine de 40°C. Le résidu est chromatographié sous pression atmosphérique, sur une colonne de gel de silice (granulométrie 70-200 µ ; masse 10 g), en éluant par un mélange dichlorométhane-méthanol-ammoniaque (40/5/0,5 en volumes). Les fractions 10 à 15 sont réunies puis concentrées à sec selon les mêmes conditions que ci-dessus. On obtient 0,04 g de d'acide 4-[3-(3-chloro-6-méthoxy-quinolin-4-yl)-3-(R,S)-fluoro-propyl]-1-[2-(2,5-difluoro-phénylthio)-éthyl]-pipéridine-4-carboxylique, sous forme d'un solide blanc.
Spectre de R.M.N. ¹H (300 MHz, (CD₃)₂SO d6, δ en ppm) : de 1,20 à 2,60 (mt : 12H) ; 2,66 (mt : 2H) ; 3,03 (t, J = 7 Hz : 2H) ; 3,93 (s : 3H) ; 6,38 (mt, J_{HF} = 48 Hz : 1H) ; 7,05 (mt : 1H) ; de 7,20 à 7,40 (mt : 2H) ; 7,52 (dd, J = 9 et 2,5 Hz : 1H) ; 7,56 (d, J = 2,5 Hz : 1H) ; 8,04(d, J = 9 Hz : 1H) ; 8,75 (s large : 1H) ; de 12,30 à 12,70 (mf étalé : 1H).

### 4-[3-(3-Chloro-6-méthoxy-quinolin-4-yl)-3-(R,S)-fluoro-propyl]-1-[2-(2,5-difluoro-phénylthio)-éthyl]-pipéridine-4-carboxylate de méthyle

A une solution de 0,25 g de 4-[3-(3-chloro-6-méthoxy-quinolin-4-yl)-3-(R,S)-hydroxy-propyl]-1 -[2-(2,5-difluoro-phénylthio)-éthyl]-pipéridine-4-carboxylate de méthyle dans 10 cm³ de dichlorométhane on ajoute sous agitation et sous atmosphère inerte, à une température voisine de 5°C, 0,1 cm³ de trifluorure de diéthylaminosulfure. Après 7 heures d'agitation à une température voisine de 20°C, on ajoute au mélange réactionnel une solution d'hydrogénocarbonate de sodium saturée. La phase aqueuse est extraite avec du dichlorométhane. La phase organique est séchée sur sulfate de magnésium, filtrée puis concentrée à sec sous pression réduite (2 kPa) à une température voisine de 40°C. Le résidu obtenu est purifié par chromatographie sous pression atmosphérique, sur une colonne de gel de silice (granulométrie 70-200 µ; masse :15 g), en éluant par 60 cm³ de dichlorométhane, puis par 45 cm³ d'un mélange de acétate d'éthyle-dichlorométhane (1/9 en volumes), puis par 30 cm³ d'un mélange de acétate d'éthyle-dichlorométhane (2/8 en volumes) puis par 210 cm³ d'un mélange de acétate d'éthyle-dichlorométhane (3/7 en volumes) puis par de l'acétate d'éthyle. Les fractions 24 à 26 sont réunies puis concentrées à sec selon les mêmes conditions que ci-dessus. On obtient 0,08 g de 4-[3-(3-chloro-6-méthoxy-quinolin-4-yl)-3-(R,S)-fluoro-propyl]-1-[2-(2,5-difluoro-phénylthio)-éthyl]-pipéridine-4-carboxylate de méthyle, sous forme d'une huile épaisse jaune.
Spectre de R.M.N. ¹H (400 MHz, (CD₃)₂SO d6, δ en ppm) : de 1,20 à 2,60 (mt : 12H) ; de 2,55 à 2,75 (mt : 2H) ; 3,11 (t, J = 7 Hz: 2H) ; 3,54 (s : 3H); 3,93 (s : 3H) ; 6,36 (mt, J_{HF} = 48 Hz : 1H) ; 7,05 (mt : 1H) ; de 7,15 à 7,35 (mt : 2H) ; de 7,45 à 7,55 (mt : 2H) ; 8,03 (d, J = 9 Hz : 1H) ; 8,75 (s : 1H).

### 4-[3-(3-Chloro-6-méthoxy-quinolin-4-yl)-3-(R,S)-hydroxy-propyl]-1-[2-(2,5-difluoro-phénylthio)-éthyl]-pipéridine-4-carboxylate de méthyle

Un mélange de 1,75 g de dichlorhydrate de 4-[3-(3-chloro-6-méthoxyquinolin-4-yl)-propyl]-pipéridin-4-carboxylate d'éthyle, 0,95 g de 2-(2-bromo-éthylthio)-1,4-difluoro-benzène, 0,622 g d'iodure de potassium et 3,11 g de carbonate de potassium dans 30 cm³ d'acétonitrile et 20 cm³ de dimétylformamide est chauffé sous agitation pendant 18 heures à une température voisine de 85°C. Après refroidissement aux environs de 20°C, le mélange réactionnel est filtré sur célite puis le filtrat est concentré à sec sous pression réduite (2 kPa) à une température voisine de 40°C. Le résidu obtenu est repris par de l'eau et de l'éther diéthylique. La phase organique est séchée sur sulfate de magnésium, filtrée puis concentrée à sec comme ci-dessus. Le résidu obtenu est purifié par chromatographie sous pression atmosphérique, sur une colonne de gel de silice (granulométrie 70-200 µ ; diamètre 4 cm), en éluant par un mélange de dichlorométhane-méthanol (97,5/2,5 en volumes). Les fractions contenant le produit sont réunies puis concentrées à sec selon les mêmes conditions que ci-dessus. On obtient 0,6 g de 4-[3-(3-chloro-6-méthoxy-quinolin-4-yl)-3-(R,S)-hydroxy-propyl]-1-[2-(2,5-difluoro-phénylthio)-éthyl]-pipéridine-4-carboxylate de méthyle.
Spectre de R.M.N. ¹H (300 MHz, (CD₃)₂SO d6, δ en ppm) : de 1,20 à 2,15 (mt : 1 10H) ; de 2,45 à 2,55 (mt : 2H) ; 2,64 (mt : 2H) ; 3,12 (t, J = 7 Hz : 2H) ; 3,47 (s : 3H) ; 3,87 (s : 3H) ; 5,41 (mt : 1H) ; 6,10 (mf: 1H) ; 7,04 (mt : 1H) ; de 7,15 à 7,35 (mt : 2H) ; 7,45 (dd, J = 9 et 2,5 Hz : 1H) ; 7,97 (d, J = 9 Hz : 1H) ; 8,13 d très large, J = 2,5 Hz : 1H) ; 8,66 (s : 1H).

Le 2-(2-bromo-éthylthio)-1,4-difluoro-benzène peut être obtenu par application de la méthode décrite dans l'exemple 14.

Le 4-[3-(3-chloro-6-méthoxy-quinolin-4-yl)-3-(R,S)-hydroxy-propyl]-pipéridin-4-carboxylate de méthyle a éte préparé l'exemple 49.

### Exemple 49

### Acide 4-[3-(3-chloro-6-méthoxy-quinolin-4-yl)-3-(R,S)-fluoro-propyl]-1-[2-(2,5-difluoro-phénoxy)-éthyl]-pipéridine-4-carboxylique

Un mélange de 0,15 g de 4-[3-(3-chloro-6-méthoxy-quinolin-4-yl)-3-(R,S)-fluoro-propyl]-1-[2-(2,5-difluoro-phénoxy)-éthyl]-pipéridine-4-carboxylate d'éthyle dans 3 cm³ de dioxanne, 3 cm³ de méthanol et 1 cm³ d'une solution aqueuse de soude 5N est porté à une température voisine de 70°C pendant 4 heures. Après refroidissement aux environs de 20°C, le mélange réactionnel est concentré à sec sous pression réduite (2 kPa) à une température voisine de 40°C. Le résidu est chromatographié sous une pression atmosphérique de 60 kPa, sur un bond élut de 60 cm³ de gel de silice (granulométrie 70-200 µ ; masse 25 g), en éluant par 60 cm³ d'un mélange dichlorométhane-méthanol-ammoniaque (40/5/0,5 en volumes), puis d'un mélange dichlorométhane-méthanol-ammoniaque (40/5/2 en volumes). Les fractions contenant le produit attendu sont réunies puis concentrées à sec selon les mêmes conditions que ci-dessus. On obtient 0,04 g d'acide 4-[3-(3-chloro-6-méthoxy-quinolin-4-yl)-3-(R,S)-fluoro-propyl]-1-[2-(2,5-difluoro-phénoxy)-éthyl]-pipéridine-4-carboxylique, sous forme d'un solide blanc.
Spectre de R.M.N. ¹H (300 MHz, (CD₃)₂SO d6, δ en ppm) : de 1,20 à 2,40 (mt : 10H) ; 2,65 (t, J = 6 Hz : 2H) ; 2,72 (mt : 2H) ; 3,93 (s : 3H) ; 4,03 (t, J = 6 Hz : 2H) ; 6,37 (mt, J_{HF} = 48 Hz : 1H) ; 6,75 (mt : 1H) ; 7,14 (mt : 1H) ; 7,25 (mt : 1H) ; 7,52 (dd, J = 9 et 2,5 Hz : 1H) ; 7,56 (d, J = 2,5 Hz : 1H) ; 8,03 (d, J = 9 Hz : 1H) ; 8,75 (d, J = 1 Hz : 1H).

### 4-[3-(3-Chloro-6-méthoxy-quinolin-4-yl)-3-(R,S)-fluoro-propyl]-1-[2-(2,5-difluoro-phénoxy)-éthyl]-pipéridine-4-carboxylate de méthyle

A une solution de 0,5 g de 4-[3-(3-chloro-6-méthoxy-quinolin-4-yl)-3-(R,S)-hydroxy-propyl]-1-[2-(2,5-difluoro-phénoxy)-éthyl]-pipéridine-4-carboxylate de méthyle dans 20 cm³ de dichlorométhane on ajoute sous agitation et sous atmosphère inerte, à une température voisine de 3°C 0,2 cm³ de trifluorure de diéthylaminosulfure. Après 7 heures d'agitation à une température voisine de 20°C, on ajoute au mélange réactionnel une solution d'hydrogénocarbonate de sodium saturée. La phase aqueuse est extraite avec du dichlorométhane. La phase organique est séchée sur sulfate de magnésium, filtrée puis concentrée à sec sous pression réduite (2 kPa) à une température voisine de 40°C. Le résidu obtenu est purifié par chromatographie sous pression atmosphérique, sur une colonne de gel de silice (granulométrie 70-200 µ ; diamètre 1,5 cm ; masse : 15 g), en éluant par un mélange de acétate d'éthyle-éther de pétrole (40-60°C) (8/2 en volumes). Les fractions 9 à 11 sont réunies puis concentrées à sec dans les conditions ci-dessus. On obtient 0,21 g de 4-[3-(3-chloro-6-méthoxy-quinolin-4-yl)-3-(R,S)-fluoro-propyl]-1-[2-(2,5-difluoro-phénoxy)-éthyl]-pipéridine-4-carboxylate de méthyle, sous forme d'une huile épaisse jaune.
- Spectre de masse :: DCI m/z=551 MH⁺
Présence d'une impureté m'=532.

### 4-[3-(3-Chloro-6-méthoxy-quinolin-4-yl)-3-(R,S)-hydroxy-propyl]-1-[2-(2,5-difluoro-phénoxy)-éthyl]-pipéridine-4-carboxylate de méthyle

Un mélange de 1,55 g de 4-[3-(3-chloro-6-méthoxyquinolin-4-yl)-3-hydroxy-propyl]-pipéridin-4-carboxylate de méthyle, 1,03 g de 1-(2-bromo-éthoxy)-2,5-difluorobenzène, 0,65 g d'iodure de potassium et 2,7 g de carbonate de potassium dans 30 cm³ d'acétonitrile et 20 cm³ de diméthylformamide est chauffé sous agitation à une température voisine de 85°C pendant 17 heures. Après refroidissement à une température voisine de 20°C, la suspension est filtrée sur célite, le filtrat est concentré à sec sous pression réduite (2 kPa) au voisinage de 40°C. Le résidu d'évaporation est repris par du dichlorométhane et de l'eau. La phase organique est séchée sur du sulfate de magnésium, filtrée, concentrée à sec comme dans les conditions précédentes. Le résidu est purifié par chromatographie sous pression atmosphérique, sur une colonne de gel de silice (granulométrie 70-200 µ ; diamètre 4 cm), en éluant par un mélange de dichlorométhane-méthanol (97/3 en volumes). Les fractions contenant le produit sont réunies puis concentrées à sec sous pression réduite (2 kPa), à une température voisine de 40°C selon les mêmes conditions que ci-dessus. On obtient 0,5g de 4-[3-(3-chloro-6-méthoxy-quinolin-4-yl)-3-(R,S)-hydroxy-propyl]-1-[2-(2,5-difluoro-phénoxy)-éthyl]-pipéridine-4-carboxylate de méthyle.
Spectre de R.M.N. ¹H (300 MHz, (CD₃)₂SO d6, δ en ppm) : de 1,20 à 2,20 (mt : 10H) ; de 2,60 à 2,80 (mt : 2H) ; 2,64 (t, J = 6 Hz : 2H) ; 3,48 (s : 3H) ; 3,88 (s : 3H) ; 4,12 (t, J = 6 Hz : 2H) ; 5,40 (mt : 1H) ; 6,10 (s large : 1H) ; 6,74 (mt : 1H) ; 7,13 (mt : 1H) ; 7,24 (mt : 1H) ; 7,44 (dd, J = 9 et 2,5 Hz : 1H) ; 7,96 (d, J = 9 Hz : 1H) ; 8,14 (d, J = 2,5 Hz : 1H) ; 8,66 (s : 1H).

### 4-[3-(3-Chloro-6-méthoxy-quinolin-4-yl)-3-(R,S)-hydroxy-propyl]-pipéridin-4-carboxylate de méthyle

Un mélange de 2,95 g de 4-[3-(3-chloro-6-méthoxy-quinolin-4-yl)-3-(R,S)-hydroxy-propyl]-(*tert*-butyloxycarbonyl)-pipéridin-carboxylate de méthyle et 2,3 cm³ d'acide sulfurique dans 100 cm³ de méthanol est chauffé à une température voisine de 80°C pendant 1 heure et demie. Après refroidissement le mélange réactionnel est concentré à sec sous pression réduite (2 kPa) à une température voisine de 40°C. Le résidu est repris par 10 cm³ d'eau, alcalinisé à pH=11 avec une solution de bicarbonate de sodium saturée puis avec une solution de carbonate de sodium saturée et concentrée à sec sous pression réduite (2 kPa) à une température voisine de 40°C. Le résidu obtenu est purifié par chromatographie sous pression atmosphérique, sur une colonne de gel de silice (granulométrie 20-45 µ; diamètre 2 cm ; volume 80 cm³), en éluant par un mélange de chloroforme-méthanol-ammoniaque (12/3/0,5 en volume). Les fractions 8 à 20 sont réunies puis concentrées à sec selon les mêmes conditions que ci-dessus. On obtient 1,37 g de 4-[3-(3-chloro-6-méthoxy-quinolin-4-yl)-3-(R,S)-hydroxy-propyl]-pipéridin-4-carboxylate de méthyle, sous forme de meringue crème.
Spectre de R.M.N.¹H (300 MHz, (CD₃)₂SO d6, δ en ppm) : de 1,10 à 1,45 (mt : 3H) ; de 1,55 à 1,75 (mt : 1H) ; de 1,80 à 2,05 (mt : 4H) ; 2,40 (mt : 2H) ; 2,72 (mt : 2H) ; 3,50 (s : 3H) ; 3,90 (s : 3H) ; 5,42 (t large, J = 6,5 Hz : 1H) ; 6,09 (mt : 1H) ; 7,45 (dd, J = 9 et 3 Hz : 1H) ; 7,97 (d, J = 9 Hz : 1H) ; 8,15 (d, J = 3 Hz : 1H) ; 8,67 (s : 1H).

### 4-[3-(3-Chloro-6-méthoxy-quinolin-4-yl)-3-(R,S)-hydroxy-propyl]-(tert-butyloxycarbonyl)-pipéridin-carboxylate de méthyle

Une solution de 5 g de 4-[3-(3-chloro-6-méthoxyquinolin-4-yl)-propyl]-1-(*tert-*butyloxycarbonyl)-pipéridin-4-carboxylate d'éthyle dans 400 cm³ de diméthylsulfoxyde et de 120 cm³ de *tert*-butanol est agité sous atmosphère saturée en oxygène à une température voisine de 20°C. Après 5 minutes une solution de 2,8 g de *tert*-butylate de potassium dans 30 cm³ de *tert*-butanol est ajoutée au mélange réactionnel. Après 2 heures de barbotage d'oxygène on ajoute délicatement 300 cm³ d'eau glacée et 3,5 cm³ d'acide acétique. La phase aqueuse est extraite avec 2 fois 200 cm³ de dichlorométhane. Les phases organiques sont réunies, lavées par 4 fois 1 dm³ d'eau. La phase organique est concentrée à sec sous pression réduite (2 kPa) à une température voisine de 40°C. Le résidu est repris par 200 cm³ d'éther diéthylique, filtré, lavé avec 20 cm³ d'éther diéthylique puis séché au dessicateur sous pression réduite (0,1 kPa) à une température voisine de 40°C. On obtient 3 g de 4-[3-(3-chloro-6-méthoxy-quinolin-4-yl)-3-(R,S)-hydroxy-propyl]-(*tert*-butyloxycarbonyl)-pipéridin-carboxylate de méthyle, sous forme d'un solide blanc fondant à 222°C.
Spectre de R.M.N.¹H (300 MHz, (CD₃)₂SO d6, δ en ppm) : de 1,10 à 1,50 (mt : 3H) ; 1,39 (s : 9H) ; 1,70 (mt : 1H) ; de 1,80 à 2,10 (mt : 4H) ; 2,81 (mt : 2H) ; 3,69 (mt : 2H) ; 3,89 (s : 3H) ; 5,41 (dd, J = 9 et 5 Hz : 1H) ; de 5,80 à 6,30 (mf étalé : 1H) ; 7,44 (dd, J = 9 et 3 Hz : 1H) ; 7,95 (d, J = 9 Hz : 1H) ; 8,16 (d, J = 3 Hz : 1H) ; 8,65 (s : 1H) ; de 12,00 à 12,90 (mf étalé : 1H).

Le 1-(2-bromo-éthoxy)-2,5-difluoro-benzène a été préparé dans l'exemple 16.

Le 4-[3-(3-chloro-6-méthoxyquinolin-yl)-propyl]-1-(*tert*-butoxycarbonyl)-pipéridine-4-carboxylate d'éthyle a été préparé dans l'exemple 5.

### Exemple 50

On prépare le 4-[3-(3-chloro-6-méthoxy-quinolin-4-yl)-propyl]-1-[2-(thiazol-2-yloxy)-éthyl]-pipéridine-4-carboxylate d'éthyle.
Spectre de R.M.N. ¹H (300 MHz, (CD₃)₂SO d6, δ en ppm) : 1,05 (t, J = 7 Hz : 3H) ; 1,40 (t très large, J = 12 Hz : 2H) ; 1,53 (mt : 2H) ; 1,69 (mt : 2H) ; de 1,90 à 2,15 (mt : 4H) ; 2,64 (t, J = 5,5 Hz : 2H) ; 2,70 (d large, J = 11,5 Hz : 2H) ; 3,16 (t large, J = 7,5 Hz : 2H) ; 3,96 (s : 3H) ; 4,01 (q, J = 7 Hz : 2H) ; 4,42 (t, J = 5,5 Hz : 2H) ; 7,04 (d, J = 3,5 Hz : 1H) ; 7,17 (d, J = 3,5 Hz : 1H) ; 7,37 (d, J = 2,5 Hz : 1H) ; 7,46 (dd, J = 9 et 2,5 Hz : 1H) ; 7,97 (d, J = 9 Hz : 1H) ; 8,67 (s : 1H).

### Acide 4-[3-(3-chloro-6-méthoxy-quinolin-4-yl)-propyl]-1-[2-(thiazol-2-yloxy)-éthyl]-pipéridine-4-carboxylique

Un mélange de 0,68 g de 4-[3-(3-chloro-6-méthoxy-quinolin-4-yl)-propyl]-1-[2-(thiazol-2-yloxy)-éthyl]-pipéridine-4-carboxylate d'éthyle dans 7 cm³ de dioxanne, 9 cm³ de méthanol et 2 cm³ d'une solution aqueuse de soude 5N est porté à une température voisine de 60°C pendant 20 heures. Après refroidissement aux environs de 20°C, la masse réactionnelle est concentrée à sec sous pression réduite (2 kPa) à une température voisine de 40°C. Le résidu obtenu est chromatographié sous une pression d'argon de 50 kPa, sur une colonne de gel de silice (granulométrie 20-45 µ ; diamètre 2.2 cm ; masse 20 g), en éluant par un mélange de dichlorométhane-méthanol (70/30 en volumes). Les fractions contenant le produit attendu sont réunies puis concentrées à sec dans les conditions ci-dessus. Le résidu obtenu est repris dans 5 cm³ d'acétate d'éthyle, agité pendant 1 heure à une température ambiante puis filtré et rincé avec 3 fois 3 cm³ d'acétate d'éthyle puis 3 fois 3 cm³ de pentane. On obtient 0,27 g d'acide 4-[3-(3-chloro-6-méthoxy-quinolin-4-yl)-propyl]-1-[2-(thiazol-2-yloxy)-éthyl]-pipéridine-4-carboxylique, sous forme d'un solide blanc fondant à 188°C.
Spectre de R.M.N. ¹H (300 MHz, (CD₃)₂SO d6, δ en ppm) : 1,39 (t large, J = 12Hz : 2H); de 1,50 à 1,65 (mt : 2H) ; 1,71 (mt : 2H) ; 1,98 (d large, J = 12 Hz : 2H) ; 2,12 (mf : 2H) ; de 2,60 à 2,85 (mt: 4H) ; 3,17 (t large, J = 7,5 Hz : 2H) ; 3,96 (s : 3H) ; 4,45 (t trèslarge, J = 5,5 Hz : 2H) ; 7,05 (d, J = 3,5 Hz : 1H) ; 7,18 (d, J = 3,5 Hz : 1H) ; 7,37 (d, J = 2,5 Hz : 1H) ; 7,46 (dd, J = 9 et 2,5 Hz : 1H) ; 7,97 (d, J = 9 Hz : 1H) ; 8,68 (s : 1H) ; de 11,80 à 12,70 (mf étalé : 1H).

### Exemple 51

### Acide 4-[3-(3-fluoro-6-méthoxy-quinolin-4-yl)-propyl]-1-[2-(4,5-dihydro-thiazol-2-ylthio)-éthyl]-pipéridine-4-carboxylique

Un mélange de 0,15 g de 4-[3-(3-fluoro-6-méthoxy-quinolin-4-yl)-propyl]-1-[2-(4,5-dihydro-thiazol-2-ylthio)-éthyl]-pipéridine-4-carboxylate d'éthyle dans 5 cm³ de dioxanne, 5 cm³ de méthanol et 1 cm³ d'une solution aqueuse de soude 5N est porté à une température voisine de 75°C pendant 20 heures. Après refroidissement aux environs de 20°C, le mélange réactionnel est concentré à sec sous pression réduite (2 kPa) à une température voisine de 40°C. Le résidu obtenu est purifié par chromatographie sous pression atmosphérique, sur une colonne de gel de silice (bond élut; granulométrie 70-200 µ ; masse 7 g), en éluant par un mélange de dichlorométhane-méthanol-ammoniaque (40/5/0,.5 en volumes). Les fractions contenant le produit sont réunies, concentrées à sec sous pression réduite (2 kPa) à une température voisine de 40°C puis séchées à l'étuve sous pression réduite (10 kPa) aux environs de 50°C. On obtient 0,1 g d'acide 4-[3-(3-fluoro-6-méthoxy-quinolin-4-yl)-propyl]-1-[2-(4,5-dihydro-thiazol-2-ylthio)-éthyl]-pipéridine-4-carboxylique sous forme d'un solide blanc.
Spectre de R.M.N. ¹H (300 MHz, (CD₃)₂SO d6, δ en ppm) : 1,33 (t très large, J = 11,5 Hz : 2H) ; 1,61 (mt : 4H) ; de 1,90 à 2,10 (mt : 4H) ; de 2,45 à 2,60 (mt : 2H) ; 2,63 (d large, J = 11,5 Hz : 2H) ; 3,05 (mt : 2H) ; 3,19 (t, J = 6,5 Hz : 2H) ; 3,44 (t, J = 8 Hz : 2H) ; 3,97 (s : 3H) ; 4,15 (t, J = 8 Hz : 2H) ; 7,35 (s large : 1H) ; 7,40 (d large, J = 9 Hz : 1H) ; 7,97 (d large, J = 9 Hz : 1H) ; 8,69 (s large : 1H).

### 4-[3-(3-fluoro-6-méthoxy-quinolin-4-yl)-propyl]-1-(2-(4,5-dihydro-thiazol-2-ylthio)-éthyl]-pipéridine-4-carboxylate d'éthyle

Un mélange de 0,7 g de dichlorhydrate de 1-(2-chloro-éthyl)-4-[3-(3-fluoro-6-méthoxy-quinolin-4-yl)-propyl]-pipéridine-4-carboxylate d'éthyle, 0,18 g de 2-mercaptothiazoline, 0,63 cm³ de tréthylamine dans 10 cm³ de diméthylformamide est agité sous atmosphère inerte pendant 23 heures à une température voisine de 80°C. Après refroidissement aux environs de 20°C, le mélange réactionnel est repris par de l'eau, extrait avec de l'acétate d'éthyle, lavé avec une solution de chlorure de sodium saturée. La phase organique est séchée sur sulfate de magnésium, filtrée puis concentrée à sec sous pression réduite (2 kPa) à une température voisine de 40°C. A une solution de 0,18 g de 2-mercaptothiazoline, de 0,06 g d'hydrure de sodium à 10 % dans 10 cm³ de diméthylformamide on ajoute le résidu d'évaporation puis on chauffe à une température voisine de 80°C pendant 15 heures. Après refroidissement aux environs de 20°C, le mélange réactionnel est repris par de l'eau, extrait avec de l'acétate d'éthyle, lavé avec une solution de chlorure de sodium saturée. La phase organique est séchée sur sulfate de magnésium, filtrée puis concentrée à sec sous pression réduite (2 kPa) à une température voisine de 40°C. Le résidu obtenu est purifié par chromatographie sous pression atmosphérique sur une colonne de gel de silice (granulométrie 70-200 µ ; diamètre 2 cm ; masse 40 g), en éluant par un mélange de dichlorométhane-éthanol (95/5 en volumes) et en recueillant des fractions de 10 cm³. Les fractions 11 à 24 sont réunies puis concentrées à sec sous pression réduite (2 kPa) à une température voisine de 40°C. On obtient 0,15 g 4-[3-(3-fluoro-6-méthoxy-quinolin-4-yl)-propyl]- 1-[2-(4,5-dihydro-thiazol-2-ylthio)-éthyl]-pipéridine-4-carboxylate d'éthyle, sous forme d'une huile brune.
Spectre de R.M.N. ¹H (300 MHz, (CD₃)₂SO d6 avec ajout de quelques gouttes de CD₃COOD d4, δ en ppm) : 1,06 (t, J = 7 Hz : 3H) ; 1,41 (mt : 2H) ; de 1,50 à 1,70 (mt : 4H) ; 1,98 (d large, J = 13,5 Hz : 2H) ; 2,14 (t large, J = 11,5 Hz : 2H) ; 2,66 (t, J = 7 Hz : 2H) ; 2,80 (d très large, J = 11,5Hz : 2H) ; 3,04 (t large, J = 6,5 Hz : 2H) ; 3,21 (t, J = 7 Hz : 2H) ; 3,42 (t, J = 8 Hz : 2H) ; 3,94 (s : 3H) ; 3,97 (q, J = 7 Hz : 2H) ; 4,12 (t, J = 8 Hz : 2H) ; 7,32 (d, J = 2,5 Hz : 1H) ; 7,38 (dd, J = 9 et 2,5 Hz : 1H) ; 7,96 (d, J = 9 Hz : 1H) ; 8,66 (d, J = 1,5 Hz : 1H).

### Dichlorhydrate de 1-(2-chloro-éthyl)-4-[3-(3-fluoro-6-méthoxy-quinolin-4-yl)-propyl]-pipéridine-4-carboxylate d'éthyle

A une suspension de 0,6 g de 4-[3-(3-fluoro-6-méthoxy-quinolin-4-yl)-propyl]-1-(2-hydroxy-éthyl)-pipéridine-4-carboxylate d'éthyle dans 10 cm³ de dichlorométhane sous agitation, on ajoute goutte à goutte 5,73 cm³ de chlorure de thionyle au voisinage de 20°C et l'ensemble est agité pendant 24 heures à une température voisine de 20°C. Le mélange réactionnel est concentré à sec sous pression réduite (2 kPa) à une température voisine de 40°C. Le résidu est repris par 3 fois 30 cm³ de cyclohexane et évaporé à sec comme dans les conditions précédentes. On obtient 0,67 g de dichlorhydrate de 1-(2-chloro-éthyl)-4-[3-(3-fluoro-6-méthoxy-quinolin-4-yl)-propyl]-pipéridine-4-carboxylate d'éthyle, sous forme d'un solide beige.
Spectre de R.M.N. ¹H (300 MHz, (CD₃)₂SO d6, δ en ppm) : 1,05 (t, J = 7 Hz : 3H) ; de 1,45 à 2,00 (mt : 6H) ; 2,19 (d large, J = 14 Hz : 2H) ; de 2,75 à 2,95 (mt : 2H) ; 3,09 (mt : 2H) ; de 3,40 à 3,60 (mt : 4H) ; de 3,95 à 4,15 (mt : 2H) ; 3,96 (s : 3H) ; 4,05 (q, J = 7 Hz : 2H) ; 7,37 (d, J = 2,5 Hz : 1H) ; 7,42 (dd, J = 9 et 2,5 Hz : 1H) ; 7,99 (d, J = 9 Hz : 1H) ; 8,73 (s large : 1H) ; 10,00 (mf: 1H).

Le 4-[3-(3-fluoro-6-méthoxy-quinolin-4-yl)-propyl]-1-(2-hydroxy-éthyl)-pipéridine-4-carboxylate d'éthyle est préparé par analogie avec la méthode décrite dans l'exemple 46.

### Exemple 52

### Acide 4-[3-(3-fluoro-6-méthoxy-quinolin-4-yl)-propyl]-1-[2-(3,4,5-trifluoro-phénoxy)-éthyl]-pipéridine-4-carboxylique

Un mélange de 0,8 g de 4-[3-(3-fluoro-6-méthoxy-quinolin-4-yl)-propyl]-1-[2-(3,4,5-trifluoro-phénoxy)-éthyl]-pipéridine-4-carboxylate d'éthyle dans 50 cm³ de dioxanne, 50 cm³ de méthanol et 4,4 cm³ d'une solution aqueuse de soude 5N est agité à une température voisine de 70°C pendant 18 heures. II est ajouté de nouveau 4,4 cm³ d'une solution aqueuse de soude 5N au mélange réactionnel qui est agité pendant 6 heures au voisinage de 70°C. Après refroidissement aux environs de 20°C, la masse réactionnelle est concentrée à sec sous pression réduite (1,1 kPa) à une température voisine de 50°C. Le résidu est purifié par chromatographie sous pression atmosphérique, sur une colonne de gel de silice (granulométrie 20-45 µ ; diamètre 4 cm ; hauteur 20 cm), en éluant par un mélange de chloroforme-méthanol-ammoniaque (12/3/0,5 en volumes) et en recueillant des fractions de 60 cm³. Les fractions 8 à 19 sont réunies, concentrées à sec sous pression réduite (2 kPa) à une température voisine de 40°C et séchées à l'étuve sous pression réduite (10 kPa) aux environs de 50°C. On obtient 0,59 g d'acide 4-[3-(3-fluoro-6-méthoxy-quinolin-4-yl)-propyl]-1-[2-(3,4,5-trifluoro-phénoxy)-éthyl]-pipéridine-4-carboxylique, sous forme d'un solide blanc fondant aux environs de 120°C.
Spectre de R.M.N. ¹H (300 MHz, (CD₃)₂SO d6, δ en ppm) : 1,32 (t très large, J = 12 Hz : 2H) ; 1,59 (mt : 4H) ; 1,95 (d large, J = 12 Hz : 2H) ; 2,07 (t large, J = 11 Hz : 2H) ; 2,59 (t, J = 5,5 Hz : 2H) ; 2,68 (d très large, J = 11 Hz : 2H) ; 3,04 (mt : 2H) ; 3,95 (s : 3H) ; 4,03 (t, J = 5,5 Hz : 2H) ; 6,97 (mt : 2H) ; 7,34 (d, J = 2,5 Hz : 1H) ; 7,39 (dd, J = 9 et 2,5 Hz : 1H) ; 7,96 (d, J = 9 Hz : 1H) ; 8,69 (s large : 1H).

### 4-[3-(3-Fluoro-6-méthoxy-quinotin-4-yl)-propyl]-1-[2-(3,4,5-trifluoro-phénoxy)-éthyl]-pipéridine-4-carboxytate d'éthyle

Un mélange de 0,62 g de 4-[3-(3-fluoro-6-méthoxy-quinolin-4-yl)-propyl]-pipéridine-4-carboxylate d'éthyle, 0,505 g de 5-(2-bromo-éthoxy)-1,2,3-trifluoro-benzène, 0,34 g d'iodure de potassium et 1,14 g de carbonate de potassium dans 80 cm³ d'acétonitrile est agité sous atmosphère inerte pendant 18 heures à une température voisine de 70°C. Après refroidissement aux environs de 20°C, la suspension est filtrée et l'insoluble est lavé par 3 fois 30 cm³ d'acétonitrile. Le filtrat est concentré à sec sous pression réduite (2 kPa) à une température voisine de 40°C. Le résidu d'évaporation est purifié par chromatographie sous pression atmosphérique sur une colonne de gel de silice (granulométrie 20-45 µ ; diamètre 3 cm ; hauteur 21 cm), en éluant par de l'acétate d'éthyle et en recueillant des fractions de 40 cm³. Les fractions 9 à 24 sont réunies puis concentrées à sec sous pression réduite (2 kPa) à une température voisine de 40°C. On obtient 0,8 g de 4-[3-(3-fluoro-6-méthoxy-quinolin-4-yl)-propyl]-1-[2-(3,4,5-trifluoro-phénoxy)-éthyl]-pipéridine-4-carboxylate d'éthyle, sous forme d'une huile visqueuse de couleur jaune claire.
Spectre de R.M.N. ¹H (300 MHz, (CD₃)₂SO d6, δ en ppm) : 0,99 (t, J = 7 Hz : 3H); 1,37 (t très large, J = 12 Hz : 2H) ; de 1,45 à 1,70 (mt : 4H) ; de 1,90 à 2,10 (mt : 4H) ; 2,59 (t, J = 5,5 Hz : 2H) ; 2,69 (d large, J = 12 Hz : 2H) ; 3,04 (t large, J = 7 Hz : 2H) ; 3,94 (s : 3H) ; 3,97 (q, J = 7 Hz : 2H) ; 4,03 (t, J = 5,5 Hz : 2H) ; 6,97 (mt : 2H) ; 7,33 (d, J = 2,5 Hz : 1H) ; 7,39 (dd, J = 9 et 2,5 Hz : 1H) ; 7,97 (d, J = 9 Hz : 1H) ; 8,69 (s large : 1H).

Le 4-[3-(3-fluoro-6-méthoxyquinolin-4-yl)-propyl]-pipéridine-4-carboxylate d'éthyle a éte préparé dans l'exemple 11.

Le 5-(2-bromo-éthoxy)-1,2,3-trifluoro-benzène a éte préparé par application de la méthode décrite dans l'exemple 13.

### Exemple 53

### Acide 4-[3-(3-fluoro-6-méthoxy-quinolin-4-yl)-propyl]-1-[2-(thiazol-2-ylthio)-éthyl]-pipéridine-4-carboxylique

Un mélange de 0,15 g de 4-[3-(3-fluoro-6-méthoxy-quinolin-4-yl)-propyl]-1-[2-(thiazol-2-ylthio)-éthyl]-pipéridine-4-carboxylate d'éthyle dans 3 cm³ de dioxanne, 3 cm³ de méthanol et 1 cm³ d'une solution aqueuse de soude 5N est agité à une température voisine de 70°C pendant 18 heures. Après refroidissement aux environs de 20°C, la masse réactionnelle est concentrée à sec sous pression réduite (2 kPa) à une température voisine de 50°C. Le résidu est repris par 2 fois 20 cm³ de toluène puis concentré à sec comme dans les conditions précédentes. Le solide est purifié par chromatographie sous pression atmosphérique, sur une colonne de gel de silice (bond élut ; granulométrie 70-200 µ ; masse 7 g), en éluant par un mélange de chloroforme-méthanol-ammoniaque (84/14/2 en volumes) et en recueillant des fractions de 5 cm³.

Les fractions 8 à 19 sont réunies, concentrées à sec sous pression réduite (2 kPa) à une température voisine de 40°C puis séchées à l'étuve sous pression réduite (10 kPa) aux environs de 50°C. On obtient 0,114 g d'acide 4-[3-(3-fluoro-6-méthoxy-quinolin-4-yl)-propyl]-1-[2-(thiazol-2-ylthio)-éthyl]-pipéridine-4-carboxylique, sous forme d'un solide blanc.
Spectre de R.M.N. ¹H (300 MHz, (CD₃)₂SO d6, δ en ppm) : 1,31 (t large, J = 12 Hz : 2H) ; de 1,45 à 1,70 (mt : 4H) ; de 1,90 à 2,10 (mt : 4H) ; 2,58 (t, J = 7 Hz : 2H) ; 2,65 (d large, J = 12 Hz : 2H) ; 3,04 (mt : 2H) ; de 3,25 à 3,45 (mt : 2H) ; 3,95 (s : 3H) ; 7,34 (d, J = 2,5 Hz : 1H) ; 7,39 (dd, J = 9 et 2,5 Hz : 1H) ; 7,63 (d, J = 3,5 Hz : 1H) ; 7,71 (d, J = 3,5 Hz : 1 H) ; 7,96 (d, J = 9 Hz : 1H) ; 8,68 (d, J = 1 Hz : 1H).

### 4-[3-(3-Fluoro-6-méthoxy-quinolin-4-yl)-propyl]-1-[2-(thiazol-2-ylthio)-éthyl]-pipéridine-4-carboxylate d'éthyle

Un mélange de 0,65 g de dichlorhydrate de 1-(2-chloro-éthyl)-4-[3-(3-fluoro-6-méthoxy-quinolin-4-yl)-propyl]-pipéridine-4-carboxylate d'éthyle, 0,183 g de 2-mercaptothiazole, 0,21 g d'iodure de potassium et 0,88 g de carbonate de potassium dans 50 cm³ d'acétonitrile est agité sous atmosphère inerte pendant 18 heures à une température voisine de 70°C. Après refroidissement aux environs de 20°C, la suspension est filtrée et l'insoluble est lavé par de l'acétonitrile. Le filtrat est concentré à sec sous pression réduite (2 kPa) à une température voisine de 40°C. Le résidu d'évaporation est repris par un mélange d'acétate d'éthyle-éther de pétrole 40-60°C (8/2 en volumes), filtré et le filtrat est purifié par chromatographie sous pression atmosphérique sur une colonne de gel de silice (granulométrie 70-200 µ ; diamètre 1,5 cm ; masse 100 g), en éluant par de l'acétate d'éthyle-éther de pétrole 40-60°C (8/2 en volumes) et en recueillant des fractions de 15 cm³. Les fractions 41 à 100 sont réunies puis oncentrées à sec sous pression réduite (2 kPa) à une température voisine de 40°C. On obtient 0,15 g de 4-[3-(3-fluoro-6-méthoxy-quinolin-4-yl)-propyl]-1-[2-(thiazol-2-ylthio)-éthyl]-pipéridine-4-carboxylate d'éthyle, sous forme d'une huile épaisse jaune.
Spectre de R.M.N. ¹H (300 MHz, (CD₃)₂SO d6, δ en ppm) : 1,00 (t, J = 7 Hz : 3H) ; 1,36 (t large, J = 11,5 Hz : 2H) ; de 1,45 à 1,70 (mt : 4H) ; de 1,85 à 2,10 (mt : 4H) ; 2,59 (t, J = 7 Hz : 2H) ; 2,68 (d large, J = 12 Hz : 2H) ; 3,05 (t très large, J = 7 Hz : 2H) ; 3,33 (mt : 2H) ; 3,96 (s : 3H) ; 3,98 (q, J = 7 Hz : 2H) ; 7,35 (d, J = 2,5 Hz : 1H) ; 7,40 (dd, J = 9 et 2,5 Hz : 1H) ; 7,64 (d, J = 3,5 Hz : 1H) ; 7,72 (d, J = 3,5 Hz : 1 H) ; 7,97 (d, J = 9 Hz : 1H) ; 8,70 (d, J = 1 Hz : 1H).

Le dichlorhydrate de 1-(2-chloro-éthyl)-4-[3-(3-fluoro-6-méthoxy-quinolin-4-yl)-propyl]-pipéridine-4-carboxylate d'éthyle a été préparé dans l'exemple 5 1.

### Exemple 54

### Acide 4-[3-(3-fluoro-6-méthoxy-quinolin-4-yl)-propyl]-1-(3-phényl-allyl)-pipéridine-4-carboxylique

Un mélange de 0,13 g de 4-[3-(3-fluoro-6-méthoxy-quinolin-4-yl)-propyl]-1-(3-phényl-allyl)-pipéridine-4-carboxylate d'éthyle dans 2 cm³ de dioxanne, 2 cm³ de méthanol et 1,32 cm³ d'une solution aqueuse de soude 5N est porté à une température voisine de 70°C pendant 18 heures. Après refroidissement aux environs de 20°C, le mélange réactionnel est concentré à sec sous pression réduite (2 kPa) à une température voisine de 40°C. Le résidu obtenu est repris par 10 cm³ d'eau, acidifié avec de l'acide acétique, extrait avec de l'acétate d'éthyle, décanté puis filtré et rincé avec 2 fois 10 cm³ d'éthanol. On obtient 0,12 g de d'acide 4-[3-(3-fluoro-6-méthoxy-quinolin-4-yl)-propyl]-1-(3-phényl-allyl)-pipéridine-4-carboxylique, sous forme d'un solide blanc fondant à 240°C.
Spectre de R.M.N. ¹H (300 MHz, (CD₃)₂SO d6, δ en ppm) : 1,35 (t large, J = 11,5 Hz : 2H) ; de 1,50 à 1,75 (mt : 4H) ; de 1,85 à 2,10 (mt : 4H) ; 2,63 (d large, J = 11,5 Hz : 2H) ; de 2,95 à 3,15 (mt : 4H) ; 3,96 (s : 3H) ; 6,26 (dt, J = 16 et 7 Hz : 1H) ; 6,50 (d, J = 16 Hz : 1H) ; de 7,10 à 7,50 (mt : 7H) ; 8,06 (d, J = 9 Hz : 1H) ; 8,69 (s large : 1 H).

### 4-[3-(3-Fluoro-6-méthoxy-quinolin-4-yl)-propyl]-1-(3-phényl-allyl)-pipéridine-4-carboxylate d'éthyle

A un mélange de 0,31 g de 4-[3-(3-fluoro-6-méthoxy-quinolin-4-yl)-propyl]-pipéridine-4-carboxylate d'éthyle, de 0,1 cm³ de trans-cinnamaldéhyde dans 10 cm³ d'éthanol on ajoute 77 mg de complexe borane-pyridine. Le mélange réactionnel est porté à une température voisine de 77°C pendant 22 heures. Après refroidissement aux environs de 20°C, le mélange réactionnel est concentré à sec sous pression réduite (2 kPa) à une température voisine de 40°C. Le résidu obtenu est repris par 10 cm³ d'eau, extrait avec 2 fois 10 cm³ de dichlorométhane, séché sur sulfate de magnésium, filtré puis concentré à sec dans les conditions ci-dessus. Le résidu obtenu est chromatographié sous une pression d'argon de 60 kPa, sur une colonne de gel de silice (granulométrie 70-200µ ; diamètre 2,5 cm ; masse 20 g), en éluant par 500 cm³ d'un mélange d'acétate d'éthyle-cyclohexane (40/60 en volumes), puis d'un mélange de dichlorométhane-méthanol (95/5 en volumes). Les fractions contenant le produit attendu sont réunies puis concentrées à sec dans les conditions ci-dessus. On obtient 0,13 g de 4-[3-(3-fluoro-6-méthoxy-quinolin-4-yl)-propyl]-1-(3-phényl-allyl)-pipéridine-4-carboxylate d'éthyle, sous forme d'une huile visqueuse.
Spectre de R.M.N. ¹H (300 MHz, (CD₃)₂SO d6, δ en ppm) : 1,01 (t, J = 7 Hz : 3H) ; 1,40 (t large, J = 11,5 Hz : 2H) ; de 1,45 à 1,75 (mt : 4H) ; de 1,90 à 2,05 (mt : 4H) ; 2,65 (d très large, J = 12 Hz : 2H) ; 3,02 (d, J = 7 Hz : 2H) ; 3,05 (mt : 2H) ; 3,96 (s : 3H) ; 3,97 (q, J = 7 Hz : 2H) ; 6,25 (dt, J = 16 et 7 Hz : 1H) ; 6,51 (d, J = 16 Hz : 1H) ; de 7,15 à 7,50 (mt : 7H) ; 7,96 (d, J = 9 Hz : 1H) ; 8,70 (d, J = 1 Hz : 1H).

Le 4-[3-(3-fluoro-6-méthoxyquinolin-4-yl)-propyl]-pipéridine-4-carboxylate d'éthyle a été préparé dansl'exemple 11.

### Exemple 55

### Acide 4-[3-(3-chloro-6-methoxy-quinolin-4-yl)-propyl]-1-(3-phenyl-propyl)-piperidine-4-hydroxamique

Une solution de 0,1 g de 4-[3-(3-chloro-6-méthoxy-quinolin-4-yl)-propyl]-1-(3-phenyl-propyl)-piperidine-4-tert-butoxy-amide carboxylique dans de 2 cm³ d'acide trifluoroacétique est laissée pendant 60 jours à une température voisine de 20°C. La solution est évaporée sous pression réduite (2 kPa) à une température voisine de 40°C puis purifiée par chromatographie sous pression atmosphérique, sur bond élut de silice (granulométrie 70-200 µ; volume 25 cm³), en éluant par un mélange de dichlorométhane méthanol ammoniaque (40/5/0,3 en volumes). Les fractions contenant le produit sont réunies puis évaporées dans les conditions décrites précédemment. On obtient 0,036 g d'acide 4-[3-(3-chloro-6-methoxy-quinolin-4-yl)-propyl]-I-(3-phenyl-propyl)-piperidine-4-hydroxamique sous forme d'une huile incolore.
Spectre de R.M.N. ¹H (300 MHz, (CD₃)₂SO d6, δ en ppm) : 1,33 (mt : 2H) ; 1,51 (mt : 2H) ; de 1,60 à 1,80 (mt : 4H) ; de 1,90 à 2,10 (mt : 4H) ; 2,18 (t, J = 7 Hz : 2H) ; de 2,45 à 2,65 (mt : 4H) ; 3,12 (t large, J = 7,5 Hz : 2H) ; 3,96 (s : 3H) ; de 7,15 à 7,25 (mt : 3H); 7,28 (t mt, J = 7,5 Hz : 2H) ; 7,36 (d, J = 2,5 Hz : 1H) ; 7,45 (dd, J = 9 et 2,5 Hz : 1H) ; 7,96 (d, J = 9 Hz : 1H) ; 8,56 (mf: 1H) ; 8,67 (s : 1H) ; 10,37 (mf: 1H).

### 4-[3-(3-Chloro-6-methoxy-quinolin-4-yl)-propyl]-1-(3-phenyl-propyl)-piperidine-4-tert-butoxy-amide

Un mélange de 0,5 g d'acide 4-[3-(3-chloro-6-méthoxyquinolin-4-yl)propyl]-1-(3-phénylpropyl)pipéridine-4-carboxylique, 0,175 g de (1-hydroxybenzotriazole hydrate), 0,498 g de (1-[3-(diméthylamino)propyl]-3-éthylcarbodiimide hydrochloride) et 0,58 cm³ de triéthylamine et 0,4 g de o-tert-butyle hydroxylamine est agitée pendant 48 heures à une température voisine de 20°C. Le milieu réactionnel est dilué par 50 cm³ d'eau, agité puis décanté. La phase aqueuse est extraite par 2 fois 25 cm³ de dichlorométhane et les extraits organiques sont réunis, séchés sur sulfate de magnésium, filtrés puis évaporés sous pression réduite (2 kPa) à une température voisine de 40°C. Le résidu obtenu est purifié par chromatographie sous pression atmosphérique, sur une colonne de gel de silice (granulométrie 20-45 µ ; diamètre 2,5 cm ; masse 18 g), en éluant par un mélange de dichlorométhane méthanol ammoniaque (40/5/0,2 en volumes). Les fractions contenant le produit sont réunies puis évaporées dans les conditions décrites précédemment. On obtient 0,27 g de 4-[3-(3-chloro-6-methoxy-quinolin-4-yl)-propyl]- 1-(3-phenyl-propyl)-piperidine-4-tert-butoxy-amide.
Spectre de R.M.N. ¹H (300 MHz, (CD₃)₂SO d6, δ en ppm) : 1,09 (s : 9H) ; 1,35 (mt : 2H) ; 1,54 (mt : 2H) ; 1,67 (mt : 4H) ; 1,95 (t large, J = 11 Hz : 2H) ; 2,08 (d large, J = 13,5 Hz : 2H) ; 2,19 (t, J = 7 Hz : 2H) ; de 2,50 à 2,65 (mt : 4H) ; 3,14 (t large, J = 7,5 Hz : 2H) ; 3,98 (s : 3H) ; de 7,10 à 7,25 (mt : 3H) ; 7,27 (t large, J = 7,5 Hz : 2H) ; 7,36 (d, J = 2,5 Hz : 1H) ; 7,45 (dd, J = 9 et 2,5 Hz : 1H) ; 7,96 (d, J = 9 Hz : 1H) ; 8,68 (s : 1H) ; 10,06 (s large : 1H).

Le dichlorhydrate de l'acide 4-[3-(3-chloro-6-méthoxyquinolin-4-yl)-propyl]-1-(3-phénylpropyl)-pipéridine-4-carboxylique a été préparé dans l'exemple 4.

Le 4-[3-(3-chloro-6-méthoxyquinolin-4-yl)propyl]-1-(3-phénylpropyl)-pipéridine-4-carboxylate de benzyle a été préparé dans l'exemple 4.

### Exemple 56

### Chlorhydrate de l'acide 4-[3-(3-fluoro-6-méthoxy-quinolin-4-yl)-propyl]-1-[3-(2,5-difluorophényl)-prop-2-ène-yl]-pipéridine-4-carboxylique

En opérant de manière analogue à l'exemple 54, on prépare le chlorhydrate de l'acide 4-[3-(3-fluoro-6-méthoxy-quinolin-4-yl)-propyl]-1-[3-(2,5-difluorophényl)-prop-2-ène-yl]-pipéridine-4-carboxylique.
Spectre de R.M.N. ¹H (400 MHz, (CD₃)₂SO d6 avec ajout de quelques gouttes de CD₃COOD d4 à une température de 383K, δ en ppm) : 1,73 (mt : 6H) ; 2,22 (d large, J = 14 Hz : 2H) ; 3,01 (mf : 2H) ; 3,11 (t large, J = 7 Hz : 2H) ; 3,42 (d très large, J = 12 Hz : 2H) ; 3,94 (d, J = 7 Hz : 2H) ; 3,98 (s : 3H) ; 6,48 (dt, J = 16,5 et 7 Hz : 1H) ; 6,93 (d, J = 16,5 Hz : 1H) ; de 7,10 à 7,30 (mt : 2H) ; 7,35 (s large : 1H) ; 7,40 (dd, J = 9 et 2,5 Hz : 1H) ; 7,44 (mt : 1H) ; 7,98 (d, J = 9 Hz : 1H) ; 8,64 (s large : 1H).

### Exemple 57

### Acide 4-[3-(3-fluoro-6-méthoxy-quinolin-4-yl)-propyl]-1-[2-(2,5-difluorophénoxy)-éthyl]-pipéridine-4-hydroxamique

En opérant de manière analogue à l'exemple 55, on prépare l'acide 4-[3-(3-fluoro-6-méthoxy-quinolin-4-yl)-propyl]-1-[2-(2,5-difluorophénoxy)-éthyl]-pipéridine-4-hydroxamique.
Spectre de R.M.N. ¹H (300 MHz, (CD₃)₂SO d6, δ en ppm) : 1,31 (t très large, J = 12,5 Hz : 2H) ; de 1,45 à 1,70 (mt : 4H) ; 2,02 (d large, J = 12,5 Hz : 2H) ; 2,10 (t large, J = 11 Hz : 2H) ; de 2,55 à 2,75 (mt : 4H) ; 3,00 (mt : 2H) ; 3,97 (s : 3H) ; 4,12 (t, J = 5,5 Hz : 2H) ; 6,75 (mt : 1H) ; 7,14 (mt : 1H) ; 7,24 (mt : 1H) ; 7,33 (d, J = 3 Hz : 1H) ; 7,40 (dd, J = 9 et 3 Hz : 1H) ; 7,96 (d, J = 9 Hz : 1H) ; 8,63 (mf: 1H) ; 8,70 (d, J = 0,5 Hz : 1H) ; 10,40 (mf : 1H).

### Exemple 58

### Acide 1-cinnamyl 4-[3-(3-fluoro-6-méthoxy-quinolin-4-yl)-propyl]-pipéridine-4-hydroxamique

En opérant de manière analogue à l'exemple 55, on prépare l'acide 1-cinnamyl 4-[3-(3-fluoro-6-méthoxy-quinolin-4-yl)-propyl]-pipéridine-4-hydroxamique.
Spectre de R.M.N. ¹H (400 MHz, (CD₃)₂SO d6, avec ajout de quelques gouttes de CD₃COOD d4, à une température de 373K, δ en ppm) : de 1,50 à 1,75 (mt : 6H) ; 2,23 (d large, J = 14 Hz : 2H) ; 2,81 (t large, J = 11,5 Hz : 2H) ; 3,04 (mt : 2H) ; 3,22 (d large, J = 11,5 Hz : 2H) ; 3,69 (d, J = 7 Hz : 2H) ; 3,97 (s : 3H) ; 6,24 (dt, J = 16 et 7 Hz : 1H) ; 6,78 (d, J = 16 Hz : 1H) ; de 7,20 à 7,50 (mt : 7H) ; 7,96 (d, J = 9 Hz : 1H) ; 8,62 (s large : 1H).

### Exemple 59

### 4-[3-(3-chloro-6-trifluorométhyl-quinolin-4-yl)-propyl]-1-[2-(2,5-difluorophénoxy)-éthyl]-pipéridine-4-carboxylate de sodium

En opérant de manière analogue à l'exemple 1, on prépare le 4-[3-(3-chloro-6-trifluorométhyl-quinolin-4-yt)-propyl]-l-[2-(2,5-difluorophénoxy)-éthyl]-pipéndine-4-carboxylate de sodium.
Spectre de R.M.N. ¹H (300 MHz, (CD₃)₂SO d6, δ en ppm) : 1,09 (t très large, J = 11,5 Hz : 2H) ; de 1,40 à 1,70 (mt : 4H) ; 1,98 (d large, J = 11,5 Hz : 2H) ; 2,14 (t, J = 10,5 Hz : 2H) ; de 2,50 à 2,70 (mt : 4H) ; 3,22 (t large,-J = 7 Hz : 2H) ; 4,10 (t, J = 5,5 Hz : 2H) ; 6,74 (mt : 1H) ; 7,12 (mt : 1H) ; 7,23 (mt : 1H) ; 8,06 (d large, J = 9 Hz : 1H) ; 8,26 (d, J = 9 Hz : 1H) ; 8,54 (s large : 1H) ; 9,00 (s : 1H).

Les composés hétérocyclylalcoyl pipéridine de formule (I) peuvent être utilisés pour préparer des compositions pharmaceutiques contenant au moins un de ces composés.

échéant sous forme de sel, à l'état pur ou sous forme d'une association avec un ou plusieurs diluants et/ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés hétérocyclylalcoyl pipéridine de formule (I)sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 750 mg et 3 g de produit actif en 2 ou 3 prises par jour par voie orale ou entre 400 mg et 1,2 g par voie intraveineuse pour un adulte.

## Revendications

1. L'un quelconque des composés de formule (III) dont les noms suivent:
- la 4-chloro-3-fluoro-6-méthoxy-quinoléine,
- la 4-bromo-3-fluoro-6-méthoxy-quinoléin,e
- la 4-iodo-3-fluoro-6-méthoxy-quinoléine.

2. L'un quelconque des composés de formule (III) dont les noms suivent:
- la 4-bromo-3-chloro-6-méthoxy-quinoléin,e
- la 4-iodo-3-chloro-6-méthoxy-quinoléine.

3. La 4-hydroxy-3-chloro-6-méthoxy-quinoléine.

4. La 3-fluoro-6-méthoxy-quinoléine.
